# EUROPEAN PATENT APPLICATION

(11) **EP 4 008 333 A1**
(43) Date of publication of application: **08.06.2022**
(21) Application number: 21205379.7
(22) Date of filing: 19.02.2019
(51) Int. Cl.: A61K 31/7088, C12N 15/11, A61K 48/00

(54) **COMPOSITIONS AND METHODS FOR ORGAN-PROTECTIVE EXPRESSION AND MODULATION OF CODING RIBONUCLEIC ACIDS**

(30) Priority: 19.02.2018 US 201862632056 P; 06.09.2018 WO PCT/US2018/049772
(62) Divisional of application: 19711652.8
(71) Applicant: Combined Therapeutics, Inc., Wilmington, DE 19808 (US)
(72) Inventor: MICOL, Romain, London SW1W 9RB (GB)
(74) Representative: Crease, Devanand John

(57) **Abstract**

Provided is an isolated mRNA sequence for expression of one or more cytokines within one or more target organs, the sequence comprising at least one coding sequence which codes for one or more cytokines (or their ligands) involved in immune response and inflammation selected from one or more of: TNFα, TNFβ, IFNα, IFNβ, IFNgamma, IL1, IL2, IL3, IL4, IL5, IL6, IL7, IL8, IL9, IL10, IL11, IL12, CCL2, CCL3, CCL4, CCL5, CXCL 9, and CXCL10. The sequence also comprises at least a first untranslated region (UTR) sequence, and at least three different micro-RNA (miRNA) binding site sequences. Each of the miRNA binding site sequences is located within, immediately 5' to or immediately 3' to, the first UTR sequence; and the miRNA binding site sequences allow for differential expression of the coding sequence in at least a first and a second cell type within the target organ or organs. Also provided are pharmaceutical compositions comprising the mRNA sequence, and methods for using the sequence or composition, particularly in treatment of disease, such as cancer of the liver, brain, lung, breast, pancreas, colon and kidney.

SEQUENCE LISTING

<110> Combined Therapeutics

<120> COMPOSITIONS AND PROCESSES FOR TARGETED DELIVERY, EXPRESSION AND
MODULATION OF CODING RIBONUCLEIC ACIDS IN TISSUE

<130> P55344EPA

<150> PCT/US18/49772
<151> 2018-09-06
<150> US62632056
<151> 2018-02-19

<160> 33

<170> BiSSAP 1.3.6

<210> 1
<211> 22
<212> RNA
<213> Artificial Sequence

<220>
<223> single miR122 binding site

<400> 1
aacgccauua ucacacuaaa ua                                                22

<210> 2
<211> 22
<212> RNA
<213> Artificial Sequence

<220>
<223> single miR125a binding site

<400> 2
acaggugagg uucuugggag cc                                                22

<210> 3
<211> 22
<212> RNA
<213> Artificial Sequence

<220>
<223> single miRNA 124a binding site

<400> 3
uaaggcacgc ggugaaugcc aa                                                22

<210> 4
<211> 22
<212> RNA
<213> Artificial Sequence

<220>
<223> single miRNA Let-7b binding site

<400> 4
cuauacaacc uacugccuuc cc                                                22


<210> 5
<211> 22
<212> RNA
<213> Artificial Sequence


<220>
<223> single miRNA-375 binding site

<400> 5
uuuguucguu cggcucgcgu ga                                                22


<210> 6
<211> 22
<212> RNA
<213> Artificial Sequence


<220>
<223> single miRNA-192 binding site

<400> 6
cugccaauuc cauaggucac ag                                                22


<210> 7
<211> 21
<212> RNA
<213> Artificial Sequence


<220>
<223> single miRNA-143 binding site

<400> 7
ugagaugaag cacuguagcu c                                                 21


<210> 8
<211> 131
<212> RNA
<213> Artificial Sequence


<220>
<223> linked binding sites for
      miRNAmiRNA122-miRNA125a-miRNA122-miRNA125a-miRNA122

<400> 8
aacgccauua ucacacuaaa uauuuaaaac aggugagguu cuugggagcc uuuaaaaacg      60

ccauuaucac acuaaauauu uaaaggugag guucuuggga gccuuuaaaa acgccauuau     120

cacacuaaau a                                                           131

<210> 9
<211> 134
<212> RNA
<213> Artificial Sequence

<220>
<223> linked binding sites for
      miRNA122-miRNA124a-miRNA122-miRNA124a-miRNA122

<400> 9
aacgccauua ucacacuaaa uauuuaaaua aggcacgcgg ugaaugccaa uuuaaaaacg      60

ccauuaucac acuaaauauu uaaauaaggc acgcggugaa ugccaauuua aaaacgccau     120

uaucacacua aaua                                                      134

<210> 10
<211> 134
<212> RNA
<213> Artificial Sequence

<220>
<223> linked binding sites for miRNA122-Let-7-miRNA122-Let-7-miRNA122

<400> 10
aacgccauua ucacacuaaa uauuuaaacu auacaaccua cugccuuccc uuuaaaaacg      60

ccauuaucac acuaaauauu uaaacuauac aaccuacugc cuucccuuua aaaacgccau     120

uaucacacua aaua                                                      134

<210> 11
<211> 134
<212> RNA
<213> Artificial Sequence

<220>
<223> linked binding sites for
      miRNA-122-miRNA125a-miRNA124a-let-7-miRNA375

<400> 11
aacgccauua ucacacuaaa uauuuaaaac agguagaguu cuugggagcc uuuaaauaag      60

gcacgcggug aaugccaauu uaaacuauac aaccuacugc cuucccuuua aauuuguucg     120

uucggcucgc guga                                                      134

<210> 12
<211> 78
<212> RNA
<213> Artificial Sequence

<220>
<223> linked binding sites for miRNA122-miRNA124a-Let7

<400> 12
aacgccauua ucacacuaaa uauuuaaaua aggcacgcgg ugaaugccaa uuuaaacuau     60

acaaccuacu gccuuccc                                                   78


<210> 13
<211> 106
<212> RNA
<213> Artificial Sequence


<220>
<223> linked binding sites for miRNA122-miRNA125a-miRNA124a-Let7

<400> 13
aacgccauua ucacacuaaa uauuuaaaac aggugagguu cuugggagcc uuuaaauaag     60

gcacgcggug aaugccaauu uaaacuauac aaccuacugc cuuccc                   106


<210> 14
<211> 106
<212> RNA
<213> Artificial Sequence


<220>
<223> linked binding sites for miRNA122-miRNA125a-miRNA375-Let7

<400> 14
aacgccauua ucacacuaaa uauuuaaaac aggugagguu cuugggagcc uuuaaauuug     60

uucguucggc ucgcgugauu uaaacuauac aaccuacugc cuuccc                   106


<210> 15
<211> 134
<212> RNA
<213> Artificial Sequence


<220>
<223> linked binding sequences for
      miRNA122-miRNA124a-Let7-miRNA375-miRNA192

<400> 15
aacgccauua ucacacuaaa uauuuaaaua aggcacgcgg ugaaugccaa uuuaaacuau     60

acaaccuacu gccuucccuu uaaauuuguu cguucggcuc gcgugauuua aacugccaau    120

uccauagguc acag                                                      134


<210> 16
<211> 133
<212> RNA
<213> Artificial Sequence


<220>
<223> linked binding sequences for
      miRNA122-miRNA125a-miRNA375-miRNA192-miRNA143

<400> 16
aacgccauua ucacacuaaa uauuuaaaac aggugagguu cuugggagcc uuuaaauuug      60

uucguucggc ucgcgugauu uaaacugcca auuccauagg ucacaguuua aaugagauga     120

agcacuguag cuc                                                        133


<210> 17
<211> 1445
<212> RNA
<213> Artificial Sequence


<220>
<223> US3 coding sequence

<400> 17
auggccuguc guaaguuuug ucgcguuuac gggggacagg gcaggaggaa ggaggaggcc      60

gucccgccgg agacaaagcc gucccgggug uuuccucaug gccccuuuua uaccccagcc     120

gaggacgcgu gccuggacuc cccgcccccg gagaccccca aaccuuccca caccacacca     180

cccggcgaug ccgagcgccu gugucaucug caggagaucc uggcccagau guacggaaac     240

caggacuacc ccauagagga cgaccccagc gcggaugccg cggacgaugu cgacgaggac     300

gccccggacg acguggccua uccggaggaa uacgcagagg agcuuuuucu gcccggggac     360

gcgcccgguc cccuuaucgg ggccaacgac cacaucccuc ccccgugugg cgcaucuccc     420

cccgguauac gacgacgcag ccgggaugag auugggccca cgggauuuac cgcggaagaa     480

cuggacgcca uggacaggga ggcggcucga gccaucagcc gcggcggcaa gcccccucgg      540

accaggccaa gcuggugacu ggcaugggcu uuacgaucca cggagcgcuc accccaggau     600

cggaggggug ugucuuugac agcagccacc cagauuaccc ccaacgggua aucgugaagg     660

cgggguggua cacgagcacg agccacgagg cgcgacugcu gaggcgacug gaccaccccg     720

cgauccugcc ccuccuggac cugcaugucg ucuccggggu cacgugucug guccucccca     780

aguaccaggc cgaccuguau accuaucuga guaggcgccu gaacccgcug ggacgcccgc     840

agaucgcagc ggucucccgg cagcuccuaa gcgccguuga cuacauucac cgccagggca     900

uuauccaccg cgacauuaag accgaaaaua uuuuuauuaa cacccccgag gacauuugcc     960

ugggggacuu uggugccgcg ugcuucgugc aggguucccg aucaagcccc uuccccuacg    1020

gaaucgccgg aaccaucgac accaacgccc ccgagguccu gaccggggau ccguauacca    1080

ccaccgucga cauuuggagc gccggucugg ugaucuucga gacugccguc cacaacgcgu    1140

ccuuguucuc ggccccccgc ggccccaaaa ggggcccgug cgacagucag aucacccgca    1200

ucauccgaca ggcccagguc cacguugacg aguuuccccc gcauccagaa ucgcgccuca    1260

ccucgcgcua ccgcucccgc gcggccggga acaaucgccc gccguacacc cgaccggccu    1320

ggacccgcua cuacaagaug gacauagacg ucgaauaucu gguuugcaaa gcccucaccu 1380

ucgacggcgc gcuucgcccc agcgccgcag agcugcuuug uuugccgcug uuucaacaga 1440

aauga 1445

<210> 18
<211> 1577
<212> RNA
<213> Artificial Sequence

<220>
<223> US3 coding sequence with linked binding sites for
      miRNAmiRNA122-miRNA125a-miRNA122-miRNA125a-miRNA122

<400> 18
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auuggggcca cgggauuuac cgcggaagaa 480

cuggacgcca uggacaggga ggcggcucga gccaucagcc gcggcggcaa gcccccucgg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cugggggacu uuggugccgc gugcuucgug caggguuccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaaacaggu gagguucuug ggagccuuua 1500

aaaacgccau uaucacacua aauauuuaaa ggugagguuc uugggagccu uuaaaaacgc 1560

cauuaucaca cuaaaua 1577

<210> 19
<211> 1580
<212> RNA
<213> Artificial Sequence

<220>
<223> US3 coding sequence with linked binding sites for
      miRNA122-miRNA124a-miRNA122-miRNA124a-miRNA122

<400> 19
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auugggccca cgggauuuac cgcggaagaa 480

cuggacgcca uggacaggga ggcggcucga gccaucagcc gcggcggcaa gccccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cugggggacu uuggugccgc gugcuucgug caggguuccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaauaaggc acgcggugaa ugccaauuua 1500

aaaacgccau uaucacacua aauauuuaaa uaaggcacgc ggugaaugcc aauuuaaaaa 1560

cgccauuauc acacuaaaua 1580

<210> 20
<211> 1580
<212> RNA
<213> Artificial Sequence

<220>
<223> US3 coding sequence with linked binding sites for
      miRNA122-Let-7-miRNA122-Let-7-miRNA122

<400> 20
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auuggggcca cgggauuuac cgcggaagaa 480

cuggacgcca uggacaggga ggcggcucga gccaucagcc gcggcggcaa gcccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cuggggggacu uuggugccgc gugcuucgug cagguucccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuuuccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaacuauac aaccuacugc cuucccuuua 1500

aaaacgccau uaucacacua aauauuuaaa cuauacaacc uacugccuuc ccuuuaaaaa 1560

cgccauuauc acacuaaaua 1580

<210> 21
<211> 1606
<212> RNA
<213> Artificial Sequence

<220>
<223> US3 coding sequence with linked binding sites for
      miRNA-122-miRNA125a-miRNA124a-let-7-miRNA375

<400> 21
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auugggccca cgggauuuac cgcggaagaa 480

cuggacgcca uggacaggga ggcggcucga gccaucagcc gcggcggcaa gccccccucg 540

accauggcca agcuggugac uggcaugggc uuacgaucca cggagcgcuc accccaggau 600

cggaggggug ugucuuugac agcagccacc cagauuaccc ccaacgggua aucgugaagg 660

cgggguggua cacgagcacg agccacgagg cgcgacugcu gaggcgacug gaccaccccg 720

cgauccugcc ccuccuggac cugcaugucg ucuccggggu cacgugucug guccucccca 780

aguaccagcc gaccuguaua ccuaucugag uaggcgccug aacccgcugg gacgcccgca 840

gaucgcagcg gucucccggc agcuccuaag cgccguugac uacauucacc gccagggcau 900

uauccaccgc gacauuaaga ccgaaaauau uuuuauuaac acccccgagg acauuugccu 960

gggggacuuu ggugccgcgu gcuucgugca ggguucccga ucaagccccu uccccuacgg 1020

aaucgccgga accaucgaca ccaacgcccc cgagguccug accggggauc cguauaccac 1080

caccgucgac auuuggagcg ccggucuggu gaucuucgag acugccgucc acaacgcguc 1140

cuuguucucg gccccccgcg gccccaaaag gggcccgugc gacagucaga ucacccgcau 1200

cauccgacag gcccaggucc acguugacga guuuuccccg cauccagaau cgcgccucac 1260

cucgcgcuac cgcucccgcg cggccgggaa caaucgcccg ccguacaccc gaccggccug 1320

gacccgcuac uacaagaugg acauagacgu cgaauaucug guuugcaaag cccucaccuu 1380

cgacggcgcg cuucgcccca gcgccgcaga gcugcuuugu uugccgcugu uucaacagaa 1440

augaaacgcc auuaucacac uaaauauuua aaacagguga gguucuuggg agccuuuaaa 1500

uaaggcacgc ggugaaugcc aauuuaaacu auacaaccua cugccuuccc uuuaaauuug 1560

uucguucggc ucgcgugauu uaaauuuguu cguucggcuc gcguga 1606

<210> 22
<211> 1524
<212> RNA
<213> Artificial Sequence

<220>
<223> US3 coding sequence with linked binding sites for
      miRNA122-miRNA124a-Let7

<400> 22
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auuggggcca cgggauuuac cgcggaagaa 480

cuggacgcca uggacaggga ggcggcucga gccaucagcc gcggcggcaa gccccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cugggggacu uuggugccgc gugcuucgug cagguucccg gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaauaaggc acgcggugaa ugccaauuua 1500

aacuauacaa ccuacugccu uccc 1524

<210> 23
<211> 1552
<212> RNA
<213> Artificial Sequence

<220>
<223> US3 coding sequence with linked binding sites for
      miRNA122-miRNA125a-miRNA124a-Let7

<400> 23
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auugggccca cgggauuuac cgcggaagaa 480

cuggacgcca uggacaggga ggcggcucga gccaucagcc gcggcggcaa gccccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cuggggacu uuggugccgc gugcuucgug cagguucccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaaacaggu gagguucuug ggagccuuua 1500

aauaaggcac gcggugaaug ccaauuuaaa cuauacaacc uacugccuuc cc 1552

<210> 24
<211> 1551
<212> RNA
<213> Artificial Sequence

<220>
<223> US3 coding sequence with linked binding sites for
      miRNA122-miRNA125a-miRNA375-Let7

<400> 24
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auugggccca cgggauuuac cgcggaagaa 480

cuggacgcca uggacaggga ggcggcucga gccaucagcc gcggcggcaa gccccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cugggggacu uuggugccgc gugcuucgug caggguuccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaaacaggu gagguucuug ggagccuuua 1500

aauuuguucg uucggcucgc gugauuuaaa cuauacaacc uacugccuuc c 1551

<210> 25
<211> 1580
<212> RNA
<213> Artificial Sequence

<220>
<223> US3 coding sequence with linked binding sequences for
      miRNA122-miRNA124a-Let7-miRNA375-miRNA192

<400> 25
   auggccuguc guaaguuuug ucgcguuuac gggggacagg gcaggaggaa ggaggaggcc 60
   gucccgccgg agacaaagcc gucccgggug uuuccucaug gccccuuuua uaccccagcc 120
   gaggacgcgu gccuggacuc cccgcccccg gagaccccca aaccuuccca caccacacca 180
   cccggcgaug ccgagcgccu gugucaucug caggagaucc uggcccagau guacggaaac 240
   caggacuacc ccauagagga cgaccccagc gcggaugccg cggacgaugu cgacgaggac 300
   gccccggacg acguggccua uccggaggaa uacgcagagg agcuuuuucu gcccggggac 360
   gcgcccgguc cccuuaucgg ggccaacgac cacaucccuc ccccgugugg cgcaucuccc 420
   cccgguauac gacgacgcag ccgggaugag auugggccca cgggauuuac cgcggaagaa 480
   cuggacgcca uggacaggga ggcggcucga gccaucagcc gcggcggcaa gcccccucgg 540
   accauggcca agcuggugac uggcaugggc uuuacgaucc acggagcgcu caccccagga 600
   ucggaggggu gugucuuuga cagcagccac ccagauuacc cccaacgggu aaucgugaag 660
   gcgggguggu acacgagcac gagccacgag gcgcgacugc ugaggcgacu ggaccacccc 720
   gcgauccugc cccuccugga ccugcauguc gucuccgggg ucacgugucu gguccucccc 780
   aaguaccagg ccgaccugua uaccuaucug aguaggcgcc ugaacccgcu gggacgcccg 840
   cagaucgcag cggucucccg gcagcuccua agcgccguug acuacauuca ccgccagggc 900
   auuauccacc gcgacauuaa gaccgaaaau auuuuuauua acacccccga ggacauuugc 960
   cugggggacu uuggugccgc gugcuucgug caggguuccc gaucaagccc cuuccccuac 1020
   ggaaucgccg gaaccaucga caccaacgcc cccgaggucc ugaccgggga uccguauacc 1080
   accaccgucg acauuuggag cgccggucug gugaucuucg agacugccgu ccacaacgcg 1140
   uccuuguucu cggccccccg cggccccaaa aggggcccgu gcgacaguca gaucacccgc 1200
   aucauccgac aggcccaggu ccacguugac gaguuucccc cgcauccaga aucgcgccuc 1260
   accucgcgcu accgcucccg cgcggccggg aacaaucgcc cgccguacac ccgaccggcc 1320
   uggacccgcu acuacaagau ggacauagac gucgaauauc ugguuugcaa agcccucacc 1380
   uucgacggcg cgcuucgccc cagcgccgca gagcugcuuu guuugccgcu guuucaacag 1440
   aaaugaaacg ccauuaucac acuaaauauu uaaauaaggc acgcggugaa ugccaauuua 1500
   aacuauacaa ccuacugccu ucccuuuaaa uuuguucguu cggcucgcgu gauuuaaacu 1560
   gccaauucca uaggucacag 1580

<210> 26
<211> 1578
<212> RNA
<213> Artificial Sequence

<220>
<223> US3 coding sequence with linked binding sequences for
      miRNA122-miRNA125a-miRNA375-miRNA192-miRNA143

<400> 26
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auuggggcca cgggauuuac cgcggaagaa 480

cuggacgcca uggacaggga ggcggcucga gccaucagcc gcggcggcaa gccccccucg 540

accauggcaa gcuggugacu ggcaugggcu uuacgaucca cggagcgcuc accccaggau 600

cggaggggug ugucuuugac agcagccacc cagauuaccc ccaacgggua aucgugaagg 660

cgggguggua cacgagcacg agccacgagg cgcgacugcu gaggcgacug gaccaccccg 720

cgauccugcc ccuccuggac cugcaugucg ucuccggggu cacgugucug guccucccca 780

aguaccaggc cgaccuguau accuaucuga guaggcgccu gaacccgcug ggacgcccgc 840

agaucgcagc ggucucccgg cagcuccuaa gcgccguuga cuacauucac cgccagggca 900

uuauccaccg cgacauuaag accgaaaaua uuuuuauuaa cacccccgag gacauuugcc 960

ugggggacuu uggugccgcg ugcuucgugc aggguucccg aucaagcccc uuccccuacg 1020

gaaucgccgg aaccaucgac accaacgccc ccgagguccu gaccggggau ccguauacca 1080

ccaccgucga cauuuggagc gccggucugg ugaucuucga gacugccguc cacaacgcgu 1140

ccuuguucuc ggccccccgc ggccccaaaa ggggcccgug cgacagucag aucacccgca 1200

ucauccgaca ggcccagguc cacguugacg aguuuucccc gcauccagaa ucgcgccuca 1260

ccucgcgcua ccgcucccgc gcggccggga acaaucgccc gccguacacc cgaccggccu 1320

ggacccgcua cuacaagaug gacauagacg ucgaauaucu gguuugcaaa gcccucaccu 1380

ucgacggcgc gcuucgcccc agcgccgcag agcugcuuug uuugccgcug uuucaacaga 1440

aaugaaacgc cauuaucaca cuaaauauuu aaaacaggug agguucuugg gagccuuuaa 1500

auuuguucgu ucggcucgcg ugauuuaaac ugccaauucc auaggucaca guuuaaauga 1560

gaugaagcac uguagcuc 1578

<210> 27
<211> 2513
<212> RNA
<213> Artificial Sequence

<220>
<223> RR1 coding sequence with binding sequences for miRNA-122,
      miRNA-125a, miRNA-124a, let-7, miRNA-375

<400> 27
augcauguga ucaagcgaga uggccgccaa gagcgaguua uguuugacaa aauuacauca 60

cgaauccaga aacucuguua uggacucaac auggacuuug uugauccugc ucagaucacc 120

augaaaguaa uccaaggccu auauaguggg gucaccacag uggaacugga cacccuggcu 180

gcugagacag ccgcgaccuu gaccacgaag cacccugacu augccauccu ggcagcaagg 240

auagccgucu cuaacuugca caaagaaaca aagaaagugu ucagugaugu gauggaggau 300

cucuacaacu acauaaaucc gcacaacggc agacacucuc ccaugguggc cagcucaaca 360

cucgacauug uuauggccaa uaaggaucgc cugaauucug ccauuaucua ugaccgagau 420

uucucuuaua acuacuuugg cuuuaagaca cuggaacggu cauauuuguu gaagaucaau 480

gguaaagugg cugaaagacc acagcauaug uugaugaggg uuucuguggg gauucacaaa 540

gaagauauug augcugcaau ugaaaccuac aaccuacuuu cugagaagug guucacucau 600

gccucuccua cucucuucaa ugcuggaccc aaccgcccac agcugucuag cuguuuccuc 660

uugaguauga aagaugacag cauugaagga auuuaugaua cucugaagca gugugccuug 720

auuucuaagu ccgcuggggg aauugguguu gcugugaguu guauucgggc cacugguagc 780

uacaucgcug ggacuaaugg caauucuaau ggccuugugc caaugcugag aguauauaac 840

aacacagcuc gcuaugugga ucaaggugga aacaagcgcc caggcgcguu ugcuauuuac 900

cuggagccuu ggcacuuaga caucuuugag uuccuugacu ugaagaagaa cacaggcaag 960

gaagaacagc gagcacgcga ucucuucuuu gcacuuugga ucccagaucu cuucaugaag 1020

cgaguggaga cuaaccagga cuggucauug auguguccca augagugucc uggucuggac 1080

gaggucuggg gagaggaguu ugagaaguua uaugaaaguu acgagaagca gggucguguc 1140

cgaaaaguug uaaaagcuca gcagcuuugg uaugccauca uugaguccca gacggagacc 1200

gguaccccau acaugcucua caaagauucc uguaaccgga agagcaacca gcagaaccug 1260

ggaaccauca aaugcagcaa ccuguguaca gaaauaguag aguacaccag uaaagaugag 1320

guugcaguuu guaacuuggc uucucuggcu cugaauaugu augucacacc ggaacauacg 1380

uaugacuuug agaaacuggc agaagucacu aaagucauug uccgaaaucu gaauaaaaua 1440

auugauauaa acuacuaccc uauuccagag gcacacuuau caaauaaacg ccaucggccc 1500

auuggaauug ggguacaagg uuuagaagau gcuuucaucc ugaugagaua ccccuuugag 1560

agcccagaag cccaguuauu aaauaagcag aucuuugaaa ccauuuacua uggagcccug 1620

gaagccagcu gugaacuagc caaggaguau ggccccuaug aaacguauga gggaucucca 1680

gucagcaagg guauucuuca guaugacaug uggaauguug cuccuacaga ccugugggac 1740

uggaagccuc ucaaggagaa gauugcaaag uaugguauaa ggaacaguuu acuuauugcc 1800

ccaaugccua cugcuucaac ugcccagauu cuggggaaua augaguccau ugagccuuau 1860

accaguaaca ucuacacucg aagagucuug ucaggggaau uucagauugu gaauccucac 1920

uuacugaaag aucuuacuga gcggggcuug uggaaugaag agaugaaaaa ucagauuauu 1980

gcaugcaaug gcuccauuca gagcauacca gaaauuccug augaccugaa gcaacucuau 2040

aagaccgugu gggaaaucuc ucagaagacu guucucaaga uggcagccga gagaggugcu 2100

uucaucgauc agagccaguc uuuaaacauc cauauugcug agcccaacua uggcaaacuc 2160

acuaguaugc acuucuacgg uuggaagcag gguuuaaaga cuggaaugua uuacuuaagg 2220

acgaggccug ccgcuaaucc aauccaguuc acucugaaca aggaaaaacu gaaagauaag 2280

gaaaaggcac ugaaggagga ggaggagaag gagaggaaca cagcagccau ggugugcucu 2340

uuggagaaca gagaggagug ucugaugugu ggauccugaa acgccauuau cacacuaaau 2400

auuuaaaaca ggugagguuc uugggagccu uuaaauaagg cacgcgguga augccaauuu 2460

aaacuauaca accuacugcc uucccuuuaa auuuguucgu ucggcucgcg uga 2513

<210> 28
<211> 2512

<212> RNA
<213> Artificial Sequence

<220>
<223> RR1 mRNA with binding sequences for miRNA-122, miRNA-125 ,
      miRNA-375, miRNA-192, miRNA-143

<400> 28
augcauguga ucaagcgaga uggccgccaa gagcgaguua uguuugacaa aauuacauca      60

cgaauccaga aacucuguua uggacucaac auggacuuug uugauccugc ucagaucacc     120

augaaaguaa uccaaggccu auauaguggg gucaccacag uggaacugga cacccuggcu     180

gcugagacag ccgcgaccuu gaccacgaag cacccugacu augccauccu ggcagcaagg     240

auagccgucu cuaacuugca caaagaaaca aagaaagugu ucagugaugu gauggaggau     300

cucuacaacu acauaaaucc gcacaacggc agacacucuc ccaugguggc cagcucaaca     360

cucgacauug uuauggccaa uaaggaucgc cugaauucug ccauuaucua ugaccgagau     420

uucucuuaua acuacuuugg cuuuaagaca cuggaacggu cauauuuguu gaagaucaau     480

gguaaagugg cugaaagacc acagcauaug uugaugaggg uuucuguggg gauucacaaa     540

gaagauauug augcugcaau ugaaaccuac aaccuacuuu cugagaagug guucacucau     600

gccucuccua cucucuucaa ugcugggacc aaccgcccac agcugucuag cuguuuccuc     660

uugaguauga aagaugacag cauugaagga auuuaugaua cucugaagca gugugccuug     720

auuucuaagu ccgcuggggg aauugguguu gcugugaguu guauucgggc cacugguagc     780

uacaucgcug ggacuaaugg caauucuaau ggccuugugc caaugcugag aguauauaac     840

aacacagcuc gcuaugugga ucaaggugga aacaagcgcc caggcgcguu ugcuauuuac     900

cuggagccuu ggcacuuaga caucuuugag uuccuugacu ugaagaagaa cacaggcaag     960

gaagaacagc gagcacgcga ucucuucuuu gcacuuugga ucccagaucu cuucaugaag    1020

cgaguggaga cuaaccagga cuggucauug auguguccca augagugucc uggucuggac    1080

gaggucuggg gagaggaguu ugagaaguua uaugaaaguu acgagaagca gggucguguc    1140

cgaaaaguug uaaaagcuca gcagcuuugg uaugccauca uugaguccca gacggagacc    1200

gguaccccau acaugcucua caaagauucc uguaaccgga agagcaacca gcagaaccug    1260

ggaaccauca aaugcagcaa ccuguguaca gaaauaguag aguacaccag uaaagaugag    1320

guugcaguuu guaacuuggc uucucuggcu cugaauaugu augucacacc ggaacauacg    1380

uaugacuuug agaaacuggc agaagucacu aaagucauug uccgaaaucu gaauaaaaua    1440

auugauauaa acuacuaccc uauuccagag gcacacuuau caaauaaacg ccaucggccc    1500

auuggaauug ggguacaagg uuuagaagau gcuuucaucc ugaugagaua ccccuuugag    1560

agcccagaag cccaguuauu aaauaagcag aucuuugaaa ccauuuacua uggagcccug    1620

gaagccagcu gugaacuagc caaggaguau ggccccuaug aaacguauga gggaucucca 1680

gucagcaagg guauucuuca guaugacaug uggaauguug cuccuacaga ccugugggac 1740

uggaagccuc ucaaggagaa gauugcaaag uaugguauaa ggaacaguuu acuuauugcc 1800

ccaaugccua cugcuucaac ugcccagauu cuggggaaua augaguccau ugagccuuau 1860

accaguaaca ucuacacucg aagagucuug ucaggggaau uucagauugu gaauccucac 1920

uuacugaaag aucuuacuga gcggggcuug uggaaugaag agaugaaaaa ucagauuauu 1980

gcaugcaaug gcuccauuca gagcauacca gaaauuccug augaccugaa gcaacucuau 2040

aagaccgugu gggaaaucuc ucagaagacu guucucaaga uggcagccga gagaggugcu 2100

uucaucgauc agagccaguc uuuaaacauc cauauugcug agcccaacua uggcaaacuc 2160

acuaguaugc acuucuacgg uuggaagcag gguuuaaaga cuggaaugua uuacuuaagg 2220

acgaggccug ccgcuaaucc aauccaguuc acucugaaca aggaaaaacu gaaagauaag 2280

gaaaaggcac ugaaggagga ggaggagaag gagaggaaca cagcagccau ggugugcucu 2340

uuggagaaca gagaggagug ucugaugugu ggauccugaa acgccauuau cacacuaaau 2400

auuuaaaaca ggugagguuc uugggagccu uuaaauuugu ucguucggcu cgcgugauuu 2460

aaacugccaa uuccauaggu cacaguuuaa augagaugaa gcacuguagc uc 2512

<210> 29
<211> 2454
<212> RNA
<213> Artificial Sequence

<220>
<223> IL-12 coding sequence with linked binding sites for
      miRNA-122-miRNA125a-miRNA124a-let-7-miRNA375

<400> 29
gcccagagca agauguguca ccagcaguug gucaucucuu gguuuucccu gguuuuucug 60

gcaucucccc ucguggccau augggaacug aagaaagaug uuuaugucgu agaauuggau 120

ugguauccgg augccccugg agaaauggug guccucaccu gugacacccc ugaagaagau 180

gguaucaccu ggaccuugga ccagagcagu gaggucuuag gcucuggcaa aacccugacc 240

auccaaguca aagaguuugg agaugcuggc caguacaccu gucacaaagg aggcgagguu 300

cuaagccauu cgcuccugcu gcuucacaaa aaggaagaug gaauuugguc cacugauauu 360

uuaaaggacc agaaagaacc caaaaauaag accuuucuaa gaugcgaggc caagaauuau 420

ucuggacguu ucaccugcug guggcugacg acaaucagua cugauuugac auucaguguc 480

aaaagcagca gaggcucuuc ugacccccaa ggggugacgu gcggagcugc uacacucucu 540

gcagagagag ucagagggga caacaaggag uaugaguacu caguggagug ccaggaggac 600

agugccugcc cagcugcuga ggagagucug cccauugagg ucauggugga ugccguucac 660

aagcucaagu augaaaacua caccagcagc uucuucauca gggacaucau caaaccugac 720

ccacccaaga acuugcagcu gaagccauua aagaauucuc ggcaggugga ggucagcugg 780

gaguacccug acaccuggag uacuccacau uccuacuucu cccugacauu cugcguucag 840

guccagggca agagcaagag agaaaagaaa gauagagucu ucacggacaa gaccucagcc 900

acggucaucu gccgcaaaaa ugccagcauu agcgugcggg cccaggaccg cuacuauagc 960

ucaucuugga gcgaaugggc aucugugccc ugcaguuagg uucugaucca ggaugaaaau 1020

uuggaggaaa aguggaagau auuaagcaaa auguuuaaag acacaacgga auagacccaa 1080

aaagauaauu ucuaucugau uugcuuuaaa acguuuuuuu aggaucacaa ugauaucuuu 1140

gcuguauuug uauaguuaga ugcuaaaugc ucauugaaac aaucagcuaa uuuauguaua 1200

gauuuuccag cucucaaguu gccaugggcc uucaugcuau uuaaauauuu aaguaauuua 1260

uguauuuauu aguauauuac uguuauuuaa cguuugucug ccaggaugua uggaauguuu 1320

cauacucuua ugaccugauc caucaggauc agucccuauu augcaaaaug ugaauuuaau 1380

uuuauuugua cugacaacuu uucaagcaag gcugcaagua caucaguuuu augacaauca 1440

ggaagaaugc aguguucuga uaccagugcc aucauacacu ugugauggau gggaacgcaa 1500

gagauacuua cauggaaacc ugacaaugca aaccuguuga gaagauccag gagaacaaga 1560

ugcuaguucc caugucugug aagacuuccu ggagauggug uugauaaagc aauuuagggc 1620

cacuuacacu ucuaagcaag uuuaaucuuu ggaugccuga auuuuaaaag ggcuagaaaa 1680

aaaugauuga ccagccuggg aaacauaaca agaccccguc ucuacaaaaa aaauuuaaaa 1740

uuagccaggc gugguggcuc augcuugugg ucccagcugu ucaggaggau gaggcaggag 1800

gaucucuuga gcccaggagg ucaaggcuau ggugagccgu gauugugcca cugcauacca 1860

gccuagguga cagaaugaga cccugucuca aaaaaaaaaa ugauugaaau uaaaauucag 1920

cuuuagcuuc cauggcaguc cucaccccca ccucucuaaa agacacagga ggaugacaca 1980

gaaacaccgu aagugucugg aaggcaaaaa gaucuuaaga uucaagagag aggacaagua 2040

guuauggcua aggacaugaa auugucagaa uggcaggugg cuucuuaaca gcccugugag 2100

aagcagacag augcaaagaa aaucuggaau cccuuucuca uuagcaugaa ugaaccugau 2160

acacaauuau gaccagaaaa uauggcucca ugaaggugcu acuuuuaagu aauguaugug 2220

cgcucuguaa agugauuaca uuuguuuccu guuuguuuau uuauuuauuu auuuuugcau 2280

ucugaggcug aacuaauaaa aacucuucuu uguaaucuga aacgccauua ucacacuaaa 2340

uauuuaaaac aggugagguu cuugggagcc uuuaaauaag gcacgcggug aaugccaauu 2400

uaaacuauac aaccuacugc cuucccuuua aauuuguucg uucggcucgc guga 2454

<210> 30

<211> 50
<212> RNA
<213> Artificial Sequence

<220>
<223> 2x miR-122 binding sites with linker

<400> 30
aacgccauua ucacacuaaa uauuuaaaaa cgccauuauc acacuaaaua 50

<210> 31
<211> 761
<212> RNA
<213> Artificial Sequence

<220>
<223> mCherry sequence with 2x miR-122 binding sequence and linker

<400> 31
auggugagca agggcgagga ggauaacaug gccaucauca aggaguucau gcgcuucaag 60

gugcacaugg agggcuccgu gaacggccac gaguucgaga ucgagggcga gggcgagggc 120

cgccccuacg agggcaccca gaccgccaag cugaagguga ccaagggugg cccccugccc 180

uucgccuggg acauccuguc cccucaguuc auguacggcu ccaaggccua cgugaagcac 240

cccgccgaca uccccgacua cuugaagcug uccuuccccg agggcuucaa gugggagcgc 300

gugaugaacu ucgaggacgg cggcguggug accgugaccc aggacuccuc ccugcaggac 360

ggcgaguuca ucuacaaggu gaagcugcgc ggcaccaacu uccccuccga cggccccgua 420

augcagaaga agaccauggg cugggaggcc uccuccgagc ggauguaccc cgaggacggc 480

gcccugaagg gcgagaucaa gcagaggcug aagcugaagg acggcggcca cuacgacgcu 540

gaggucaaga ccaccuacaa ggccaagaag cccgugcagc ugcccggcgc cuacaacguc 600

aacaucaagu uggacaucac cucccacaac gaggacuaca ccaucgugga acaguacgaa 660

cgcgccgagg gccgccacuc caccggcggc auggacgagc uguacaagua aaacgccauu 720

aucacacuaa auauuuaaaa acgccauuau cacacuaaau a 761

<210> 32
<211> 1496
<212> RNA
<213> Artificial Sequence

<220>
<223> US3 with 2x miR-122 binding sites and linker

<400> 32
auggccuguc guaaguuuug ucgcguuuac gggggacagg gcaggaggaa ggaggaggcc 60

gucccgccgg agacaaagcc gucccgggug uuuccucaug gccccuuuua uaccccagcc 120

gaggacgcgu gccuggacuc cccgcccccg gagaccccca aaccuuccca caccacacca 180

cccggcgaug ccgagcgccu gugucaucug caggagaucc uggcccagau guacggaaac 240

caggacuacc ccauagagga cgaccccagc gcggaugccg cggacgaugu cgacgaggac 300

gccccggacg acguggccua uccggaggaa uacgcagagg agcuuuuucu gcccggggac 360

gcgcccgguc cccuuaucgg ggccaacgac cacaucccuc ccccgugugg cgcaucuccc 420

cccgguauac gacgacgcag ccgggaugag auugggccca cgggauuuac cgcggaagaa 480

cuggacgcca uggacaggga ggcggcucga gccaucagcc gcggcggcaa gccccccucg 540

accauggcca agcuggugac uggcaugggc uuuacgaucc acggagcgcu caccccagga 600

ucggaggggu gugucuuuga cagcagccac ccagauuacc cccaacgggu aaucgugaag 660

gcgggguggu acacgagcac gagccacgag gcgcgacugc ugaggcgacu ggaccacccc 720

gcgauccugc cccuccugga ccugcauguc gucuccgggg ucacgugucu gguccucccc 780

aaguaccagg ccgaccugua uaccuaucug aguaggcgcc ugaacccgcu gggacgcccg 840

cagaucgcag cggucucccg gcagcuccua agcgccguug acuacauuca ccgccagggc 900

auuauccacc gcgacauuaa gaccgaaaau auuuuuauua acacccccga ggacauuugc 960

cugggggacu uuggugccgc gugcuucgug caggguuccc gaucaagccc cuuccccuac 1020

ggaaucgccg gaaccaucga caccaacgcc cccgaggucc ugaccgggga uccguauacc 1080

accaccgucg acauuuggag cgccggucug gugaucuucg agacugccgu ccacaacgcg 1140

uccuuguucu cggccccccg cggccccaaa aggggcccgu gcgacaguca gaucacccgc 1200

aucauccgac aggcccaggu ccacguugac gaguuucccc cgcauccaga aucgcgccuc 1260

accucgcgcu accgcucccg cgcggccggg aacaaucgcc cgccguacac ccgaccggcc 1320

uggacccgcu acuacaagau ggacauagac gucgaauauc ugguuugcaa agcccucacc 1380

uucgacggcg cgcuucgccc cagcgccgca gagcugcuuu guuugccgcu guuucaacag 1440

aaaugaaacg ccauuaucac acuaaauauu uaaaaacgcc auuaucacac uaaaua 1496

<210> 33
<211> 3466
<212> RNA
<213> Artificial Sequence

<220>
<223> ICP6 with 2x miRNA-122 binding site sequences

<400> 33
augauggcca gccgcccagc cgcauccucu cccgucgaag cgcgggcccc gguuggggga 60

caggaggccg gcggccccag cgcagccacc cagggggagg ccgccggggc cccucucgcc 120

cacggccacc acguguacug ccagcgaguc aauggcguga uggugcuuuc cgacaagacg 180

cccggguccg cguccuaccg caucagcgau agcaacuuug uccaaugugg uuccaacugc 240

accaugauua ucgacggaga cguggugcgc gggcgccccc aggacccggg ggccgcggca 300

ucccccgcuc ccuucguugc ggugacaaac aucggagccg gcagcgacgg cgggaccgcc 360

gucguugcau ucgggggaac cccacgucgc ucggcgggga cgucuaccgg uacccagacg 420

gccgacgucc cagccgaggc ccuugggggc cccccuccuc cuccccgcuu cacccugggu 480

ggcggcuguu gcuccugucg cgacacacgg cgccgcucug cgguauucgg gggggagggg 540

gaucccgucg gccccgcgga guucgucucg gacgaccggu cguccgauuc cgacucggau 600

gacucggagg acaccgacuc ggagacgcug ucacacgccu ccucggacgu guccggcggg 660

gccacguacg acgacgcccu ugacuccgau ucgucaucgg augacucccu gcagauagau 720

ggccccgugu gucgcccgug gagcaaugac accgcgcccc uggauguuug ccccgggacc 780

cccggcccgg gcgccgacgc cggugguccc ucagcgguag acccacacgc gccgacgaca 840

ggggccggcg cuggucuugc ggccgauccc gccguggccc gggacgacgc ggaggggcuu 900

ucggaccccc ggccacgucu gggaacgggc acggccuacc ccgucccccu ggaacucacg 960

cccgagaacg cggaggccgu ggcgcgcuuu cugggagaug ccgugaaccg cgaacccgcg 1020

cucaugcugg aguacuuuug ccggugcgcc cgcgaggaaa ccaagcgugu cccccccagg 1080

acauucugca gccccccucg ccucacggag gacgacuuug ggcuucucaa cuacgcgcuc 1140

guggagaugc agcgccugug ucuggacguu ccuccggucc cgccgaacgc auacaugccc 1200

uauuaucuca gggaguaugu gacgcggcug gucaacgggu ucaagccgcu ggugagccgg 1260

uccguucgcc uuuaccgcau ccuggggguu cuggugcacc ugcggauccg gacccgggag 1320

gccuccuuug aggaguggcu gcgauccaag gaaguggccc uggacuuugg ccugacggaa 1380

aggcuucgcg agcacgaagc ccagcuggug auccuggccc aggcucugga ccauuacgac 1440

ugucugaucc acagcacacc gcacacgcug gucgagcggg ggcugcaauc ggcccugaag 1500

uaugaggagu uuuaccuaaa gcgcuuuggc gggcacuaca uggaguccgu cuuccagaug 1560

uacacccgca ucgccggcuu uuuggccugc cgggccacgc gcggcaugcg ccacaucgcc 1620

cuggggcgag aggggucgug gugggaaaug uucaaguucu uuuuccaccg ccucuacgac 1680

caccagaucg uaccgucgac ccccgccaug cugaaccugg ggacccgcaa cuacuacacc 1740

uccagcugcu accugguaaa cccccaggcc accacaaaca aggcgacccu gcgggccauc 1800

accagcaacg ucagugccau ccucgcccgc aacgggggca ucgggcuaug cgugcaggcg 1860

uuuaacgacu ccggccccgg gaccgccagc gucaugcccg cccucaaggu ccucgacucg 1920

cugguggcgg cgcacaacaa agagagcgcg cguccgaccg gcgcgugcgu guaccuggag 1980

ccguggcaca ccgacgugcg ggccgugcuc cggaugaagg ggguccucgc cggcgaagag 2040

gcccagcgcu gcgacaauau cuucagcgcc cucuggaugc cagaccuguu uuucaagcgc 2100

cugauucgcc accuggacgg cgagaagaac gucacaugga cccuguucga ccgggacacc 2160

agcaugucgc ucgccgacuu ucacggggag gaguucgaga agcucuacca gcaccucgag 2220

gucauggggu ucggcgagca gauacccauc caggagcugg ccuauggcau ugugcgcagu 2280

gcggccacga ccgggagccc cuucgucaug uucaaagacg cggugaaccg ccacuacauc 2340

uacgacaccc agggggcggc caucgccggc uccaaccucu gcaccgagau cguccauccg 2400

gccuccaagc gauccagugg ggucugcaau cugggaagcg ugaaucuggc ccgaugcguc 2460

uccaggcaga cguuugacuu ugggcggcuc cgcgacgccg ugcaggcgug cgugcugaug 2520

gugaacauca ugaucgacag cacgcuacaa cccacgcccc agugcacccg cggcaacgac 2580

aaccugcggu ccaugggaau cggcaugcag ggccugcaca cggccugccu gaagcugggg 2640

cuggaucugg agucugccga auuucaggac cugaacaaac acaucgccga ggugaugcug 2700

cugucggcga ugaagaccag caacgcgcug ugcguucgcg gggcccgucc cuucaaccac 2760

uuuaagcgca gcauguaucg cgccggccgc uuucacuggg agcgcuuucc ggacgcccgg 2820

ccgcgguacg agggcgagug ggagaugcua cgccagagca ugaugaaaca cggccugcgc 2880

aacagccagu uugucgcgcu gaugcccacc gccgccucgg cgcagaucuc ggacgucagc 2940

gagggcuuug ccccccuguu caccaaccug uuuagcaagg ugacccggga cggcgagacg 3000

cugcgcccca acacgcuccu gcuaaaggaa cuggaacgca cguuuagcgg gaagcgccuc 3060

cuggagguga uggacaguuc gacgccaagc agugguccgu ggcgcaggcg cucccgugcc 3120

uggagcccac ccacccccuc cggcgauuca agaccgcguu ugacuacgac cagaaguugc 3180

ugaucgaccu gugugcggac cgcgcccccu acgucgacca uagccaaucc augacccugu 3240

augucacgga gaaggcggac gggacccucc cagccuccac ccugguccgc cuucuggucc 3300

acgcauauaa gcgcggacua aaaacaggga uguacuacug caagguucgc aaggcgacca 3360

acagcggggu cuuuggcggc gacgacaaca uugucugcac gagcugcgcg cuguagaacg 3420

ccauuaucac acuaaauauu uaaaaacgcc auuaucacac uaaaua 3466

## Description

### FIELD

The present invention relates to messenger ribonucleic acid (mRNA) delivery technologies, and methods of using these mRNA delivery technologies in a variety of therapeutic, diagnostic and prophylactic indications. Such delivery systems may be used as stand-alone interventions, or in combination with other therapeutic components.

### BACKGROUND

Gene therapy is the process of introducing coding polynucleotides into the cells of a patient in order to treat disease. For example, a mutated and/or functionless gene can be replaced in target cells by an intact copy. Gene therapy often relies on viral vectors to introduce coding polynucleotides into target cells, but other techniques exist to deliver polynucleotides to cells without the use of viruses. The advantages of viruses include relatively high possible transfection rates, as well as the ability to target the virus to particular cell types by control of the binding proteins by which viruses enter a target cell. In contrast, non-viral methods of introducing coding polynucleotides into cells can have problems with low transfection rates, as well as having limited options for targeting expression to particular organs and cell types. However, the nature of viral intervention carries risks of toxicity and inflammation, but also has limited control over the duration and degree of the expression of the introduced factor.

Tumour therapies based upon biological approaches have advantages over traditional chemotherapeutics because they can employ numerous diverse mechanisms to target and destroy cancers more precisely - e.g. via direct cell lysis, cytotoxic immune effector mechanisms and vascular collapse amongst others. As a result, there has been a significant increase in the number of clinical studies into the potential of such approaches. However due to the diverse range of therapeutic activities, pre-clinical and clinical study is complex, as multiple parameters may affect their therapeutic potential and, hence, defining reasons for treatment failure or methodologies that might enhance the therapeutic activity can be difficult. Maintaining on-target activities, tumour specificity and reducing side effects is also a major challenge for such experimental and powerful therapies.

In non-clinical contexts, too, the ability to induce expression of a particular gene product such as a polypeptide in a particular target tissue or organ is frequently desired. In many situations, a target tissue or organ, will comprise more than one type of cell, and in such cases it is also frequently desired to express the gene product to different degrees in the different cell types - that is, to provide differential expression in the different cell types. While methods exist to introduce polynucleotides *in vitro* and *in vivo*, they have the same limitations as discussed above.

WO-2017/132552-A1 describes recombinant oncolytic virus with an engineered genome that includes micro-RNA binding sites.

US-2013/156849-A1 relates to methods for expressing a polypeptide of interest in a mammalian cell or tissue, the method comprising, contacting said mammalian cell or tissue with a formulation comprising a modified mRNA encoding the polypeptide of interest. WO-2016/011306-A2 describes design, preparation, manufacture and/or formulation of nucleic acids comprising at least one terminal modification that may comprise a micro-RNA binding site. The aforementioned prior art do not address the problems of ensuring effective protection of single or multiple organ types in the body of a subject who is treated with a co-administered therapeutic agent or factor.

There is therefore a need to further develop methods and compositions for delivery of polynucleotide sequences, such as mRNA, to specific organs and/or tissues, and methods to modulate the expression of the delivered polynucleotide sequences in specific cells.

### SUMMARY

In a first aspect, there is provided an isolated mRNA sequence for expression of one or more polypeptides within one or more target organs. The sequence comprises at least one coding sequence which codes for the at least one polypeptide; at least a first untranslated region (UTR) sequence; and a plurality of micro-RNA (miRNA) binding site sequences. Each of the miRNA binding site sequences is located within, immediately 5' to or immediately 3' to, the first UTR sequence. The miRNA binding site sequences allow for differential expression of the coding sequence in at least a first and a second cell type within the target organ or organs.

The mRNA sequence may comprise greater than two, suitably greater than three, typically greater than four binding site sequences. The plurality of miRNA binding site sequences may comprise at least two substantially similar sequences, and/or the plurality of miRNA binding site sequences may comprise at least two substantially different sequences.

In some embodiments, the plurality of miRNA binding site sequences are substantially complementary to miRNA sequences selected from at least one or more of the group consisting of: miRNA-122; miRNA-125a; miRNA-125b; miRNA-199, miRNA-124a; Let-7; miRNA-148a; miRNA-148b; miRNA-375; miRNA-143; miRNA-145; miRNA192; miRNA194; miRNA-204; miRNA215; miRNA-30b, and miRNA-30c. At least one of the plurality of miRNA binding site sequences may comprises one or more of SEQ ID NOS: 1 to 7, suitably SEQ ID NO: 1.

In some embodiments, the binding site sequences comprise each of SEQ ID NOs: 1, 2, 3, 4 and 5; or comprise each of SEQ ID NOs: 1, 2, 5, 6 and 7.

In some embodiments, the first and second cell types are different selections from the group consisting of non-neoplastic cells, a transformed cell phenotype; a pre-cancerous phenotype; and a neoplastic phenotype. The target organ or organs may be selected from the group consisting of: liver; brain; lung; breast; pancreas; colon and kidney.

In some embodiments, at least one of the one or more polypeptides comprises a therapeutic enhancement factor. The therapeutic enhancement factor may be selected from the group consisting of:
(i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
(ii) dendritic cell activators selected from one or more of: GM-CSF, TLR7 and TLR9;
(iii) molecules targeting the following cellular receptors and their ligands selected from one or more of: CD40, CD40L, CD160, 2B4, Tim-3, GP-2, B7H3 and B7H4;
(iv) TGF β inhibitors;
(v) T-cell membrane protein 3 inhibitors;
(vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
(vii) NF-κB inhibitors.

The mRNA comprises may comprise than one open reading frame (ORF).

In some embodiments, the mRNA comprises a sequence selected from one of the group consisting of: SEQ ID NOs: 18 to 29.

In another aspect, there is provided a pharmaceutical composition comprising the isolated mRNA sequence as described herein, and a delivery particle, the sequence being comprised within the delivery particle, and a pharmaceutically acceptable carrier. The delivery particle may be selected from at least one of the group consisting of: an aminoalcohol lipidoid particle; a liposome; an exosome; a cell-derived vesicle; and a polymeric particle.

In some embodiments, the delivery particle is targeted towards one or more of the target organ or organs. In such cases, the delivery particle may comprise a targeting agent selected from: proteins, peptides, carbohydrates, glycoproteins, lipids, small molecules and nucleic acids; wherein the targeting agents associate preferentially with cells in the target organ or organs.

In a still further aspect, there is provided a polynucleotide expression vector construct encoding the mRNA sequence as described herein.

In yet another aspect, there is provided a viral vector comprising the the mRNA sequence or the polynucleotide expression vector construct as described herein.

Another aspect provides a method for the treatment of cancer, the method comprising administering to a subject in need thereof a composition comprising the isolated mRNA sequence, composition, vector construct, or viral vector as described herein.

The method may further comprise administering a therapy or therapeutic agent to the subject. The therapy or therapeutic agent may be selected from chemotherapy, radiotherapy, a biological agent, an oncolytic virus, a small molecule drug, a CAR-T or adoptive cell therapy, and combinations thereof. In embodiments of the method, the subject is a human, or is a non-human animal.

In certain embodiments, the cancer is selected from at least one of the group consisting of: liver, brain, lung, breast, pancreas, colorectal and kidney cancer, suitably liver cancer. Liver cancer may include primary liver cancer, or secondary liver cancer. Primary liver cancer may be selected from the group consisting of: a hepatocarcinoma; a hepatoblastoma; a cholangiocarcinoma; and a angiosarcoma. Secondary liver cancer may be a metastatic liver cancer from a known or unknown primary solid tumor.

The method may further comprise administering an oncolytic virus to the subject. In such embodiments, the isolated mRNA sequence may code for a therapeutic agent which increases the efficacy of the oncolytic virus. The oncolytic virus may have been attenuated by mutation of one or more virulence genes, and in such embodiments the mRNA sequence may code for the one or more virulence genes, or an equivalent or homologue thereof. In some embodiments, the oncolytic virus is selected from any one of the Groups I - VII of the Baltimore classification of viruses. The oncolytic virus may be selected from the group comprising one or more of: Vesicular Stomatitis Virus, Maraba virus, Polio virus, Reovirus, Measles virus, Newcastle disease virus, Coxsackievirus A21, Parvovirus, Herpes Simplex Virus Type 1, Vaccinia Virus, and Adenovirus. Typically, the oncolytic virus is a Herpes Simplex Virus.

The method as described may in some embodiments further comprise administering a CAR-T or adaptive cell therapy to the subject. In such embodiments, the isolated mRNA sequence, composition, vector construct, or viral vector may encode one or more immunomodulatory molecules selected from the group consisting of:
(i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
(ii) dendritic cell activators selected from one or more of: GM-CSF, TLR7 and TLR9;
(iii) molecules targeting the following cellular receptors and their ligands selected from one or more of: CD40, CD40L, CD160, 2B4, Tim-3, GP-2, B7H3 and B7H4;
(iv) TGF β inhibitors;
(v) T-cell membrane protein 3 inhibitors;
(vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
(vii) NF-κB inhibitors.

The method may in other embodiments further comprise administering a cell checkpoint inhibitor to the subject. Again, in such embodiments, the isolated mRNA sequence, composition, vector construct, or viral vector may encode one or more immunomodulatory molecules selected from the group consisting of:
(i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
(ii) dendritic cell activators selected from one or more of: GM-CSF, TLR7 and TLR9;
(iii) molecules targeting the following cellular receptors and their ligands selected from one or more of: CD40, CD40L, CD160, 2B4, Tim-3, GP-2, B7H3 and B7H4;
(iv) TGF β inhibitors;
(v) T-cell membrane protein 3 inhibitors;
(vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
(vii) NF-κB inhibitors.

In a further aspect, a composition comprising the isolated mRNA sequence, vector construct, or virus as described herein, or a composition described herein, is provided for use in medicine.

In another aspect, a composition comprising the isolated mRNA sequence, vector construct, or virus as described herein, or a composition described herein, is provided for use in the treatment of cancer, suitably wherein the cancer is selected from the group consisting of: liver, brain, lung, breast, pancreas, colorectal, and kidney cancer.

### DRAWINGS

The invention is further illustrated by reference to the accompanying drawings in which:

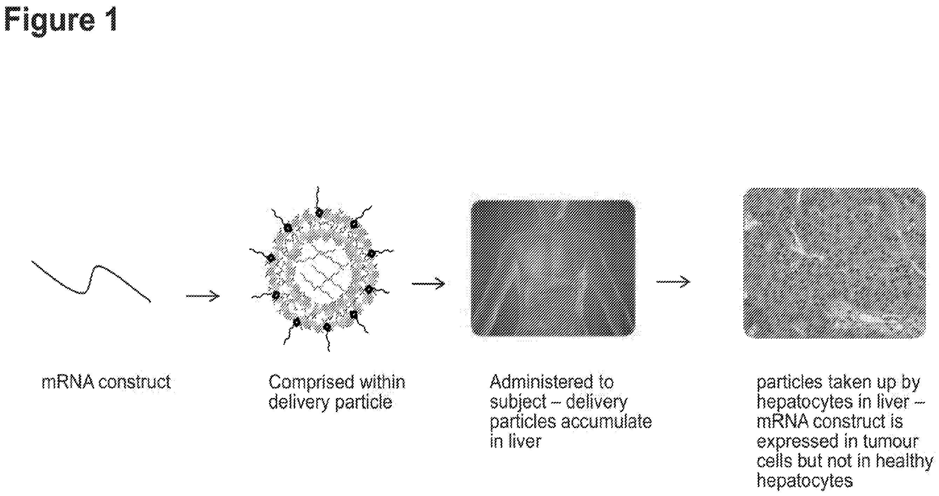

Figure 1 shows a schematic of a method of administration of a lipidoid encapsulated mRNA composition according to one embodiment of the invention.
Figure 2 shows an example of a cloning method to produce DNA synthesis vectors, which vectors were used to produce the mRNA constructs according to embodiments of the invention.
Figure 3 shows three variants of mRNA constructs used in embodiments of the invention, and illustrated in Figure 4, and possible options for the insertion point of a pair of miRNA binding sequences (here sequences that bind to miR-122) within or adjacent to a UTR sequence located 3' to the coding sequence.
Figure 4 shows examples of DNA plasmids, template plasmids, as well as synthesis vectors for producing the mRNA constructs depicted in Figure 3.
Figures 5, 6 and 7 show examples of methods which may be used to produce a synthesis vector for producing the mRNA construct variants as depicted in Figure 3.
Figure 8 shows the chemical formulae of examples of constituent compounds that can be used in the preparation of delivery particles according to an embodiment of the invention.
Figure 9A shows a method of preparation of a nanoformulation of delivery particles comprising mRNA according to an embodiment of the invention.
Figure 9B shows the structure of a cross section of a delivery particle comprising mRNA according to an embodiment of the invention, and further comprising the encapsulating constituent compounds depicted in Figure 8.

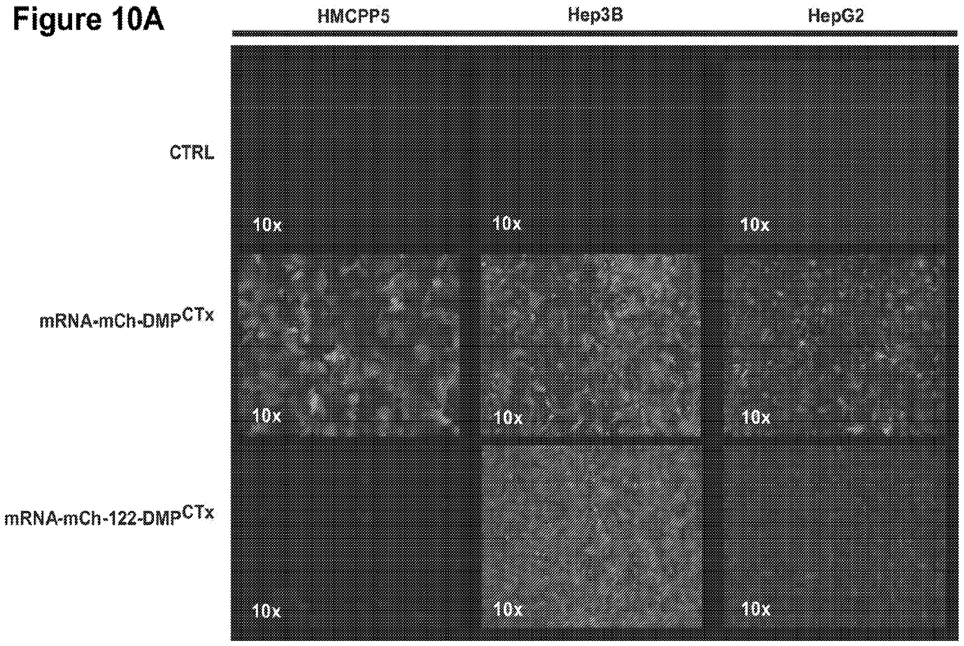

Figure 10A fluorescent microscopy images indicating the results of an experiment where cells from healthy human hepatocyte culture (Human Plateable Hepatocytes, HMCPP5), human hepatocarcinoma (Hep3B) and human hepatoblastoma (HepG2) cells were transfected *in vitro* with compositions according to embodiments of the invention. Two delivery particles were administered: one containing a mRNA encoding the fluorescent protein mcherry (mRNA-mCh-DMP^{CTx}) and one one containing an mRNA encoding the fluorescent protein mcherry but where differential expression is controlled by miRNA-122 content in the the target cells (mRNA-mCh-122-DMP^{CTx}) .
Figure 10B shows a quantification of fluorescence intensity after 48 hours of cells transfected according to the experiment of Figure 10A. Results are shown as means ± SD. Statistical significance was determined using the t test. Asterisks indicate statistically significant difference between mRNA-mCherry, mRNA-mCherry-122 expression in transfected cells (****p < 0.0001, ***p < 0.001).
Figure 11 shows a graph of results from an experiment in which human hepatocytes (HMCPP5) were transfected either multiple times (MPT) or singly (ST) with the delivery particles used in Figure 10A. Expression of mCherry is determined by the level of fluorescence intensity measured at 24, 28, 72, 96 and 144 hours after transfection. Results are shown as means ± SD. Statistical significance was determined using the t test. Asterisks indicate statistically significant difference between mRNA-mCherry, mRNA-mCherry-122 expression in transfected cells (*p < 0.01, **p < 0.05).
Figure 12A shows fluorescent microscopy images indicating the results of an experiment where healthy mice hepatocytes (AML12 cell line) were transfected *in vitro* with the delivery particles used in Figure 10A with relative expression levels of mCherry shown at 24 hours post transfection..
Figure 12B shows a graph providing quantification of fluorescence intensity as % pixels counted for the results of Figure 12A as well as a further post-transfection time point of 72 hours.
Figure 13 shows the results of a Western blot in two experiments (denoted Run 1 and Run 2) where human hepatocytes (HMCPP5), human hepatoblastoma (HepG2) and human hepatocarcinoma (Hep3B) cells were transfected with a composition according to an embodiment of the invention which comprised an mRNA encoding an exemplary human polypetide of 25 kDa molecular mass under miRNA differential expression control.
Figure 14 shows the effect of the Herpes Simplex Virus variant R7041 on the viability of human cells from a model of hepatocarcinoma (Hep3B) and hepatoblastoma (HepG2). The effects of viral application on relative cell viability are shown.
Figure 15 shows a timetable for an *in vitro* experiment where human cells from a model of hepatocarcinoma were treated with a composition and method according to an embodiment of the invention and then tested via MTS colorimetric assay.
Figures 16A and 16B show the results of *in vitro* experiments where human cells from a model of hepatoblastoma (Figure 16 A) and hepatocarcinoma (Figure 16 B) were treated with virus alone or in combination with a composition according to an embodiment of the invention following the timetable of Figure 15. The composition is a delivery particle comprising mRNA coding for US3 (US3 mRNA DMP^{CTx}). The effects of the treatments on cell viability are shown.
Figures 17A and 17B show the results of an *in vivo* experiments using a mouse model of human hepatocarcinoma. Figure 17A shows the tumor growth (Hep3B cells are labelled with luciferase). Figure 17B shows fluorescent microscopy images of the healthy mouse liver using mRNA coding for mCherry - no fluorence was detected when using the mCherry-DMP^{CTx}-miRNA122 compositon.
Figure 18 shows an immunohistochemistry micrograph result of an in vivo experiment using the same mouse model as shown in Figure 17A. A delivery particle comprising mRNA coding for US3 (US3 mRNA DM^{pCTx} miRNA-122) is administered via the tail vein and provides differential expression between non-diseased hepatocytes and tumoural liver tissue as evidenced by darker staining for US3 protein in the tumoural tissue. The boundary between the tumour tissue and the non-diseased tissue is shown with a dashed line.
Figure 19A shows examples of miRNA binding site sequences which can be used in mRNA constructs according to the present invention.
Figure 19B shows general schematics for examples of mRNA constructs according to some embodiments of the invention, where up to five binding sites are included.
Figures 19C and 19D show examples of combinations of binding site sequences which can be used in mRNA constructs according to the invention.
Figure 19E shows some specific combinations of coding sequences, including coding sequences for multiple polypeptides, and binding sites according to some embodiments.

### DETAILED DESCRIPTION

Unless otherwise indicated, the practice of the present invention employs conventional techniques of chemistry, molecular biology, microbiology, recombinant DNA technology, and chemical methods, which are within the capabilities of a person of ordinary skill in the art. Such techniques are also explained in the literature, for example, M.R. Green, J. Sambrook, 2012, Molecular Cloning: A Laboratory Manual, Fourth Edition, Books 1-3, Cold Spring Harbor Laboratory Press, Cold Spring Harbor, NY; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridisation: Principles and Practice, Oxford University Press; M. J. Gait (Editor), 1984, Oligonucleotide Synthesis: A Practical Approach, IRL Press; and D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

Prior to setting forth the invention, a number of definitions are provided that will assist in the understanding of the invention. All references cited herein are incorporated by reference in their entirety. Unless otherwise defined, all technical and scientific terms used herein have the same meaning as commonly understood by one of ordinary skill in the art to which this invention belongs.

As used herein, the term 'comprising' means any of the recited elements are necessarily included and other elements may optionally be included as well. 'Consisting essentially of' means any recited elements are necessarily included, elements that would materially affect the basic and novel characteristics of the listed elements are excluded, and other elements may optionally be included. 'Consisting of means that all elements other than those listed are excluded. Embodiments defined by each of these terms are within the scope of this invention.

The term 'isolated', when applied to a polynucleotide sequence, denotes that the sequence has been removed from its natural organism of origin and is, thus, free of extraneous or unwanted coding or regulatory sequences. The isolated sequence is suitable for use in recombinant DNA processes and within genetically engineered protein synthesis systems. Such isolated sequences include cDNAs, mRNAs and genomic clones. The isolated sequences may be limited to a protein encoding sequence only, or can also include 5' and 3' regulatory sequences such as promoters and transcriptional terminators. Prior to further setting forth the invention, a number of definitions are provided that will assist in the understanding of the invention.

A 'polynucleotide' is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Polynucleotides include DNA and RNA, and may be manufactured synthetically in vitro or isolated from natural sources. Sizes of polynucleotides are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called 'oligonucleotides'. The term 'nucleic acid sequence' as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acid sequences may include DNA and RNA, and may be manufactured synthetically in vitro or isolated from natural sources. Sizes of nucleic acid sequences, also referred to herein as 'polynucleotides' are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called 'oligonucleotides' and may comprise primers for use in manipulation of DNA such as via polymerase chain reaction (PCR).

The term 'nucleic acid' as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acids may include DNA and RNA, and may be manufactured synthetically in vitro or isolated from natural sources. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as 'polynucleotides' are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 100 nucleotides in length are typically called 'oligonucleotides' and may comprise primers for use in manipulation of DNA such as via polymerase chain reaction (PCR). In specific embodiments of the present invention the nucleic acid sequence comprises messenger RNA (mRNA).

According to the present invention, homology to the nucleic acid sequences described herein is not limited simply to 100% sequence identity. Many nucleic acid sequences can demonstrate biochemical or functional equivalence to each other despite having apparently low sequence identity. In the present invention homologous nucleic acid sequences are considered to be those that will hybridise to each other under conditions of low stringency (Sambrook J. et al, supra). In this regard, the term "substantially similar", relating to two sequences, means that the sequences have at least 70%, 80%, 90%, 95% or 100% similarity. Likewise, the term "substantially complementary", relating to two sequences, means that the sequences are completely complementary, or that at least 70%, 80%, 90%, 95% or 99% of the bases are complementary. That is, mismatches can occur between the bases of the sequences which are intended to hybridise, which can occur between at least 1%, 5%, 10%, 20% or up to 30% of the bases.

The term 'operatively linked', when applied to nucleic acid sequences, for example in an expression construct, indicates that the sequences are arranged so that they function cooperatively in order to achieve their intended purposes. By way of example, in a DNA vector a promoter sequence allows for initiation of transcription that proceeds through a linked coding sequence as far as a termination sequence. In the case of RNA sequences, one or more untranslated regions (UTRs) may be arranged in relation to a linked protein coding sequence referred to as an open reading frame (ORF). A given mRNA may comprise more than one ORFs, a so-called polycistronic RNA. A UTR may be located 5' or 3' in relation to an operatively linked coding sequence ORF. UTRs may comprise sequences typically found in mRNA sequences found in nature, such as Kozak consensus sequences, initiation codons, cis-acting regulatory elements, poly-A tails, internal ribosome entry sites (IRES), structures regulating mRNA longevity, sequences directing the localisation of the mRNA, and so on. A mRNA may comprise multiple UTRs that are the same or different.

The term 'expressing a polypeptide' in the context of the present invention refers to production of a polypeptide for which the polynucleotide sequences described herein code. Typically, this involves translation of the supplied mRNA sequence by the ribosomal machinery of the cell to which the sequence is delivered.

The term 'delivery particle' as used herein refers to particles which can comprise therapeutic components by encapsulation, holding within a matrix, the formation of complex or by other means, and deliver a therapeutic component such as a coding nucleic acid sequence into a target cell. Delivery particles may on the micro- scale, but in specific embodiments may typically be on the nanoscale - i.e. nanoparticles. Nanoparticles are typically sized at least 50 nm (nanometres), suitably at least approximately 100 nm and typically at most 150nm, 200 nm, although optionally up to 300 nm in diameter. In one embodiment of the invention the nanoparticles have a mean diameter of approximately at least 60 nm. An advantage of these sizes is that this means that the particles are below the threshold for reticuloendothelial system (mononuclear phagocyte system) clearance, i.e. the particle is small enough not to be destroyed by phagocytic cells as part of the body's defence mechanism. This facilitates the use of intravenous delivery routes for the compositions of the invention.

Alternative possibilities for the composition of the nanoparticles include polylactic acid (PLA), poly(lactic-co-glycolic acid) (PLGA), a lipid- or phospholipid-based particles such as liposomes; particles based on proteins and/or glycoproteins such as collagen, albumin, gelatin, elastin, gliadin, keratin, legumin, zein, soy proteins, milk proteins such as casein, and others (Lohcharoenkal et al. BioMed Research International; Volume 2014 (2014)); and particles based on metals or metallic compounds such as gold, silver, aluminium, copper oxides and so on.

In particular, polymers comprising polyethyleneimine (PEI) have been investigated for the delivery of nucleic acids. Nanoparticle vectors composed of poly(β-amino esters) (PBAEs) have also been shown to be suitable for nucleic acid delivery, especially in coformulation with polyethylene glycol (PEG) (Kaczmarek JC et al Angew Chem Int Ed Engl. 2016; 55(44): 13808-13812). Particles of such coformulations have been used to deliver mRNA to the lung.

Also considered are particles based on polysaccharides and their derivatives, such as cellulose, chitin, and chitosan. Chitosan is a cationic linear polysaccharide obtained by partial deacetylation of chitin, with nanoparticles comprising this substance possessing promising properties for drug delivery such as biocompatibility, low toxicity and small size (Felt et al., Drug Development and Industrial Pharmacy, Volume 24, 1998 - Issue 11). It is envisioned that combinations between the above constituents may be used.

US2010/0331234, US2011/0293703 and US2015/0203439 - which are incorporated herein by reference - describe the production of aminoalcohol lipidoids by reacting an amine with an epoxide-terminated compound. Complexes, micelles, liposomes and particles, including nanoparticles, may be prepared with these lipidoids and their chemical structure makes them particularly suited to the delivery of a 'cargo' - e.g. nucleic acids such as coding mRNAs - to target cell types within the body of a human or animal subject. Delivery platforms comprising aminoalcohol lipidoid compounds are particularly suitable for use in the delivery of net negatively charged cargo molecules given the tertiary amines available for protonation thus forming a cationic moiety. For example, aminoalcohol lipidoid compounds may be used in the preparation of particulate compositions to deliver DNA, RNA, or other polynucleotide cargoes to a subject or to a target cell or tissue. Suitable particles may be in the form of microparticles, nanoparticles, liposomes, exosomes, or micelles.

The aminoalcohol lipidoid based delivery particles possess tertiary amines that are available to interact with a polynucleotide cargo, such as a coding mRNA. Polynucleotides, or derivatives thereof, are contacted with the aminoalcohol lipidoid compounds under conditions suitable to form polynucleotide/lipidoid complexes. The lipidoid is preferably at least partially protonated so as to form a complex with the negatively charged polynucleotide. In this way, the polynucleotide/lipidoid complexes form particles that are useful in the delivery of cargo polynucleotides to cells and tissues. In certain embodiments, multiple aminoalcohol lipidoid molecules may be associated with a polynucleotide molecule. The complex may include at least 1, at least 5, at least 10, at least 20, at least 50, or suitably at least 100 aminoalcohol lipidoid molecules. The complex may include at most 10,000, at most 5000, at most 2000, at most 1000, at most 500, or typically at most 100 aminoalcohol lipidoid molecules.

Those of ordinary skill in the art will appreciate that a population of particles follow principles of particle size distribution. Widely used, art-recognized methods of describing particle size distributions include, for example, average diameters and D values, such as the D50 value, which is commonly used to represent the mean diameter of the range of the particle sizes of a given sample. In certain embodiments, the diameter of the nanoparticles particles ranges from 10-500 nm, more suitably the diameter of the particles ranges from 10-1200 nm, and particularly from 50-150 nm. In some embodiments, the nanoparticles have average diameters of at least about 10 nm, suitably at least about 30 nm. In some embodiments, nanoparticles have average diameters of less than about 150 nm in average diameter and greater than 50 nm in average.

The particles may be further associated with a targeting agent at facilitates binding of the delivery particle to a target cell type. The term 'targeted' as used herein in relation to refers to an object, or composition such as comprising a delivery particle, which is intended to associate with and facilitate transfection of cells within a particular organ, tissue or cell type within the body. In a particular embodiment, a delivery particle - such as a delivry nanoparticle - may be targeted to deliver its cargo only to a certain organ, tissue or cell type. Targeting may be geographical, for example by the delivery of the targeted object directly to a particular tissue, or may be mediated chemically, through targeting agents or binding moieties which preferentially associate with target cells or tissues.

A variety of targeting agents that direct pharmaceutical compositions to particular cells are known in the art (see, for example, Cotten et al. Methods Enzym. 217:618, 1993; Wagner et al. Advanced Drug Delivery Reviews, Volume 14, Issue 1, April-May 1994, 113-135; Fiume et al. Advanced Drug Delivery Reviews, Volume 14, Issue 1, April-May 1994, 51-65). The targeting agents may be included throughout the particle or may be localised only on the surface. The targeting agent may be a protein, peptide, carbohydrate, glycoprotein, lipid, small molecule, nucleic acids, etc. The targeting agent may be used to target specific cells or tissues or may be used to promote endocytosis or phagocytosis of the particle. Examples of targeting agents include, but are not limited to, antibodies, fragments of antibodies, low-density lipoproteins (LDLs), transferrin, asialoglycoproteins, gp120 envelope protein of the human immunodeficiency virus (HIV), carbohydrates, receptor ligands, sialic acid, aptamers etc. If the targeting agent is distributed throughout the particle, the targeting agent may be included in the mixture or composite that is used to form the particles. If the targeting agent is only located on the surface, the targeting agent may be associated with the formed particles using standard chemical techniques e.g. by covalent binding, hydrophobic, hydrogen bonding, van der Waals, biotin-avidin linkage, or other interactions.

The particulate compositions of certain embodiments of the invention may suitably deliver the encapsulated mRNA cargo over a period of time that may be controlled by the particular choice or formulation of the encapsulating biodegradable non-toxic polymer or biocompatible material. For example, the particulate compositions may release the encapsulated mRNA cargo over at least 30 minutes, at least 1 hour, at least 2 hours, at least 6 hours, at least 12 hours, or at least 1 day. The particulate compositions may release the encapsulated mRNA cargo over at most 2 days, at most 3 days, or at most 7 days.

The term 'diseased' as used herein, as in 'diseased cells' and/or 'diseased tissue' indicates tissues and organs (or parts thereof) and cells which exhibit an aberrant, non-healthy or disease pathology. For instance, diseased cells may be infected with a virus, bacterium, prion or eukaryotic parasite; may comprise deleterious mutations; and/or may be cancerous, precancerous, tumoural or neoplastic. Diseased cells may comprise an altered intra-cellular miRNA environment when compared to otherwise normal or so-called healthy cells. In certain instances disease cells may be pathologically normal but comprise an altered intra-cellular miRNA environment that represents a precursor state to disease. Diseased tissues may comprise healthy tissues that have been infiltrated by diseased cells from another organ or organ system. By way of example, many inflammatory diseases comprise pathologies where otherwise healthy organs are subjected to infiltration with immune cells such as T cells and neutrophils. By way of a further example, organs and tissues subjected to stenotic or cirrhotic lesions may comprise both healthy and diseased cells in close proximity.

The term 'cancer' as used herein refers to neoplasms in tissue, including malignant tumours which may be primary cancer starting in a particular tissue, or secondary cancer having spread by metastasis from elsewhere. The terms cancer, neoplasm and malignant tumours are used interchangeably herein. Cancer may denote a tissue or a cell located within a neoplasm or with properties associated with a neoplasm. Neoplasms typically possess characteristics that differentiate them from normal tissue and normal cells. Among such characteristics are included, but not limited to: a degree of anaplasia, changes in morphology, irregularity of shape, reduced cell adhesiveness, the ability to metastasize, and increased cell proliferation. Terms pertaining to and often synonymous with 'cancer' include sarcoma, carcinoma, malignant tumour, epithelioma, leukaemia, lymphoma, transformation, neoplasm and the like. As used herein, the term 'cancer' includes premalignant, and/or precancerous tumours, as well as malignant cancers.

The term 'healthy' as used herein, as in 'healthy cells' and/or 'healthy tissue' indicates tissues and organs (or parts thereof) and cells which are not themselves diseased and approximate to a typically normal functioning phenotype. It can be appreciated that in the context of the invention the term 'healthy' is relative, as, for example, non-neoplastic cells in a tissue affected by tumours may well not be entirely healthy in an absolute sense. Therefore non-healthy cells' is used mean cells which are not themselves neoplastic, cancerous or pre-cancerous but which may be cirrhotic, inflamed, or infected, or otherwise diseased for example. Similarly, 'healthy or non-healthy tissue' is used to mean tissue, or parts thereof, without tumours, neoplastic, cancerous or pre-cancerous cells; or other diseases as mentioned above; regardless of overall health. For instance, in the context of an organ comprising cancerous and fibrotic tissue, cells comprised within the fibrotic tissue may be thought of as relatively 'healthy' compared to the cancerous tissue.

In an alternative embodiment, the health status of a cell, cell type, tissue and/or organ is determined by the quantification of miRNA expression. In certain disease types, such as cancer, the expression of particular miRNA species is affected, and can be up- or down-regulated compared to unaffected cells. This difference in the miRNA transcriptome can be used to identify relative states of health, and/or to track the progression of healthy cells, cell types, tissues and/or organs towards a disease state. The disease state may include the various stages of transformation into a neoplastic cell. In embodiments of the present invention the differential variations in the miRNA transcriptome of cell types comprised within a given organ or organ system is leveraged in order to control protein expression in the different cell types.

As used herein, the term 'organ' is synonymous with an 'organ system' and refers to a combination of tissues and/or cell types that may be compartmentalised within the body of a subject to provide a biological function, such as a physiological, anatomical, homeostatic or endocrine function. Suitably, organs or organ systems may mean a vascularized internal organ, such as a liver or pancreas. Typically organs comprise at least two tissue types, and/or a plurality of cell types that exhibit a phenotype characteristic of the organ.

The term 'therapeutic virus' as used herein refers to a virus which is capable of infecting and killing cancer cells, sometimes by direct viral lysis (oncolysis), but also including indirect killing by the stimulation of host anti-tumoural responses. Oncolytic viruses are frequently characterised by having increased activity in diseased cells, including cancer cells, compared with healthy cells.

Examples of oncolytic viruses include those provided in Table 1, and subtypes thereof.

**Table 1**

| **Oncolytic virus** | **Type** |
|---|---|
| Vesicular Somatitis Virus | Enveloped RNA |
| Vaccinia Virus | Enveloped DNA |
| Maraba virus | Enveloped rhabdovirus |
| Polio virus | Non enveloped RNA |
| Reovirus | Non enveloped RNA |
| Measles virus | Enveloped RNA |
| Newcastle disease virus | Enveloped RNA |
| Coxsackievirus A21 | Non enveloped RNA |
| Parvovirus | Non enveloped DNA |
| Herpes Simplex Virus Type 1 | Enveloped DNA |
| Adenovirus | Non enveloped DNA |

In embodiments of the invention viruses may be selected from any one of the Groups I - VII of the Baltimore classification of viruses (Baltimore D (1971). "Expression of animal virus genomes". Bacteriol Rev. 35 (3): 235-41). In specific embodiments of the invention suitable viruses may be selected from Baltimore Group I, which are characterised as having double stranded DNA viral genomes; Group IV, which have single stranded positive RNA genomes; and Group V, which have single stranded negative RNA genomes.

The term 'virulence gene' or 'virulence factor' as used herein refers to a gene or gene product which aids in the replication of a therapeutic virus such as an oncolytic virus within or lysis of the cells which it infects. The term 'replication factor' is used as a synonymous term herein. Virulence factors may typically be viral genes encoded by the viral genome. Virulence factors may be involved in functions such as intracellular immune system suppression and evasion, viral genome replication, the spread or transmission of virions, the production or assembly of structural coat proteins, the activation of viruses in a latent state, the prevention of viral latency, and the takeover of host cell processes. Several virulence factors have cellular or other equivalents which can compensate for the function of these genes if lacking in the virus genome. Some viruses can be modified with exogenous virulence genes which increase their ability to replicate, lyse cells, and spread.

In specific embodiments of the present invention the mRNA sequences enhance or sustain the oncolytic potency of a co-administered virus in a tumor located within an organ through differential expression of one or more proteins or polypeptides that enhance virion replication preferentially in the tumor. In this way the mRNA may code for one or more factors that enhance the biological activity of the oncolytic virus by: increased replication and/or increased direct oncolytic effect and/or increased viral progeny and/or an adaptive antitumor immune response In further embodiments of the invention the compositions may encode a gene product that controls the interaction between host immune cells and oncolytic virus within a tumour. In yet a further embodiment, the compositions of the invention can be used to produce gene products that modulate differential patterns of oncolytic virus activity as well as expression of immune co-stimulatory molecules that are administered via the virion, exogenously or via a delivery particle of the invention.

The term 'polypeptide' as used herein is a polymer of amino acid residues joined by peptide bonds, whether produced naturally or in vitro by synthetic means. Polypeptides of less than around 12 amino acid residues in length are typically referred to as "peptides" and those between about 12 and about 30 amino acid residues in length may be referred to as "oligopeptides". The term "polypeptide" as used herein denotes the product of a naturally occurring polypeptide, precursor form or proprotein. Polypeptides can also undergo maturation or post-translational modification processes that may include, but are not limited to: glycosylation, proteolytic cleavage, lipidization, signal peptide cleavage, propeptide cleavage, phosphorylation, and such like. The term "protein" is used herein to refer to a macromolecule comprising one or more polypeptide chains.

The term 'gene product' as used herein refers to the product of the at least one coding sequence or ORF comprised within an mRNA construct of the invention as described herein. The gene product(s) may comprise a polypeptide or a protein. A polycistronic mRNA construct may be used which results in the production of multiple gene products. It will be appreciated that multiple ORFs may lead to the production *in situ* of a variety of products that may cooperate functionally, or may form complexes and/or multimeric proteins with diverse biological and potentially therapeutic effects.

Delivery of mRNA directly to cells allows direct and controllable translation of the desired gene products such as polypeptides and/or proteins in the cells. Provision of mRNA specifically allows not only for the use of cell expression modulation mechanisms such as miRNA mediated control (as detailed in specific embodiments below), but also represents a finite and exhaustible supply of the product, rather than the potentially permanent change to the transcriptome of a target cell which an episomal or genomically inserted DNA vector might provide.

In embodiments of the present invention an mRNA sequence is provided that comprises a sequence that codes for at least one polypeptide in operative combination with one or more untranslated regions (UTRs) that may confer tissue specificity, and stability to the nucleic acid sequence as a whole. By 'tissue specificity' it is meant that translation of the protein product encoded by the mRNA is modulated according to the presence of the UTR. Modulation may include permitting, reducing or even blocking detectable translation of the mRNA into a protein product. The UTRs may be linked directly to the mRNA in cis - i.e. on the same polynucleotide strand. In an alternative embodiment, a first sequence that codes for a gene product is provided and a further second sequence, that hybridises to a portion of the first sequence, is provided that comprises one or more UTRs that confer tissue specificity to the nucleic acid sequence as a whole. In this latter embodiment the UTR is operatively linked to the sequence that encodes the gene product in trans.

According to specific embodiments of the invention, an mRNA is provided that comprises such associated nucleic acid sequences operatively linked thereto as are necessary to prevent or reduce expression of a gene product in non-diseased liver tissue, e.g. in healthy hepatocytes. As such, an mRNA construct, or transcript, is provided that comprises a 5' cap and UTRs necessary for ribosomal recruitment and tissue and/or organ specific expression (typically, but not exclusively positioned 3' to the ORF), as well as start and stop codons that respectively define one or more ORFs. When the construct is introduced into a non-diseased liver, lung, pancreas, breast, brain, kidney and/or colon-GI tract, expression of the gene product is prevented or reduced. In contrast, neoplastic or otherwised diseased cells comprised within the aforementioned organs typically do not conform to normal non-diseased cell expression patterns, posessing a quite different miRNA transcriptome. The gene product(s) comprised within the mRNA is translated specifically in these aberrant cells but not in neighboring healthy cells. Delivery of the mRNA construct to the organs mentioned above may be achieved via a particulate delivery platform as described herein, or in any suitable way known in the art. Cell type specific expression can be mediated via microRNA modulation mechanisms such as those described in more detail below.

A 'therapeutic component' or 'therapeutic agent' as defined herein refers to a molecule, substance, cell or organism that when administered to an individual human or other animal as part of a therapeutic intervention, contributes towards a therapeutic effect upon that individual human or other animal. The therapeutic effect may be caused by the therapeutic component itself, or by another component of the therapeutic intervention. The therapeutic component may be a coding nucleic acid component, in particular an mRNA. The coding nucleic acid component(s) may code for therapeutic enhancement factors, as defined below. A therapeutic component may also comprise a drug, optionally a chemotherapeutic drug such as a small molecule or monoclonal antibody (or fragment thereof). In some embodiments, a therapeutic component may comprise a cell, such as a recombinantly modified immune effector cell - e.g. a CAR-T cell. In other embodiments of the invention, the therapeutic agent comprises a therapeutic virus, such as an oncolytic virus or a viral vector.

The term 'therapeutic effect' refers to a local or systemic effect in an animal subject, typically a human, caused by a pharmacologically or therapeutically active agent that comprises a substance, molecule, composition, cell or organism that has been administered to the subject, and the term 'therapeutic intervention' refers to the administration of such a substance, molecule, composition, cell or organism. The term thus means any agent intended for use in the diagnosis, cure, mitigation, treatment or prevention of disease or in the enhancement of desirable physical or mental development and conditions in an animal or human subject. The phrase 'therapeutically- effective amount' means that amount of such an agent that produces a desired local or systemic effect at a reasonable benefit/risk ratio applicable to any treatment. In certain embodiments, a therapeutically effective amount of an agent will depend on its therapeutic index, solubility, and the like. For example, certain therapeutic agents of the present invention may be administered in a sufficient amount to produce a reasonable benefit/risk ratio applicable to such treatment. In the specific context of treatment of cancer, a 'therapeutic effect' can be manifested by various means, including but not limited to, a decrease in solid tumour volume, a decrease in the number of cancer cells, a decrease in the number of metastases observed, an increase in life expectancy, decrease in cancer cell proliferation, decrease in cancer cell survival, a decrease in the expression of tumour cell markers, and/or amelioration of various physiological symptoms associated with the cancerous condition.

In one embodiment, the subject to whom therapy is administered is a mammal (e.g., mouse, rat, primate, non-human mammal, domestic animal or livestock, such as a dog, cat, rabbit, cow, horse, sheep, goat and the like), and is suitably a human. In a further embodiment, the subject is an animal model of cancer. For example, the animal model can be an orthotopic xenograft animal model of a human-derived cancer, suitably liver, lung, pancreas, breast, brain, kidney and/or colon-GI tract cancer.

In a specific embodiment of the methods of the present invention, the subject has not yet undergone a therapeutic treatment, such as therapeutic viral therapy, chemotherapy, radiation therapy, targeted therapy, and/or anti-immune checkpoint therapy. In still another embodiment, the subject has undergone a therapeutic treatment, such as the aforementioned therapies.

In further embodiments, the subject has had surgery to remove cancerous or precancerous tissue. In other embodiments, the cancerous tissue has not been removed, for example, the cancerous tissue may be located in an inoperable region of the body, such as in a tissue or organ that if subjected to surgical intervention may compromise the life of the subject, or in a region where a surgical procedure would cause considerable risk of permanent harm or even lethality.

In some embodiments, the provided mRNA may code for a 'therapeutic enhancement factor'. According to the present invention therapeutic enhancement factors are gene products or polypeptides that may enhance or facilitate the ability of another, co-administered therapeutic agent, to exert a therapeutic effect upon a given cell, suitably the target cell. When introduced into or in the vicinity of the target cell, expression of the therapeutic enhancement factor may cooperate with a co-administered therapeutic agent thereby enabling or enhancing the therapeutic activity of the agent. In some embodiments, the therapeutic enhancement factor may enhance the ability of a co-administered oncolytic virus to lyse cancer cells. In other embodiments of the invention, the therapeutic enhancement factor may effect an alteration of a tumour microenvironment so as to assist or recruit the subject's own immune response. In this latter embodiment, the alteration of the tumour microenvironment may assist coadministration of an oncolytic virus or a CAR-T or other adoptive cell based therapy. In some embodiments, the therapeutic enhancement factor may enable the conversion of a prodrug into an active form.

Multiple therapeutic enhancement factors may be combined in compositions according to specific embodiments of the present invention. In such embodiments, the coding sequences for each therapeutic enhancement factor may be present in separate mRNA molecules. In some embodiments, sequences for more than one therapeutic enhancement factor may be present on the same mRNA molecule. In such cases the polycistronic mRNA molecule further comprises sequences as necessary for the expression of all coded sequences, such as internal ribosome entry sites (IRES).

In embodiments where multiple different mRNA molecules are comprised in one or more delivery system, it is contemplated that each delivery system - e.g. particle, liposome, viral vector system - may comprise one or more than one type of mRNA molecule as the 'payload'; that is, not every delivery payload in a particular embodiment will necessarily comprise all of the mRNA molecules provided in said embodiment. In this way, it is also considered possible to direct different delivery systems and their associated sequences to different target cells, with the targeting agents described herein.

The mRNA constructs of certain embodiments of the invention may be synthesised from a polynucleotide expression construct, which may be for example a DNA plasmid. This expression construct may comprise any promoter sequence necessary for the initiation of transcription and a corresponding termination sequence, such that transcription of the mRNA construct can occur. Such polynucleotide expression constructs are contemplated to comprise embodiments of the invention in their own right.

The gene product encoded by the mRNA is typically a peptide, polypeptide or protein. Where a particular protein consists of more than one subunit, the mRNA may code for one or more than one subunit within one or more ORFs. In alternative embodiments, a first mRNA may code for a first subunit, whilst a second co-administered mRNA may code for a second subunit that, when translated *in situ*, leads to assembly of a multi-subunit protein gene product.

The gene product encoded by the mRNA may be of any type suitable for producing a therapeutic effect. In the context of treating cancer, the gene product encoded by the mRNA may suitably include genes which when expressed by a cancer cell cause or aid in the destruction of the cancer cell.

Tumour suppressor genes such as p53 may be provided by the constructs of the invention. p53 plays a role in cell processes including apoptosis and genomic stability. It is involved in the activation of the DNA repair process in response to genomic damage, and can arrest cell growth and reproduction.

Genes which promote cell death by apoptosis - so-called suicide genes - which when expressed cause the cell to activate the process of apoptosis, may also be provided by the compositions and constructs of the invention. Cancer cells often possess mutated and/or functionless versions of these apoptosis-related genes, and so cannot undergo apoptosis in response to external signals. Suicide gene therapy may also refer to the introduction of genes which allow the conversion of a non-toxic compound or prodrug into a lethal drug (Duarte et al. Cancer Letters, 2012). According to embodiments of the invention, such gene products can be introduced selectively into diseased cells, such as neoplastic cells, marking them for destruction by induced apoptosis or delivery of an otherwise non-toxic compound or prodrug.

In specific embodiments of the invention, the mRNA may encode inhibitors of the programmed cell death pathway, such as inhibitors of PD-1 receptor (CD279) or its ligands PD-L1 (B7-H1; CD274) and PD-L2 (B7-DC; CD273). Hence, the mRNA may encode a protein or polypeptide that binds to or otherwise interferes with the function of the PD-1/PDL-1 or PD-1/PDL-2 axis within diseased or neoplastic cells within a target organ. Suitable proteins or polypeptides may include antibodies, which may be monoclonal or polyclonal, or antigen binding fragments thereof, or other antigen binding microproteins, that bind to PD-1 receptor, PDL-1, PDL-2, or complexes of ligand and receptor. This effect may also be observed by use of protein or polypeptide inhibitors of the cytotoxic T lymphocyte antigen 4 (CTLA4) pathway, another so-called immune checkpoint. Inhibition of either or both pathways is known to result in a change in the immune response within the tumour microenvironment that may positively benefit the health of the patient. In addition, by modulating the immune response in a subject the compositions of the present invention may show particular utility in combinatorial therapies with other anti-cancer therapeutic approaches, such as radiotherapy or chemotherapy. FDA approved anti-PD1 pathway inhibitors include pembrolizumab and nivolumab. Known anti-PDL-1 inhibitors include MPDL-3280A, BMS-936559 and atezolizumab. Anti-CTLA4 therapeutic inhibitors include ipilimumab and tremelimumab. The compositions of the invention may be used to deliver such inhibitors of the programmed cell death pathway- or functional mimetics thereof - selectively to diseased cells within a target organ in a subject by leveraging the differential miRNA environment in those cells

Chimeric antigen receptor T-cells (CAR-T cells) are immune cells, typically T-lymphocytes, which have been modified to express receptors which target cancer cells.

Adoptive immunotherapy, which involves the transfer of autologous antigen-specific T cells generated ex vivo, is a promising strategy to treat viral infections and cancer. The T cells used for adoptive immunotherapy can be generated either by expansion of antigen-specific T cells or redirection of T cells through genetic engineering (see e.g., Park, T. S., S. A. Rosenberg, et al. (2011). "Treating cancer with genetically engineered T cells." Trends Biotechnol 29(11): 550-7).

Novel specificities in T cells, also known as immune effector cells, have been successfully generated through the genetic transfer of transgenic T cell receptors or chimeric antigen receptors (CARs) (see e.g., Jena, B., G. Dotti, et al. (2010). "Redirecting T-cell specificity by introducing a tumor-specific chimeric antigen receptor." Blood 116(7): 1035-44). CARs are synthetic receptors consisting of at least three parts: an extracellular antigen recognition domain (also known as the ectodomain), a transmembrane domain, and an intracellular T-cell activation domain (also known as the endodomain). In some embodiments, the engineered T cells comprise a specific class of T cells, such as, for example, gamma delta T cells, a subtype of T cells that selectively target tumoral cells without affecting healthy ones. CARs have successfully allowed T cells to be redirected against antigens expressed at the surface of tumor cells from various malignancies including lymphomas and solid tumors (Jena, Dotti et al. supra). In some embodiments, the engineered T cells comprise at least a population of autologous T cells in which the CAR-T cells are engineered to eliminate expression of the endogenous αβ T-cell receptor (TCR) to prevent a graft-versus-host response without compromising CAR-dependent effector functions. In some embodiments, the engineered T cells comprise at least a population of allogeneic T cells. In some embodiments, the engineered T cells comprise at least a population of autologous T cells and a population of allogeneic T cells.

Generally, the extracellular antigen recognition domain is a targeting moiety that is associated with one or more signaling domains in a single fusion molecule from an antibody, receptor, or ligand domain that binds a specific target, typically a tumor-associated target. In some embodiments, the extracellular antigen recognition domain is or is derived from a single-chain Fragment variant (scFv) of an antigen-binding domain of a single-chain antibody (scFv), comprising the light and heavy variable fragments of a monoclonal antibody joined by a flexible linker.. In some embodiments, In some embodiments, extracellular antigen recognition domain is linked to the transmembrane domain by a linker, such as, for example, a flexible linker such as the IgG1 hinge linker. In some embodiments, the transmembrane is or is derived from a CD28 transmembrane domain. In some embodiments, the endodomain includes a costimulatory domain designed to enhance the immune response, for example, by enhancing survival and increasing proliferation of CAR modified T cells, and an internal T-cell activation domain designed to activate the T cell when it binds to the desired target. In some embodiments, the costimulatory domain is or is derived from a CD28 costimulatory domain, an OX-40 (CD134) costimulatory domain, an ICOS costimulatory domain, a 4-1BB (CD137) costimulatory domain, or any combination thereof. In some embodiments, the intracellular T-cell activation domain comprises the CD3 zeta (CD3ζ) domain or a biologically active portion thereof. In some embodiments, T cell activation results in immune cell activation in which inflammatory cytokines are released by the T cells to promote an inflammation and/or immune response. In some embodiments, T cell activation results in cytotoxic activity in which cytotoxins are released by the T cells to promote cancer cell apoptosis. In some embodiments, T cell activation results in proliferation in which interleukins are released by the T cells to promote cell development and division. In some embodiments, T cell activation results in a combination of at least two of immune cell activation, cytotoxic activity, and/or proliferation.

In some embodiments, the extracellular antigen recognition domain specifically binds to CD19. CD19 is an attractive target for immunotherapy because the vast majority of B-acute lymphoblastic leukemia (B-ALL) uniformly express CD19, whereas expression is absent on non-hematopoietic cells, as well as myeloid, erythroid, and T cells, and bone marrow stem cells. Clinical trials targeting CD19 on B-cell malignancies are underway with encouraging antitumor responses. Many of the current CAR-T therapies being evaluated in clinical trials use T cells genetically modified to express a chimeric antigen receptor (CAR) with specificity derived from the scFv region of a CD19-specific mouse monoclonal antibody FMC63 (see e.g., Nicholson, Lenton et al. (1997);. "Construction and characterisation of a functional CD19 specific single chain Fv fragment for immunotherapy of B lineage leukaemia and lymphoma." Mol Immunol. 1997 Nov-Dec;34(16-17):1157-65; Cooper, Topp et al. (2003). "T-cell clones can be rendered specific for CD19: toward the selective augmentation of the graft-versus-B-lineage leukemia effect." Blood. 2003 Feb 15;101(4):1637-44; Cooper, Jena et al. (2012) (International application: WO2013/126712).

In some embodiments, extracellular antigen recognition domain specifically binds to CD22. CD22 is a transmembrane phosphoglycoprotein that belongs to the Siglec family of lectins and specifically binds sialic acid with its N-terminus seven extracellular immunoglobulin domains. It mainly acts as an inhibitory receptor for B cell activation and signaling and regulates the interaction of B cells with T cells and antigen presenting cells (APCs). Similar to CD19, CD22 is a B cell lineage-restricted marker, expressed explicitly by B lymphoid cells from the pre-B to mature B cell stage. However, it is lost during differentiation to plasma cells. CD22 is universally expressed in most B cell malignancies, including acute lymphoblastic leukemia (ALL), chronic lymphocytic leukemia (CLL), and various subtypes of non-Hodgkin lymphoma (NHL) such as diffuse large B cell lymphoma. Targeting CD22 as an attractive therapeutic target for B cell malignancies has been confirmed by positive results in clinical trials of anti-CD22 monoclonal antibodies (e.g., epratuzumab) and immunotoxins (e.g., BL22, HA22). CD22 has been shown to be expressed on ALL cells that lost CD19 expression after treatment with anti-CD19 CAR-T cells, making anti-CD22 CAR-T cells suitable for combination and/or follow-on therapy of anti-CD19 CAR-T cells.

However, although numerous clinical studies have demonstrated the potential of adoptive transfer of CAR T cells for cancer therapy, they have also raised the risks associated with the cytokine-release syndrome (CRS) and the "on-target off-tumour" effect.

The mRNA delivery compositions provided in some embodiments herein are useful to improve the safety and efficacy of CAR-T-cells. For example, the mRNA nanoparticle delivery systems of embodiments described herein may be used to recruit specific immune cells or modified subsets of immune cells such as CAR-T cells to the tumour microenvironment. Additionally, the mRNA nanoparticle delivery systems may be used to inhibit expression of endogenous T cell receptors (TCRs) to avoid graft-versus-host disease and/or to selectively delete immune checkpoint genes in these cells to strengthen their anti-cancer activity in the suppressive tumour milieu. (See e.g., Moffett, Coon, et al. (2017) "Hit-and-run programming of therapeutic cytoreagents using mRNA nanocarriers." Nature Communications. 8:389.)

In some embodiments, the coding mRNA is used to attract CAR-T cells to a particular site in a subject. In some embodiments, the coding mRNA is used to overcome insufficient migration of an immune cell to the tumour microenvironment. In response to specific chemokines, different immune cell subsets migrate into the tumour microenvironment and regulate tumour immune responses in a spatiotemporal manner. In some embodiments, the coding mRNA is used to enhance CAR-T cell activation. In addition, chemokines can directly target non-immune cells, including tumour cells and vascular endothelial cells, in the tumour microenvironment, and they have been shown to regulate tumour cell proliferation, cancer stem-like cell properties, cancer invasiveness and metastasis. In some embodiments, the immune cell is a T cell, a natural killer (NK) cell, a B cell, an antigen-presenting cell (APC) such as a macrophage or dendritic cell, or any combination thereof.

In some embodiments, the coding mRNA can be used to overcome insufficient migration of CAR-T cells to the tumour microenvironment, and prevent off-target CAR-T activity. In some embodiments, the mRNA is delivered to the tumour microenvironment, and the coding mRNA encodes a gene product that attracts or otherwise recruits CAR-T cells to the tumour microenvironment. In some embodiments, the coding mRNA expresses a chemokine. By way of non-limiting example, one or more coding mRNAs can encode one or more chemokines that attract T-cells such as CCL2, CCL3, CCL4, CCL5, CCL20, CCL22, CCL28, CXCL8, CXCL9, CXCL10, CXCL11, CXCL12, XCL1, and any combination thereof. In situations where the reverse effect is desired, such as in autoimmune disease, the coding mRNA can express blockers, antagonists and/or inhibitors of the above-mentioned factors.

In some embodiments, the the coding mRNA is transiently expressed in the tumour microenvironment. In some embodiments, the coding mRNA encodes a cytokine or other gene product involved in regulating the survival, proliferation, and/or differentiation of immune cells in the tumour response, such as, for example, activated T cells and NK cells. By way of non-limiting example, the coding mRNA can encode for a cytokine such as IL-1, IL-2, IL-4, IL-6, IL-8, IL-10, IL-12, IL-17, IL-33, IL-35, TGF-beta, and any combination thereof. Again, in situations where the reverse effect is desired, such as in autoimmune disease, the coding mRNA can express blockers, antagonists and/or inhibitors of the above-mentioned factors.

In some embodiments, of the invention, the coding mRNA is delivered in conjunction with CAR-T or other adoptive cell therapy to provide transient expression of the coding mRNA.

In some embodiments, a mRNA delivery system as described herein delivers an mRNA that codes for a gene-editing agent to a target cell population. In some embodiments, the mRNA codes for a sequence-specific nuclease that targets a gene locus and disrupts expression of one or more endogenous gene produces in the target cell population. In some embodiments, the mRNA codes for a sequence-specific nuclease that targets a T cell receptor (TCR)-related gene locus, thereby disrupting expression of one or more domains in the TCR.

In some embodiments, the described mRNA delivery systems may be used to deliver an mRNA that codes for one or more agents that program engineered T cells toward a desired phenotype. In some embodiments, the mRNA may be used to induce markers and transcriptional patterns that are characteristic of a desired T cell phenotype. In some embodiments, the mRNA may be used to promote development of CD26L+ central memory T cells (Tcm), which have been shown to improve CAR-T treatment. (See e.g., Moffett, Coon supra).

MicroRNAs (miRNAs) are a class of noncoding RNAs each containing around 20 to 25 nucleotides some of which are believed to be involved in post-transcriptional regulation of gene expression by binding to complementary sequences in the 3' untranslated regions (3' UTR) of target mRNAs, leading to their silencing. These sequences are also referred to herein as miRNA binding sites, or miRNA binding site sequences. Certain miRNAs are highly tissuespecific in their expression; for example, miR-122 and its variants are abundant in the liver and infrequently expressed in other tissues (Lagos-Quintana (2002), Current Biology, Vol.12, Apr).

The miRNA system therefore provides a robust platform by which nucleic acids introduced into cells can be silenced in selected cell types in a target tissue, and expressed in others. By including a binding site for a particular given miRNA sequence into an mRNA construct to be introduced into target cells, particularly in or immediately 5' or 3' to a UTR, expression of certain introduced genes can be reduced or substantially eliminated in some cell types, while remaining in others (Brown and Naldini, Nature Reviews Genetics volume 10, pages 578-585 (2009)). The use of the term 'immediately' is understood to be synonymous with terms such as 'highly proximate to' or 'very close to'. When referring to 5' or 3' positioning relative to a UTR sequence it encompasses variants in which typically up to around twenty, suitably not more than fifty, intervening nucleotide bases may be placed between the miRNA binding sequence and the adjacent UTR. It is contemplated that one, or a plurality, of such miRNA binding site sequences can be included in the mRNA construct. Where a plurality of miRNA binding site sequences are present, this plurality may include for example greater than two, greater than three, typically greater than four miRNA binding site sequences. These miRNA binding site sequences may be arranged sequentially, in tandem or at predetermined locations within, 3' to, or 5' to a specified UTR within the mRNA constructs. Multiple binding site sequences may be separated with a linker sequence, which may vary, or may comprise a particular sequence, for example, "*uuuaaa*"*.* In some embodiments, no linker sequence may be present between binding site sequences. Other parts of the mRNA sequence can incorporate linker sequences, such as between the stop codon of the ORF and the UTR or binding sites.

miRNA-122, despite its abundance in healthy non-diseased liver tissue, is reduced in the majority of liver cancers as well as in diseased cells (Braconi et al. 2011, Semin Oncol; 38(6): 752-763, Brown and Naldini Nature 2009;10 578). By the above-mentioned method, it has been found that when the target tissue is the liver, translation of the introduced mRNA sequences can be facilitated in cancerous liver cells and reduced or substantially eliminated in transfected healthy cells, by including miRNA-122 binding sites (for example, SEQ ID NO: 1) in or adjacent to their 3' UTRs.

In a similar way, differential translation of such mRNA is also possible between cancer cells and healthy cells in other organs, by using other miRNA binding site sequences. Suitable candidates include (but are not limited to) binding sites for miRNA-125, miRNA-124a, miRNA-Let7, miRNA-375, miRNA-143, miRNA-145, miRNA-192, miRNA-194, miRNA-204, miRNA-215 and miRNA-30b,c. Table 2 demonstrates further (non-limiting) examples of miRNA sequences where differential expression has been demonstrated.

miRNA-125 is downregulated in several solid tumors, such as hepatocellular carcinoma (Coppola et al. Oncotarget 2017;8); breast (Mattie et al. Mol Cancer 2006;5), lung (Wang ewt al. FEBS J 2009), ovarian (Lee et al. Oncotarget 2016;7), gastric (Xu et al. Mol Med Rep 2014;10), colon (Tong et al. Biomed Pharmacother 2015;75), and cervical cancers (Fan et al Oncotarget 2015;6); neuroblastoma, medulloblastoma (Ferretti et al. Int J Cancer 2009;124), glioblastoma (Cortez et al. Genes Chromosomes Cancer 2010;49), and retinoblastoma (Zhang et al; Cell signal 2016;28).

Several miRNA species are also differentially expressed in glioblastoma multiforme cells (Zhangh et al. J Miol Med 2009;87 / Shi et al. Brain Res 2008;1236) compared to non diseased brain cells (e.g. neurons), with miRNA-124a one of the most dysregulated (Karsy et al. Gene Cancer 2012;3; Riddick et al. Nat Rev Neurol 2011;7; Gaur et al. Cancer Res 2007;67 / Silber et al. BMC Med 2008;6).

In lung cancer, a recent meta-analysis confirmed the downregulation of Let-7 (as well as miRNA-148a and miRNA-148b) in non-small-cell lung cancer (Lamichhane et al. Disease Markers 2018).

Similarly, miRNA-375 expression has be found to be downregulated in pancreatic cancer cells, compared to healthy pancreatic cells (Shiduo et al. Biomedical Reports 2013;1).

In another embodiment, using IL-12 secreting cells can enhance the activity of CAR-T cells. Without wishing to be bound by theory, it is believed that IL-12 induced IFNy accumulation in tumors promotes the penetration of CAR-T or other host immune cells (e.g. NK cells) into the tumours, thereby enhancing the therapeutic effects (Chinnasamy D. et al. Clin Cancer Res 2012:18 / Chmielewski M. et al. Cancer Res 2011;71 / Kerkar SP. Et al. J Clin Invest 2011;121 / Jackson HJ. Et al. Nat Rev Clin Oncol 2016;13). Hence, according to an embodiment of the present invention the mRNA may be used to promote exogenous expression of IL-12 in the tumour microenvironment, by coding for IL-12 or a functional equivalent or derivative thereof.

In the context of disease-specific expression of introduced polynucleotides, binding sequences for any miRNA sequence which is disrupted in a particular disease - that is, upregulated or downregulated in diseased cells (such as tumour cells) in comparison to non-diseased cells - is considered suitable for use in the invention. Table 2 discusses further, non-limiting examples of tumour-associated miRNA binding sequences of this kind which may be used in embodiments of the present invention. It will be appreciated, however, that the present invention is not limited only to instances where a given miRNA or class of miRNAs is downregulated in a first cell type versus a second cell type within a given organ or organ system. On the contrary, it is merely required that there exists a differential expression pattern of a regulatory miRNA between first and second cell types comprised within the organ or organ system. The differential expression of the miRNA can be exploited using the compositions and methods described herein to enable corresponding differential translation of protein products in those cells.

Examples of cancers where evidence has been found for similar differential miRNA expression between healthy and cancer cells include breast (Nygaard et al, BMC Med Genomics, 2009 Jun 9;2:35), ovarian (Wyman et al, PloS One, 2009 ;4(4):e5311), prostate (Watahiki et al, PloS One, 2011; 6(9):e24950), and cervical cancers (Lui et al. Cancer Research, 2007 Jul 1;67(13):6031-43). WO 2017/132552 A1 describes a wide range of miRNAs with differing expression levels in various cancer cells.

**Table 2**

| Tissue/cancer type | Implicated miRNA | Expression profile | Reference |
|---|---|---|---|
| Liver | miRNA-122 | Reduced in cancer cells | Braconi, 2011, Brown, 2009 |
| Liver | miRNA-125, miRNA-199 | Reduced in hepatocarcinoma | Coppola N. Oncotarget, 2017. Vol 8 Murakami Y. Oncogene 2006;25 |
| Brain | miRNA-124a | Reduced in glioblastoma multiforme | Mazzacurati L. Molecular therapy 23, 2015 |
| Lung, breast | Let-7 miRNA-148a/b | Reduced in cancer cells | Edge RE et al. Mol Ther 2008;16:1437 Yu F. Cell 2007; 131 (6):11 09-23 Takamizawa J. Cancer Res 2004;64 |
| Pancreas | miRNA-375 | Reduced in cancer cells | Song S, Zhou J et al. Biomed Reports: 393-398, 2013 |
| Colon | miRNA-143, miRNA-145 | Reduced in cancer cells | Michael MZ. Mol Cancer Res 2003;334 |
| Kidney | miRNA-192, -194, -204, -215, -30b,c | Kidney-specific expression | Sempere LF. Genome Biol 2004;5 Sun Y. Nucleic acids res 2004;32 |

In the pancreas, miRNA-375 expression has been indicated to be high in normal pancreas cells but significantly lower in diseased and/or cancerous tissues (Song, Zhou et al. 2013). This expression has been shown to relate to the stage of cancer, with expression further reduced with more advanced cancer. It is thought that miRNA-375 is involved with the regulation of glucose-induced biological responses in pancreatic β-cells, by targeting 3-phosphoinositide- dependent protein kinase-1 (PDK1) mRNA and so affecting the PI 3-kinase/PKB cascade (EI Ouaamari et al. Diabetes 57:2708-2717, 2008). An anti-proliferative effect of miRNA-375 is implicated by this putative mode of action, which may explain its downregulation in cancer cells.

It is known that variants and polymorphisms of miRNA sequences can be found, and that miRNA families exist with similar properties. In the present invention, it is envisioned that all suitable variants and family members of particular miRNA sequences and associated binding sites can be used where appropriate. On the other hand, apparently closely related miRNA sequences can have different expression profiles (Sun et al, World J Gastroenterol. 2017 Nov 28), so in some situations it will be necessary to determine whether a specific substitution is appropriate, by reference to the literature. For example, Let-7 is part of a wider family with a number of related variants, which can be denoted as Let-7a to Let-7k, and so on.

Treating patients with immunotherapies like oncolytic viruses or CAR-T therapies may have safety issues due to the possibility of off-target effects. Even the expression of certain polypeptides by the provision of coding mRNA sequences can have negative effects on certain organs. Protecting healthy tissues, for example liver, brain, breast, lung, pancreas, colon/GI-tract and kidneys is thus paramount for successful clinical applications. Hence, the present compositions may represent an enabling technology platform for enhancing and facilitating the successful adoption of hitherto 'experimental' cellular or viral therapies.

The presence of a plurality of miRNA binding site sequences in the mRNA construct enables improved efficacy of the differential expression of the supplied polypeptide or polypeptides. Without being bound by theory, it is thought that with an increased number of sites for binding, the likelihood of miRNA binding and consequent degradation is increased. Multiple miRNA binding sites can comprise multiple copies of substantially the same binding site sequence, thereby introducing redundancy. Alternatively or additionally, the multiple binding site sequences can comprise substantially different sequences, thereby allowing the mRNA construct to be targeted by more than one species of miRNA. In this way, differential expression of a supplied mRNA construct can be achieved for more than one cell type, and/or in more than one organ, as is evident from the discussion of organs and their associated miRNA sequences above. Both approaches are considered to be possible within the same sequence or multiple sequences. An intermediate approach is also envisioned, wherein multiple binding sequences are included which are intended to be targets for the same miRNA sequence, but have differences in order to bind different variants of the same miRNA sequence.

Some advantages associated with the use of multiple binding sites include an increase in the efficiency of differential expression of polypeptides supplied by the mRNA sequences of the present invention, within a single organ. Use of different binding site sequences, or sequences which are applicable to more than one tissue or organ type can enable differential expression to be achieved in different cell types in more than one organ or tissue. This may be desirable when systemic administration of compositions according to the invention is used, and it is necessary to avoid off-target effects in more than one organ.

Even with localised or targeted administration, it is possible that supplied mRNA constructs may encounter or accumulate in organs, tissues, and/or cells for which they were not intended. In particular, liver and kidney tissue may accumulate administered compositions, due to the physiological function of these organs. In these cases, to avoid off-target effects, it may be advantageous for the supplied constructs to comprise miRNA binding site sequences which would enable reduced expression in these tissues. Conversely, it may be desirable for expression to be encouraged in some organs, tissues and/or cell types but not others, which can be achieved by the selection of miRNA binding site sequences accordingly.

In some embodiments, more than one different mRNA sequence may be provided in a single composition. These different sequences can encode different polypeptides, and/or different miRNA binding sequences. In this way, a single composition can allow for multiple different polypeptides to be expressed. By using different combinations of miRNA binding sequences in the separate mRNA sequences, different cell types or target organs can express, or be protected from the expression of certain polypeptides, according to the desired objective. For instance, if healthy cells in liver and brain must be protected from the expression of a polypeptide 'A', but it is desired to express a polypeptide 'B' in healthy brain, but not liver, a first mRNA sequence could comprise the sequence of 'A', with binding sites for miRNA-122, miRNA-125a and miRNA-124a, while a second mRNA sequence could comprise the sequence of 'B', with binding sites for miRNA-122 and miRNA-125a.

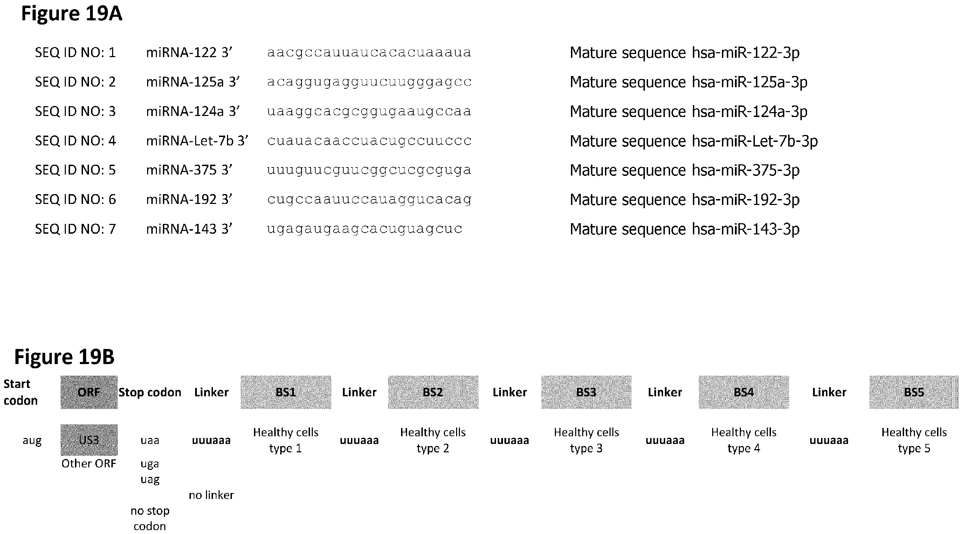

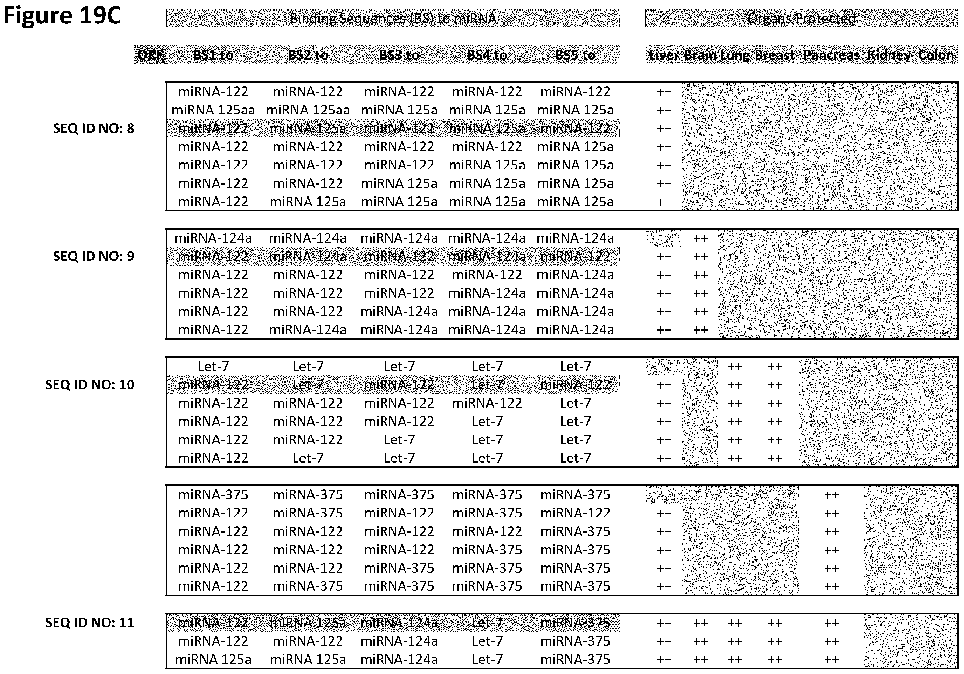

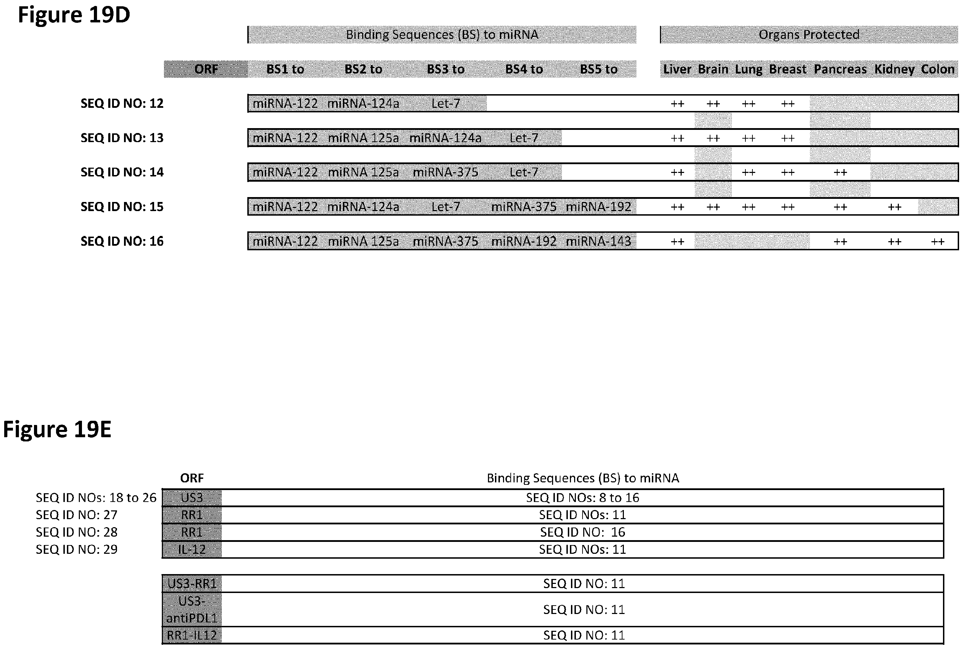

It can be appreciated that the person of skill in the art will be able to devise combinations of binding sites, polypeptide sequences and multiple mRNA sequences in order to achieve any combination of expression in a given set of organ and cell types. Some examples are shown in Figures 19A, 19B, 19C, 19D and 19E and in SEQ ID NOs: 1 to 29. Figure 19A shows the sequences of possible miRNA binding sites for miRNA-122, miRNA-125a, miRNA-124a, Let-7, miRNA-375, miRNA-192, and miRNA-143 (relating to SEQ ID NOs: 1 to 7, respectively). The relevant organs and tissue types relating to these sequences are discussed above and in Table 2. Figure 19B shows schematic views of mRNA constructs according to some embodiments of the invention. An ORF is preceded by a start codon and terminated with a stop codon, and a subsequent series of up to five binding site sequences follow. As shown in this figure, the binding site sequences may be separated by linkers, or no linker. The ORF can code for example for US3, or may be any ORF. Variability in the stop codon is envisioned in any embodiment, and there may in all embodiments be no stop codon between the ORF and the binding site sequences.

Particular combinations of miRNA binding site sequences are shown in Figures 19C and 19D, together with the organs which have improved protection as a result of these selections. These can be seen to be exemplified by SEQ ID NOs: 8 to 16. It will be appreciated that protection is relative, rather than absolute, and that levels of microRNA expression will vary within an organ between different tissues, and between organs. Variations will also exist within a population, based upon ethnicity, age, sex, health status, and pathology, as well as genetic variation and polymorphisms between individuals.

In one embodiment, the mRNA sequence can comprise SEQ ID NO: 11. This sequence comprises binding sequences for the micro RNA sequences miRNA-122, miRNA-125, miRNA-124a, Let-7 and miRNA-375, These sequences provide protection against unwanted expression of the provided polypeptide in healthy tissue of organs such as (but not restricted to) liver, brain, lungs, pancreas, and breast. Similarly, a sequence comprising binding sequences for the miRNA sequences miRNA-122, miRNA-124a, Let-7, miRNA-375, miRNA-192 will provide improved protection to liver, brain, lungs, pancreas, breast and kidneys. For the treatment of gastro-intestinal tumours such as colo-rectal cancer, protecting healthy tissues in liver, lung, breast, pancreas, kidney and colon will be important for medical use.

Figure 19E indicates particular combinations of polypeptide coding sequences and binding sites. Namely, the US3 coding sequence is indicated in combination with any of the binding site sequence combinations shown in Figures 19B and 19C (for example, with SEQ ID NOs: 8 to 16), as exemplified by SEQ ID NOs: 18 to 26, and the RR1 coding sequence is indicated in combination with the binding site sequences of, for example, SEQ ID NOs: 11 and 16, as exemplified by SEQ ID NOs: 27 and 28. The IL-12 coding sequence is indicated in combination with the binding site sequences encompassed in, for example, SEQ ID NO: 11, as exemplified by SEQ ID NO: 29. An ORF comprising coding sequences for both US3 and RR1, both US3 and anti-PDL1, both US3 and anti-PDL1, and both RR1 and IL12 are indicated in combination with the binding site sequences of, for example, SEQ ID NO: 11. However, all combinations of coding sequences and multiple binding site sequences are considered herein. In particular, the coding sequences for US3, RR1, anti-PDL1 and IL12 can be combined with any binding site sequences.

In some embodiments, it may be desirable for the binding site sequences to have mismatches with the miRNA sequences which target them. For example, mismatches can occur in relation to up to 5%, up to 10%, up to 20%, or up to 30% of the bases of the binding site sequences, compared to the whole target miRNA sequences. Without wishing to be bound by theory, it is thought that excessive uptake of miRNA sequences within cells may in some cell types lead to dysregulation of the endogenous miRNA system. Such dysregulation of miRNA profiles is associated with different liver diseases (Szabo G et al. Gastroenterol Hepatol 2013;10 / Schueller F. et al. Int J Mol Sci 2018;19). Reduced complementarity between the provided binding site sequences and miRNA sequences can alleviate this potential side effect.

The UTR of the mRNA sequences supplied by the present invention can be selected to have similarity, for example greater than 90% similarity, to part or all of a UTR sequence expressed in one of the cell types within the target organ. Particular cell types can have genes which are up- or down-regulated in expression, and the UTR sequence can mediate this regulation, for instance through encouraging the stability or degradation of the relevant mRNA sequences.

As an example, UTRs associated with genes which are known to be upregulated in cancer cells may have one or more features, such as miRNA binding site sequences, which encourage their stability and translation in these cancer cells. By incorporating similar sequences into supplied mRNA sequences, stability and translation can be improved in cancerous cells but not non-cancerous or healthy cells.

It is also considered that the cancer to be treated by the invention may be a secondary cancer in the target tissue, that is, a metastasis from a cancer elsewhere than the target tissue. For example, a liver metastasis might originate from a cancer of the oesophageal, stomach, colon, rectum, breast, kidney, skin, pancreas or lung, and may be adenocarcinoma or another type of cancer. In these cases alternative miRNA sequences may need to be selected in order to provide differential expression in healthy, non-cancerous and/or cancerous cells. Indeed, there may be an increased choice of candidate miRNA sequences in such cases, due to the different tissue origin of metastasised cells.

In certain situations, it is possible that more than one candidate for an miRNA sequence which exhibits differential expression in different cell types in a target tissue may exist. In such cases, it may be advantageous that a plurality of miRNA binding site sequences are included in the mRNA construct, and that these sequences may be substantially different sequences. However, it is also envisaged that each of the plurality of miRNA binding site sequences may be substantially the same sequence.

### COMBINATION THERAPIES

### Oncolytic Viruses

As mentioned above, oncolytic viral therapy is the process of using viruses to infect and kill cancer cells, sometimes by direct viral lysis, but also including indirect killing by the stimulation of host anti-tumoural responses. While oncolytic viruses are frequently characterised by having increased activity in cancer cells compared with healthy cells, off-target effects caused by damage to healthy cells have been documented (Russell et al. Nature Biotechnology, 2012).

In order to increase safety and decrease off-target effects, oncolytic viruses may be modified or selected to reduce their virulence, for example by the deletion of virulence factors or genes involved in functions such as intracellular immune system suppression and evasion, viral genome replication, and the takeover of host cell processes. The historical production of safe forms of live viruses for use in vaccination is another source of attenuated viruses. In other cases, particular mutations or even additional genes have been seen to enhance oncolytic activity in particular oncolytic viruses. Non-exhaustive examples of the virulence genes commonly added, mutated or deleted in oncolytic viruses may be found in Table 3.

**Table 3**

| **Oncolytic virus** | **Mutation** | **Reference** |
|---|---|---|
| Vesicular Stomatitis Virus, marabavirus | G protein (Q242R mutation) M protein (L123W mutation) | Brun et al 2010, Mol Ther.; 18(8): 1440-1449. |
| Vaccinia virus | Ribonucleotide reductase (RR1, RR2) inactivation Thymidine kinase (TK) inactivation | Buller et al. Nature (London) 1985;317. Puhlamm M et al. Cancer Gene Ther 2000;7 |
| | A56R inactivation | Slabaugh MB et al. J Virol 1984;52 |
| Measles virus | NIS gene - Human thyroidal iodide symporter | Aref et al 2016, Viruses, 8, 294 |
| Newcastle disease virus | Fusion protein (F) cleavage site | Vigil et al 2007 Cancer Res; 67: (17). |
| Parvovirus | NS protein NS1 | Marchini et al 2015 Virology Journal 12:6 |
| Herpes Simplex Virus Type 1 (HSV-1) | Viral ribonucleotide reductase (ICP6) inactivation; serine/threonine-protein kinase (US3) inactivation; ICP34.5 and ICP47 inactivation (Neurovirulence and immune system evasion); UL43 inactivation (Cell fusion) inactivation; UL49.5 inactivation (T-cell evasion) inactivation; UL55 and UL56 | Liu et al (2003) Gene Therapy volume 10, 292-303; Goldsmith et al 1998 J Exp Med. 187(3): 341-348; |
| Adenovirus | E1B-55, E3, E1a promoter, E3 gp19kD, E1A 924bp), E1A, deletion in E3 and E4, E3 quaitotal deletion, chimeric ad3/Ad11p E2B region, E3-6.7K + gp19K E1A | Baker et al 2018, Cancers, 10, 201 |

The attenuation or modification of oncolytic viruses in this way can play a role in the selectivity of oncolytic viruses to cancer cells: since the process of carcinogenesis often involves the inactivation of genes that play protective roles against both cancer (such as by regulating cell division or apoptosis), and viral infection, oncolytic viruses which are attenuated as described can retain their virulence in cancer cells, due to the absence of the usual antiviral genes in these cells. Therefore in healthy cells the attenuated virus cannot defend against the normal antiviral responses, and is eliminated, whereas in cancer cells this response is absent, and the virus can lyse the cells. However, this approach is rarely completely effective, as firstly partial inactivation of antiviral responses in cancer cells is more common than a complete lack of antiviral activity (Haralambieva et al, Mol. Ther., 2007), meaning that virulence can still be reduced in these cells, and secondly infection of healthy cells can still occur.

Similarly, as viruses typically utilise the cellular machinery of the host cell in order to replicate their genomes, but this machinery is typically downregulated in healthy, quiescent, non-replicating cells which are not replicating their own genomes, many viruses possess genes which reactivate or compensate for the host machinery. For example, ribonucleotide reductase enzymes are necessary for the production of deoxyribonucleotides from ribonucleotides; these enzymes are typically downregulated in quiescent host cells, and several viruses possess genes for their own enzyme of this type, in order to have a source of deoxyribonucleotides. Since replicating cancer cells may have these enzymes reactivated, an attenuated oncolytic virus with its own ribonucleotide reductase enzyme gene deleted can still replicate in cancer cells. However, for reasons similar to the above, this approach may not be completely effective, either in protecting healthy cells from infection, or in restoring virulence in cancer cells. For example, not all cells in a tumour are replicating at any given time, and as such sufficient deoxyribonucleotides may not be available for viral replication in the majority of cancer cells.

Following the above, when a composition or method according to the present invention is used in conjunction with oncolytic viral therapy, the therapeutic enhancement factor provided by the constructs of the invention may be a factor which increases the efficacy of the oncolytic virus in cancer cells, for example enhancing replication of the virus, or the ability of the virus to lyse the cells in which it resides. In particular, where the oncolytic virus has been modified to attenuate its function, for example by the deletion of one or more genes for virulence factors, the therapeutic factor may replace the deleted gene with mRNA for a gene product which is a copy of the viral gene product, or a gene product with substantial homology to the deleted gene, or which otherwise compensates for the deletion of the gene. In such embodiments, by the differential expression in healthy and cancerous cells which is made possible by the invention, the replacement gene product can be expressed only in cancer cells, enhancing viral activity and lysis in these cells, rather than in healthy cells, where expression of the provided mRNA is inhibited by the presence of the miRNA binding sites.

By similar means, mRNA coding for factors which increase the resistance of cells to oncolytic viruses can be expressed preferentially in healthy cells, again promoting viral activity in cancerous cells compared to healthy cells.

A benefit of this approach is that, unlike previous therapies using oncolytic viruses, it does not rely on which cellular antiviral genes and processes may be inactivated due to carcinogenesis, nor on cell replication processes which may be activated in some cancer cells but not others. As a result, a greater scope of which virulence genes can be deleted from oncolytic viruses is allowed. Thus, oncolytic viruses can be modified to completely lack replicative ability in healthy cells, and, in cancer cells where the function of the deleted virulence genes are replaced by means of the invention, the virus can be restored to full potency. As a result, side effects can be reduced, and efficacy increased. Similarly, since the differential expression of the provided mRNA relies on miRNA expression differences between cancer and healthy cells, virulence can be restored in all transfected cancer cells, and not only those that, for example, are undergoing replication at time of administration.

In a particular embodiment, the oncolytic virus is HSV-1, part of the herpesvirus family. Attenuated versions of HSV may be engineered or selected to be deficient in ICP6, which encodes a viral ribonucleotide reductase (Aghi et al, Oncogene. 2008) and/or in US3, which encodes a serine/threonine-protein kinase, and plays several roles in the virus' lifecycle, including blocking host cell apoptosis (Kasuya et al, Cancer Gene Therapy, 2007).

In another embodiment, the oncolytic virus is part of the poxvirus family, in particular, it may be Vaccinia virus. Attenuated versions of Vaccinia may be engineered or selected to be deficient in one or more subunit of ribonucleotide reductase (RR1 and RR2) and/or in thymidine kinase (TK) (Buller et al. Nature (London) 1985;317. Puhlamm M et al. Cancer Gene Ther 2000;7 Slabaugh MB et al. J Virol 1984;52).

Examples of sequences comprising specific coding sequences for particular polypeptides associated with oncolytic viruses, as well as a plurality of miRNA binding site sequences, are shown for example in SEQ ID NOs: 18 to 28, as well as in Figures 19B to 19D.

### Cytokines

It is contemplated that the compositions and methods as described herein may act to induce an immune response against disease. In particular, immune responses may be induced against cancer cells. The process of carcinogenesis frequently involves ways in which the cancer cells attempt to evade the immune system, involving changes to the antigens produced and displayed by these cells,

In some embodiments, the mRNA provided by the invention comprises at least one polynucleotide encoding a protein that is a bispecific T-cell engager (BiTE), an anti-immunosuppressive protein, or an immunogenic antigen. The term "anti-immunosuppressive protein" as used herein is a protein that inhibits an immunosuppressive pathway.

The invention encompasses compositions supplying mRNA coding for an anti-immunosuppressive protein that is an anti-regulatory T-cell (Treg) protein or an anti-myeloid-derived suppressor cell (MDSC) protein. In some embodiments, the anti-immunosuppressive protein is a VHH-derived blocker or a VHH-derived BiTE.

The term "immunogenic antigen" as used herein refers to a protein that increases an inflammatory or immunogenic immune response. In particular embodiments, the anti-immunosuppressive and immunogenic antigens induce an anti-tumour immune response. Examples of such proteins include antibody or antigen binding fragments thereof that bind to and inhibit immune checkpoint receptors (e.g. CTLA4, LAG3, PD1, PDL1, and others), proinflammatory cytokines (e.g., IFNy, IFNα, IPNβ , TNFα, IL-12, IL-2, IL-6, IL-8, GM-CSF, and others), or proteins that binding to and activate an activating receptor (e.g., FcyRI, Fcylla, Fcyllla, costimulatory receptors, and others). In particular embodiments, the protein is selected from EpCAM, IFNβ, anti-CTLA-4, anti-PD1, anti-PDL1, A2A, anti-FGF2, anti-FGFR/FGFR2b, anti-SEMA4D, CCL5, CD137, CD200, CD38, CD44, CSF-1R, CXCL10, CXCL13, endothelin B Receptor, IL-12, IL-15, IL-2, IL-21, IL-35, ISRE7, LFA-1, NG2 (also known as SPEG4), SMADs, STING, TGFβ, and VCAM1.

The invention encompasses compositions supplying mRNA coding for functional macromolecules to targeted cell populations used in cell-based therapies. In some embodiments, the targeted cell population is a genetically engineered T cell population. In some embodiments, the targeted cell population is a population of chimeric antigen receptor T cells (CAR-T cells).

The coding mRNA may be used to attract a population of immune cells or a combination of immune cell populations to a particular site in a subject. In some embodiments, the coding mRNA and the delivery particles are used to attract immune cells to the tumour microenvironment. In some embodiments, the coding mRNA and the delivery particles are used to overcome insufficient migration of an immune cell to the tumour microenvironment. In some embodiments, the immune cell is a T cell, a natural killer (NK) cell, a B cell, an antigen-presenting cell (APC) such as a macrophage or dendritic cell, or any combination thereof. In some embodiments, the coding mRNA and the delivery particles are used to attract CAR-T cells to the tumour microenvironment.

The coding mRNA may be used to overcome insufficient migration of CAR T cells to the tumour microenvironment. In some embodiments, the delivery particles specifically target the tumour microenvironment, and the coding mRNA encodes a gene product that attracts or otherwise recruits CAR-T cells to the tumour microenvironment. In some embodiments, the coding mRNA expresses a chemokine. By way of non-limiting example, the coding mRNA can encode a chemokine that attracts T-cells such as CCL2, CCL3, CCL4, CCL5, CCL20, CCL22, CCL28, CXCL8, CXCL9, CXCL10, CXCL11, CXCL12, XCL1, and any combination thereof. In situations where the reverse effect is desired, such as in autoimmune disease, the coding mRNA can express blockers, antagonists and/or inhibitors of the above-mentioned factors.

The coding mRNA may be delivered to and transiently expressed within the tumour microenvironment. In some embodiments, the coding mRNA encodes a cytokine or other gene product involved in regulating the survival, proliferation, and/or differentiation of immune cells in the tumour response, such as, for example, activated T cells and NK cells. By way of non-limiting example, the coding mRNA can encode for a cytokine such as IL-1, IL-2, IL-4, IL-6, IL-8, IL-10, IL-12, IL-17, IL-33, IL-35, TGF-beta, and any combination thereof. Again, in situations where the reverse effect is desired, such as in autoimmune disease, the coding mRNA can express blockers, antagonists and/or inhibitors of the above-mentioned factors.

The compositions supplying mRNA may be designed to target particular cell subtypes and, upon binding to them, stimulate receptor-mediated endocytosis, thereby introducing the synthetic mRNA they carry to the cells, which can now express the synthetic mRNA. Because nuclear transport and transcription of the transgene are not required, this process is fast and efficient.

In some embodiments, the mRNA delivery system delivers an mRNA that codes for a gene-editing agent to a target cell population. In some embodiments, the mRNA codes for a sequence-specific nuclease that targets a gene locus and disrupts expression of one or more endogenous gene produces in the target cell population. In some embodiments, the mRNA codes for a sequence-specific nuclease that targets a T cell receptor (TCR)-related gene locus, thereby disrupting expression of one or more domains in the TCR.

In some embodiments, the mRNA delivery systems may be used to deliver an mRNA that codes for one or more agents that program engineered T cells toward a desired phenotype. In some embodiments, the mRNA nanoparticle delivery compositions may be used to induce markers and transcriptional patterns that are characteristic of a desired T cell phenotype. In some embodiments, the mRNA nanoparticle delivery compositions may be used to promote development of CD26L+ central memory T cells (Tcm), which have been shown to improve CAR-T treatment. (See e.g., Moffett, Coon supra). In some embodiments, compositions supply mRNA encoding one or more transcription factors to control cell differentiation in a target cell population. In some embodiments, the transcription factor is Foxo1, which controls development effector-to-memory transition in CD8 T-cells.

In some embodiments, the mRNA delivery compositions include a surface-anchored targeting domain that is specific for a T cell marker, such as, for example, a surface antigen found on T cells. In some embodiments, the surface-anchored targeting domain is specific for an antigen that selectively binds the nanoparticle to T-cells and initiates receptor-induced endocytosis to internalize the mRNA nanoparticle delivery compositions. In some embodiments, the surface-anchored targeting domain selectively binds CD3, CD8, or a combination thereof. In some embodiments, surface-anchored targeting domain is or is derived from an antibody that selectively binds CD3, CD8, or a combination thereof.

By means of the invention, differential expression of the above-mentioned gene products can be achieved in different cell types, for example, in healthy cells, non-diseased, diseased and cancer cells. By this method, an immune response can be triggered targeted towards diseased cells while sparing the non-diseased or healthy cells.

The introduction of coding nucleotide sequences into a target cell often requires the use of a delivery agent to transfer the desired substance from the extracellular space to the intracellular environment. Frequently, such delivery agents are in the form of delivery particles, which may undergo phagocytosis and/or fuse with a target cell. Delivery particles may contain the desired substance by encapsulation or by comprising the substance within a matrix or structure.

The delivery particles may be targeted to the cells of the target tissue. This targeting may be mediated by a targeting agent on the surface of the delivery particles, which may be a protein, peptide, carbohydrate, glycoprotein, lipid, small molecule, nucleic acid, etc. The targeting agent may be used to target specific cells or tissues or may be used to promote endocytosis or phagocytosis of the particle. Examples of targeting agents include, but are not limited to, antibodies, fragments of antibodies, low-density lipoproteins (LDLs), transferrin, asialycoproteins, gp120 envelope protein of the human immunodeficiency vims (HIV), carbohydrates, receptor ligands, sialic acid, aptamers etc.

The delivery particles may comprise aminoalcohol lipidoids. These compounds may be used in the formation of particles including nanoparticles, liposomes and micelles, which are particularly suitable for the delivery of nucleic acids. An illustrative example for the production of nanoformulations comprising particles according to some embodiments of the invention may be found in the Examples.

When administered to a subject, a therapeutic component is suitably administered as part of a composition that comprises a pharmaceutically acceptable vehicle. Acceptable pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilising, thickening, lubricating and colouring agents may be used. When administered to a subject, the pharmaceutically acceptable vehicles are preferably sterile. Water is a suitable vehicle when the compound of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skimmed milk, glycerol, propylene, glycol, water, ethanol and the like. Pharmaceutical compositions, if desired, can also contain minor amounts of wetting or emulsifying agents, or buffering agents.

The medicaments and pharmaceutical compositions of the invention can take the form of liquids, solutions, suspensions, gels, modified-release formulations (such as slow or sustained-release), emulsions, capsules (for example, capsules containing liquids or gels), liposomes, microparticles, nanoparticles or any other suitable formulations known in the art. Other examples of suitable pharmaceutical vehicles are described in Remington's Pharmaceutical Sciences, Alfonso R. Gennaro ed., Mack Publishing Co. Easton, Pa., 19th ed., 1995, see for example pages 1447-1676.

For any compound or composition described herein, the therapeutically effective amount can be initially determined from *in vitro* cell culture assays. Target concentrations will be those concentrations of active component(s) that are capable of achieving the methods described herein, as measured using the methods described herein or known in the art.

As is well known in the art, therapeutically effective amounts for use in human subjects can also be determined from animal models. For example, a dose for humans can be formulated to achieve a concentration that has been found to be effective in animals. The dosage in humans can be adjusted by monitoring compounds effectiveness and adjusting the dosage upwards or downwards, as described above. Adjusting the dose to achieve maximal efficacy in humans based on the methods described above and other methods is well within the capabilities of the ordinarily skilled artisan.

It is contemplated that embodiments of the invention may include compositions formulated for use in medicine. As such, the composition of the invention may be suspended in a biocompatible solution to form a composition that can be targeted to a location on a cell, within a tissue or within the body of a patient or animal (i.e. the composition can be used in vitro, ex vivo or in vivo). Suitably, the biocompatible solution may be phosphate buffered saline or any other pharmaceutically acceptable carrier solution. One or more additional pharmaceutically acceptable carriers (such as diluents, adjuvants, excipients or vehicles) may be combined with the composition of the invention in a pharmaceutical composition. Suitable pharmaceutical carriers are described in 'Remington's Pharmaceutical Sciences' by E. W. Martin. Pharmaceutical formulations and compositions of the invention are formulated to conform to regulatory standards and can be administered orally, intravenously, topically, intratumorally, or subcutaneously, or via other standard routes. Administration can be systemic or local or intranasal or intrathecal.

Further intended are embodiments wherein the composition of some embodiments of the invention is administered separately to or in combination with alternative antitumoral or otherwise anti-cancer therapeutic components. These components can include oncolytic viruses, small molecule drugs, chemotherapeutics, radiotherapeutics or biologicals. The components may be administered concurrently with the composition of the invention, and may be comprised within delivery particles, or may be administered separately, before or after administration of the composition of the invention, by any means suitable.

It is also contemplated that the composition of some embodiments of the invention may be used in *in vitro* and/or *ex vivo* methods, for example in a laboratory setting. An example of an *in vivo* method is wherein a composition including a delivery system comprising an mRNA sequence as described herein is administered to target *in vitro* cells, and the miRNA binding site sequences comprised in the mRNA sequence allow for differential expression of the coding sequence of the mRNA in different cell types within the target in vitro cells. Similarly, a method is contemplated wherein a composition comprising a delivery system and an mRNA sequence as described herein is administered to a target *ex vivo* sample taken from an animal, and the miRNA binding site sequences comprised in the mRNA sequence allow for differential expression of the coding sequence of the mRNA in different cell types within the target sample.

The device of the invention is exemplified by, but in no way limited to, the following Examples.

### EXAMPLES

### General Protocols

### Cell lines

Human liver hepatocarcinoma (HCC) HepG2 (ATCC^{®} HB-8065^{™}) and Hep3B (ATCC^{®} HB-8064^{™}) cell were purchased from ATCC. Cells were cultured in Eagle's Minimum Essential Medium (EMEM) (Cellgro, USA), 10 % FBS (HyClone, USA), streptomycin (100 µg/mL) and penicillin (100 U/mL⁻¹) (Cellgro) as monolayers at 37°C in an atmosphere of 5% CO₂. HepG2 cells were grown on collagen (Gibco, USA) coated plates at a collagen concentration of 5 µg/cm².

HMCPP5 (pooled plateable human hepatocytes; a mixture of plateable primary hepatocytes produced by combining cells from 5 individual donors) were purchased from ThermoFisher Scientific, USA. Cells were plated in Williams E Medium (WEM), supplemented with 5% FBS, 1 µM Dexamethasone, and Cocktail A (Penicillin/Streptomycin, Human Recombinant Insulin, GlutaMax, and HEPES, pH 7.4). 24 hours after plating, the WEM/Cocktail A medium was changed to maintenance/incubation medium WEM supplemented with 0.1 µM Dexamethasone and Cocktail B (Penicillin/Streptomycin, ITS (Human Recombinant Insulin, Human transferrin, selenous acid, BSA, linoleic acid), GlutaMax, and HEPES, pH 7.4) as monolayers at 37°C in an atmosphere of 5% CO₂. During all experiments, cells were cultured in WEM/Cocktail B medium with the exception of during transfection with nanoformulated mRNA. WEM/Cocktail B medium was exchanged for fresh every 3 days. HMCPP5 cell growth on collagen (Gibco) coated plates at protein concentration 5 µg/cm².

Aml12 (mouse healthy hepatocytes) were purchased from ATCC, USA. Cells were seeded into a 12-well plate at a density of 1×10⁵/well.

### Vector Constructs

### Constructing of pMRNA-CTx-mRNA Template

Plasmid pMRNA-CTx-mRNA template forming matrices for in vitro synthesis of all mRNA used in the experiments were constructed according to commercially available mRNAExpress^{™} mRNA Synthesis Kit (SBI, USA). All plasmids were propagated in *E.coli* (Invitrogen, USA) and purified using Qiagen Mini or Maxi Kit (Qiagen, USA). Restriction maps of all plasmids were generated using pDRAW32 software (www.acaclone.com).

### Cloning method 1 - restriction endonuclease

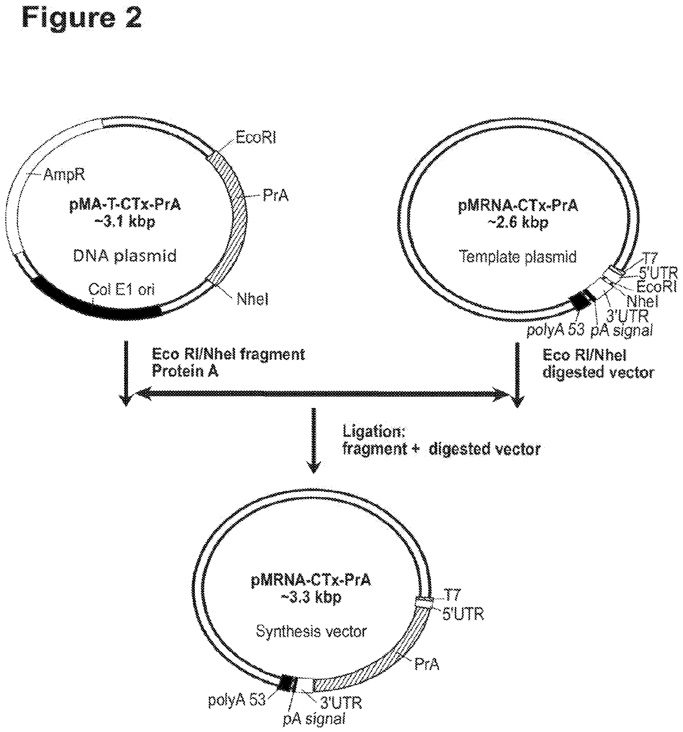

As shown in Figure 2, the sequence of one or more genes, flanked with Kozak sequence for optimal translation directly before the ATG codon at the 5'-end or a stop codon (TAA, TAG, TGA) at the 3'-end of gene, were synthesised by company GeneArt (without codon optimisation) and delivered as plasmid DNA (referred to as DNA plasmids or vectors), shown in Figure 2 as pMA-T-CTx-Gene. Proprietary 5' and 3' UTR regions flanking the coding sequence were included in all synthesised sequences (not shown in the appended sequences). The 5' UTR is synthetic, and contains the Kozak sequence, and the 3' UTR is based on a mouse alpha globin UTR and also comprises a poly A tail of 120 bases. To generate the synthesis vector shown in Figure 2 as pMRNA-CTx-mRNA, a nucleotide fragment containing the gene or genes was cut out from the DNA plasmid using restriction endonucleases, here EcoRI and Nhel, and subcloned into EcoRI/Nhel restriction sites in the pMRNA template plasmid, this template plasmid comprising T7 promoter recognized by T7 RNA polymerase, 5'- and 3'-UTRs, and a polyA sequence.

### Cloning method 2 - cold fusion

The sequence of one or more genes, flanked as in Cloning Method 1 with a Kozak sequence and a stop codon (TAA, TAG, TGA), was synthesised by company GeneArt (without codon optimisation) and delivered as a plasmid DNA pMAT-CTx-Gene, with the backbone of this plasmid the same as described above. To construct the pMRNA-CTx-mRNA template vector, the Cold Fusion cloning kit (SBI, USA) was deployed. Briefly, the gene sequence from the DNA plasmid was amplified by PCR with specific primers, the primers adding an extension of 14 bases of homology to each end of the gene sequence. These 14 bases were designed to be homologous to the ends of the linearised vector produced by digestion of the template plasmid with a restriction endonuclease cutting in the multicloning site located between the 5' and 3' UTRs. To produce the synthesis vector, the predicted PCR product was purified by PCR purification kit (Qiagen, USA) and incorporated to the pMRNA template plasmid following a Cold Fusion reaction (homology recombination) according to the manufacturer's protocol.

### Construction of a template containing miRNA binding site sequences

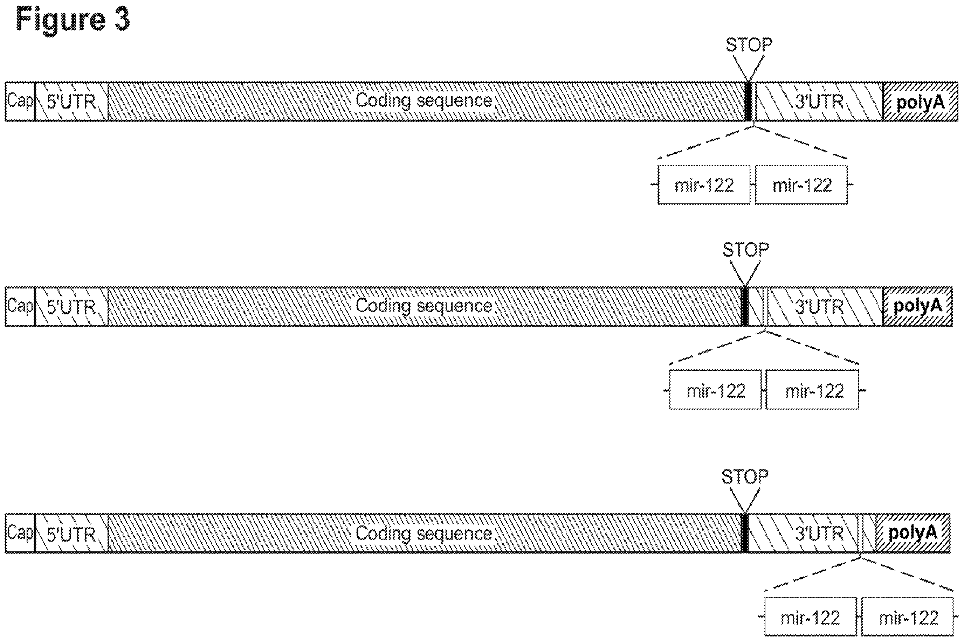

For the production of mRNA sequences comprising miRNA binding site sequences, for which examples using miR-122 are shown in Figure 3, exemplary methods of creating three variants are discussed below. In variant 1, two copies of the miRNA binding site sequence are included between the stop codon and the +1 position of the 3' UTR. In variant 2, two copies of the miRNA binding site sequence are included at the beginning or 5' end of the 3' UTR, and in variant 3, two copies of the miRNA binding site sequence are included at the end or 3' end of the 3' UTR.

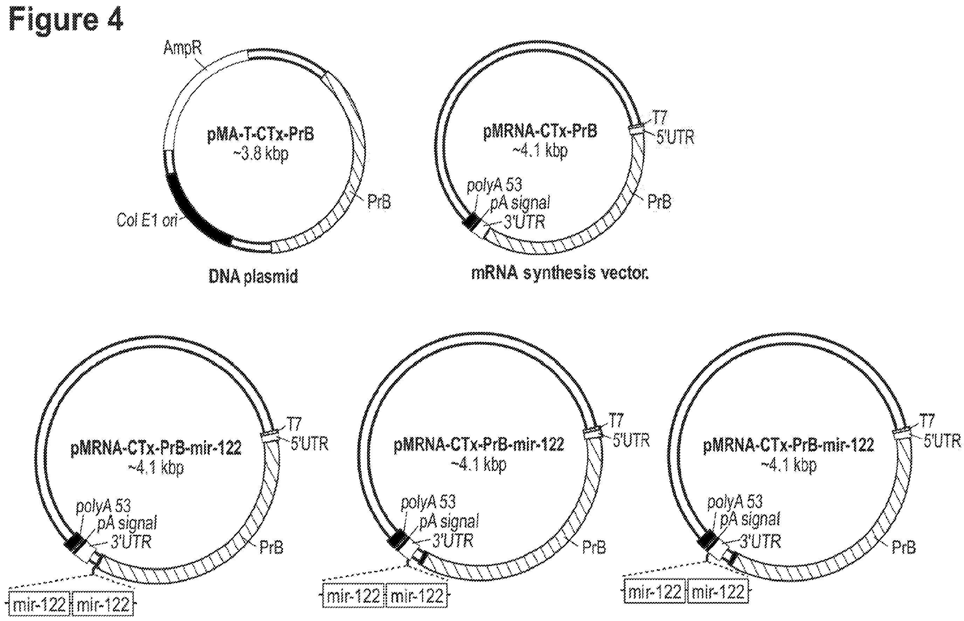

Figure 4 shows examples of synthesis vectors comprising these three variants, using as an example Protein B, a gene of approximately 1400 base pairs.

### Variant 1

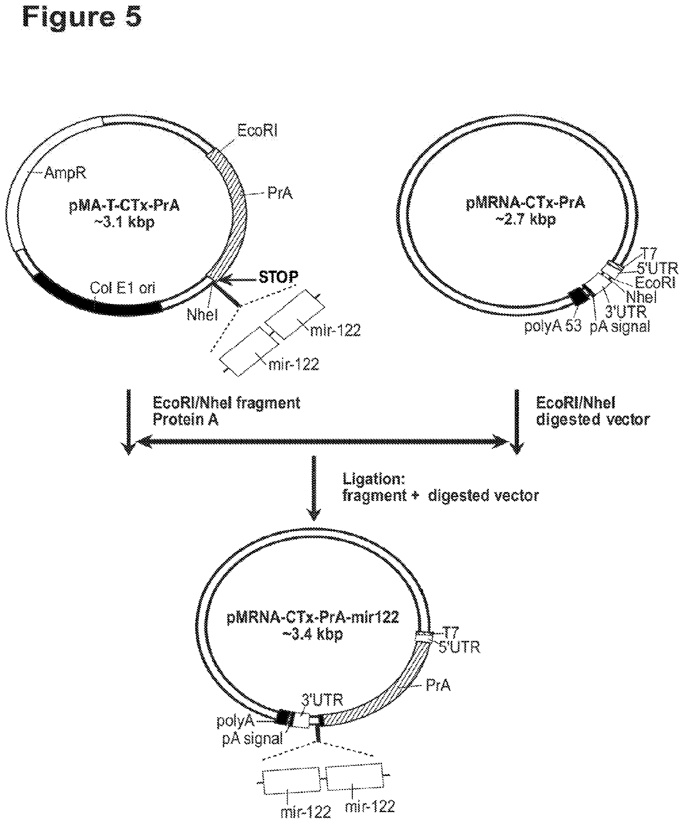

As shown in Figure 5, the sequence of one or more genes, flanked as in the above methods with a Kozak sequence and a stop codon (TAA, TAG, TGA), and additionally comprising two copies of a miRNA binding site sequence following the stop codon, was synthesised by company GeneArt (without codon optimisation) and delivered as a plasmid (here illustrated again with Protein A) DNA pMAT-CTx-Gene, with the backbone of this plasmid the same as described above. This sequence was then cloned into the template plasmid to create a synthesis vector by either of the methods described above.

### Variants 2 and 3

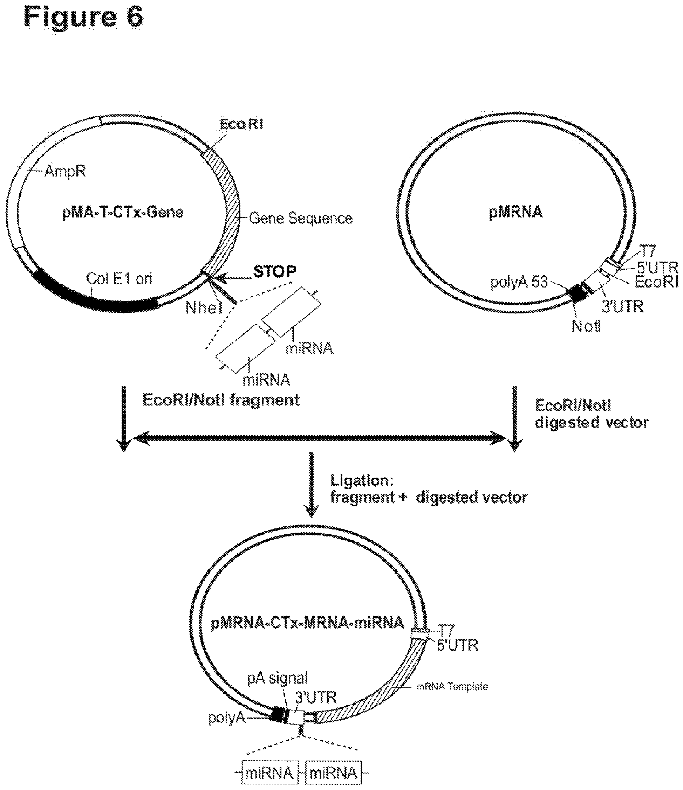

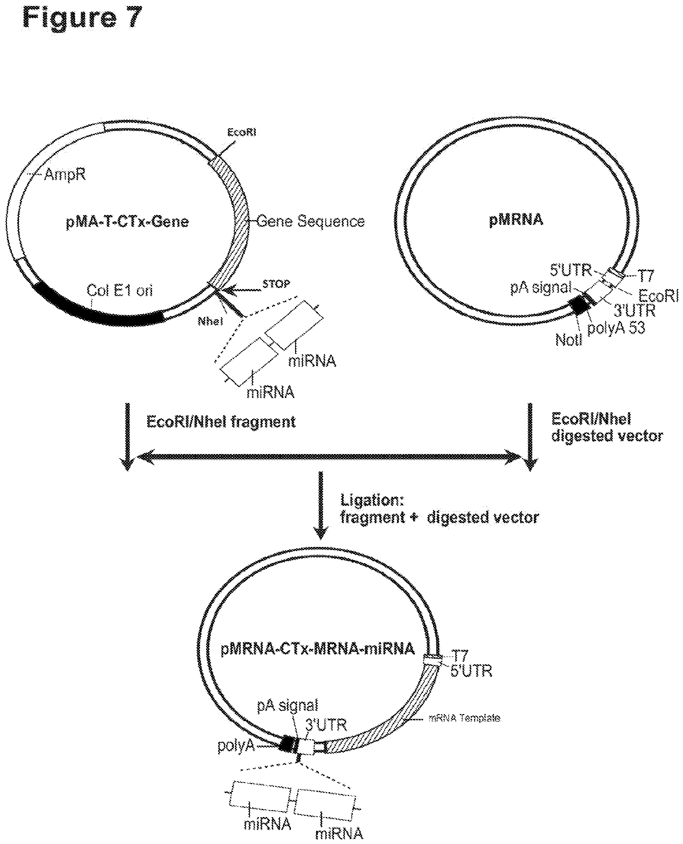

The sequence of one or more genes, flanked as in the above methods with a Kozak sequence and a stop codon (TAA, TAG, TGA), and additionally comprising a 3' UTR, including two copies of a miRNA binding site sequence either at the beginning/5' end of this region (variant 2, as shown in Figure 6) or at the end/3'end of this region (variant 3, as shown in Figure 7), was synthesised by company GeneArt (without codon optimisation) and delivered as a plasmid DNA pMAT-CTx-Gene, with the backbone of this plasmid the same as described above. This sequence was then cloned into a template plasmid to create a synthesis vector by either of the methods described above, modified in that restriction enzymes were chosen (here EcoRI and Notl) to remove the 3' UTR from the template vector, such that the 3' UTR from the supplied DNA sequence would be present in the final synthesis vector, as this contained the miRNA binding site sequences.

### In vitro transcription (IVT) of mRNA with in vitro mRNA synthesis

To perform IVT of mRNA with or without miRNA-modified 3' UTRs the commercially available mRNAExpress^{™} mRNA Synthesis Kit was used. DNA templates for IVT vectors were constructed as described in the protocols as set forth above. The procedure of in vitro mRNA synthesis was performed according to the manufacturer's protocol. Briefly, a polyA tail was added to the DNA sequence using a PCR reaction with specific 5' and 3' primers (provided with the kit). During in vitro transcription, the synthesised mRNA on DNA template was capped with anti-reverse cap analog (ARCA)-modified nucleotides (5-Methylcytidine-5'-Triphosphate). Cap analog, pseudouridine-5'-triphosphate and poly-A tail were incorporated in the in vitro transcribed mRNAs to enhance stability and to reduce the immune response of host cells.

### Synthesis of DM^{pCTx} and formulation of mRNA

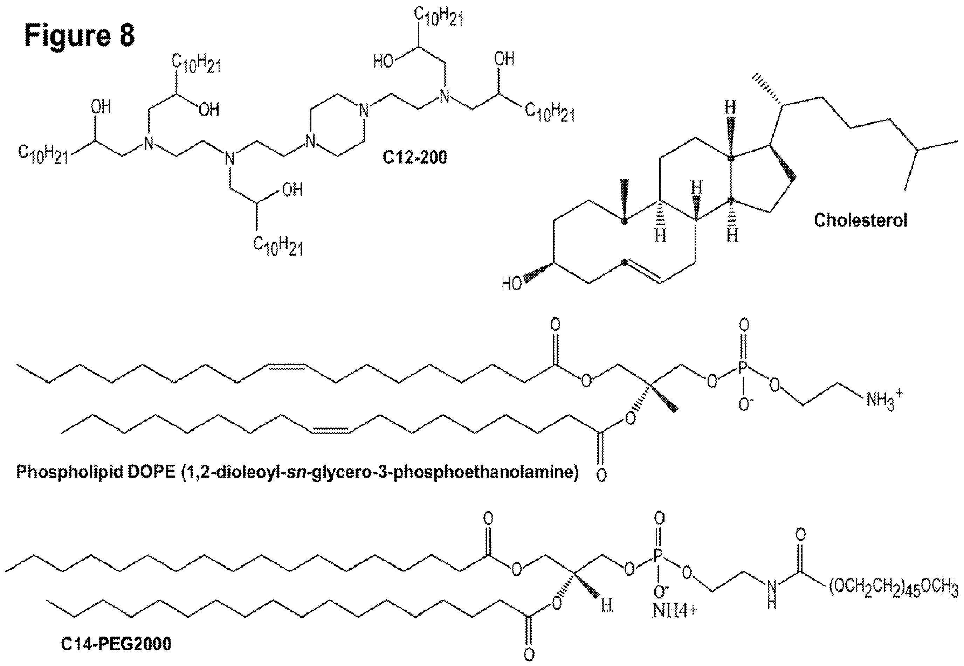

The delivery and modulation platform of Combined Therapeutics (DMP^{CTx}) formulation is a multi-component nanoparticle of ionizable lipid-like material C12-200, phospholipid DOPE, cholesterol and lipid-anchored polyethylene glycol C14-PEG-DSPE2000 mixture. This particular composition of DM^{pCTx} and specific weight ratio (10:1) of C12-200:mRNA and molar [%] composition of lipid-like material, phospholipid, cholesterol and PEG (**Table 4**) was optimized and has revealed high efficiency of the formulation in vivo (Kauffman K.J., Nano Letter. 2015, 15, 7300-7306). The chemical structures of these exemplary components are shown in Figure 8.

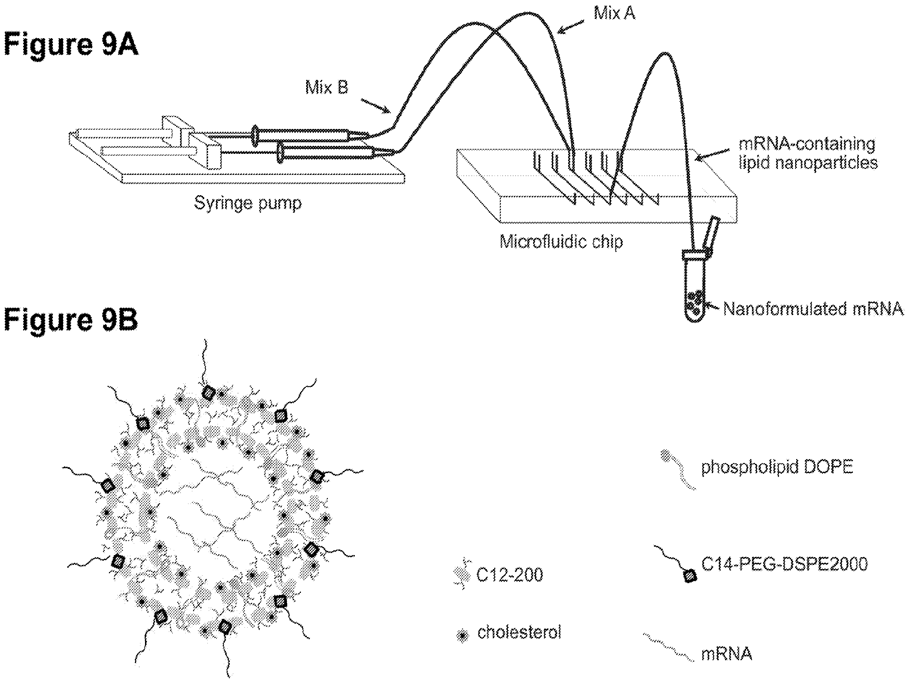

To synthesize DM^{pCTx} an ethanolic solution (Mix A) of C12-200 (WuXi, China), as shown in Figure 9A, phospholipid DOPE (1,2-dioleyl-snglycero-3-phosphoethanolamine) (Avanti Polar Lipids, Alabaster, AL, USA), cholesterol (Sigma, USA) and C14-PEG-DSPE2000 (Avanti Polar Lipids, Alabaster, AL, USA) and an aqueous buffered solution of mRNA (Mix B) in (10 mM citrate, pH 4.5) was prepared. Both ethanolic Mix A and aqueous Mix B at ratio 3:1 were mixed/combined using syringe pumps and microfluidic chip device (Chen D, at al J. Am. Chem. Soc. 2012, 134 (16), 6948-6951). Alcoholic solution of nanoformulated mRNA from microfluidic chip was collected to the 1.5 mL tubes.

**Table 4**

| Compound | Weight Ratio | Formulation Molar Composition [%] |
|---|---|---|
| C12-200:mRNA | 10:1 | n/a |
| C12:200 | n/a | 35 |
| DOPE | | 16 |
| Cholesterol | | 46.5 |
| C14-PEG-DSPE200 | | 2.5 |

To remove alcohol after formulation the DMP^{CTx}-mRNA mixture was transferred to the Slide-A-Lyzer^{®} Dialysis Cassette G2 and dialyzed in PBS in room temperature on the magnetic stirrer for 4 hours. Subsequently, formulated mRNA using syringe with 18 gauge, 1-inch beveled needles was transferred to new 1.5 mL tubes and ready for characterization.

To calculate efficacy of mRNA encapsulation RiboGreen RNA assay (Invitrogen) was used according manufacture protocol. Polydispersity (PDI) and size of lipid nanoparticles was measured using dynamic light scattering (ZetaPALS, Brookhaven, Instruments). The surface charge of DM^{pCTx} (Zeta potential) was measured using the same instrument. Solutions of mRNA sequences with and without two copies of an miR-122 sequence connected by a linker (SEQ ID NO: 30) and inserted after the stop codon of the coding mRNA sequence were prepared from a 1.05 and 1 mg/ml stock, respectively. Examples of parameters after encapsulation of mRNA sequences comprising the mCherry (mCh) sequence, the sequence of protein A, a human protein of approximately 25 kDa, are shown in Table 5, including the size, encapsulation efficiency and polydispersity of the delivery and modulation platform of Combined Therapeutics (DMP^{CTx}). An illustrative diagram of a delivery particle according to DMP^{CTx} may be seen in Figure 9B.

**Table 5**

| mRNA | Formulation | Conc (ug/mL) | Encapsulation efficacy (%) | Size (nm) | Polydispersity |
|---|---|---|---|---|---|
| Protein A - 022 | C12-200 | 202 | 78 | 93 | 0.12 |
| US3 - 052 | | 172 | 78 | 93 | 0.12 |
| mCherry - 062 | | 120 | 76 | 96 | 0.12 |

### Differential expression of delivered mRNA constructs in vitro

To investigate the potential of the present invention to successfully transfect target cells with construct mRNA and subsequently drive differential expression in different cell types, the DMP^{CTx} mRNA platform, modified with miRNA-122 binding sites, was used in a model of liver hepatocarcinoma.

### Transfection of cell lines

### Fluorescence imaging and quantification

Single transfections of the human liver hepatocarcinoma cell lines HepG2 and Hep3B were performed as follows: one day prior to transfection, HepG2 and Hep3B cells were seeded separately into a 12-well plate at a density of 2.7×10⁵/well, and 2×10⁵/well (EMEM/10%FCS), respectively. The next day, cells were transfected either with a vehicle control of PBS alone, with 0.5 µg/well of mRNA-mCherry-DMP^{CTx}, or with 0.5 µg/well of mRNA-mCherry-122-DMP^{CTx} (the sequence comprising SEQ ID NO: 31). The transfection was carried out by direct addition of mRNA-DMP^{CTx} to the cultured medium in the well, with gentle mixture of the cultured cells as needed.

Single transfections of HMCPP5 (pooled plateable human hepatocytes), were performed as follows: one day prior to transfection HMCPP5 cells were seeded into a 12-well plate at a density of 2.5×10⁵/well (WEM/Cocktail B). The next day, cells were transfected either with a vehicle control of DM^{pCTx} (PBS), with 0.5 µg/well of mRNA-mCherry-DMP^{CTx}, or with 0.5 µg/well of mRNA- mCherry-122-DMP^{CTx}. The transfection was carried out by the direct addition of mRNA-DMP^{CTx} to the cultured medium in the well, with gentle mixture of the cultured cells as needed. During transfection of HMCPP5 the WEM/Cocktail B medium was supplemented with 5% FBS. Transfection was carried out in the manner described for liver cancer cells, above. 24 hours after transfection, the medium was again changed to WEM/Cocktail B.

To evaluate constitutive activity and expression of miRNA-122 in healthy human hepatocytes, multiple transfections of HMCPP5 cells were performed as follows: the HMCPP5 cells were seeded and cultured as above, and were transfected with mRNA-mCherry- DMP^{CTx}, or mRNA-mCherry-122-DMP^{CTx}" three times (MPT) in total, with an interval of 48 hrs between each transfection. Transfection was carried out in the same manner described for single transfections of HMCPP5, as described above.

Single transfections of mouse healthy hepatocytes (Aml12, ATCC, USA) were performed as follows: one day prior to transfection Aml12 cells were seeded into a 12-well plate at a density of 1×10⁵/well. The next day, cells were transfected either with a vehicle control of DM^{pCTx} (PBS), with 0.5 µg/well of mRNA-mCherry-DMP^{CTx}, or with 0.5 µg/well of mRNA- mCherry-122-DMP^{CTx}. The transfection was carried out by the direct addition of mRNA-DMP^{CTx} to the cultured medium in the well, with gentle mixture of the cultured cells as needed.

Following transfection, mCherry expression in the above cell lines was detected using a fluorescence imaging system (application from EVOS^{®} FL Imaging Systems). Pictures showing mCherry fluorescence were taken 16, 24, 48, 72, 96 and 144 hours after transfection.

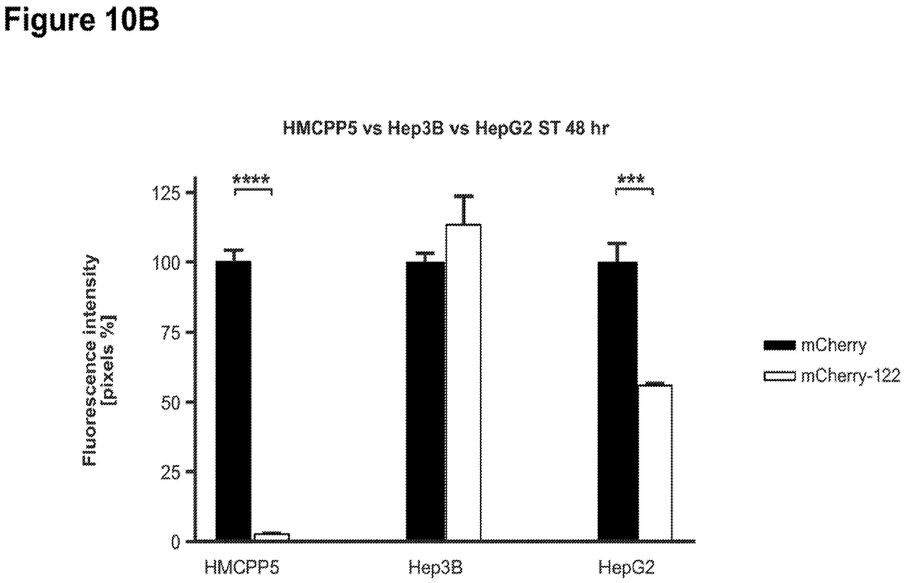

Quantification of the mCherry fluorescence signal was performed using ImageJ software (NIH, USA) from 3 randomized fields on culture plates (mRNA-mCherry, mRNA-mCherry-122). Figures 10B, 11 and 12B show the results of such quantifications. The pixel count of mCherry transfected wells were set at 100% (mCherry fluorescence). Statistical significance was determined using the Student *t*-test. Results are shown as means ± SD. Significant difference was defined with p value <0.05. Asterisks indicate a statistically significant difference between mCherry fluorescence in cells transfected with mRNA-mCherry, compared to cells transfected with mRNA-mCherry-122 (****, p < 0.0001, ***p < 0.001, **p < 0.01, *p < 0.05).

### Example 1: Tumor-specific gene expression by miRNA-122 regulation

miRNA-122 is an abundant, liver-specific miRNA, the expression of which is significantly decreased in human primary hepatocarcinoma (HCC) and HCC derived cell lines such as Hep3B and HepG2. The objective of this study was to demonstrate that modification of the 3'-untranslated region (UTR) of an mRNA sequence by the insertion of miRNA-122 targeted sequences (for example, SEQ ID NO: 30, as illustrated in variant 1, top of Figure 3) may result in translational repression and/or deadenylation followed by decapping of exogenous mRNA in normal hepatocytes, but not in tested HCC cell lines.

To examine endogenous miRNA-122 activity in healthy hepatocytes, HMCPP5 cells (pooled plateable human hepatocytes, which are a mixture of plateable primary hepatocytes produced by combining cells from 5 individual donors) were transfected with mRNA-mCherry or mRNA-mCherry-122 prepared according to the above general protocols, using mCherry (red fluorescent protein) as the introduced gene of interest and followed mCherry (red fluorescent protein) expression over time. As illustrated in Figure 10A, mCherry (mCh) expression was analyzed by fluorescence microscopy 48 hours post-transfection. During the entirety of the post-transfection time, mCherry expression was observed in HMCPP5 cells transfected with mRNA-mCherry (that is, without the 3'UTR modification to introduce an miR-122 sequence), indicating successful transfection and translation. In contrast, in healthy hepatocytes, which are known to be miRNA-122 positive, the expression of mRNA-mCherry-122 was downregulated to practically undetectable levels comparable to those seen in control untransfected cells, even 3 days after transfection. This indicated that the presence of an miRNA-122 targeted sequence in the mRNA-mCherry-122 inserted in 3' UTR (Variant 1) prevents translation of the mRNA, most likely due to translational repression in recipient cells.

Quantification of the fluorescence signal exhibited by these cells confirmed the above. As shown in Figure 10B, fluorescence intensity was drastically reduced in healthy cells transfected with mRNA-mCherry-122, compared with those transfected with mRNA-mCherry.

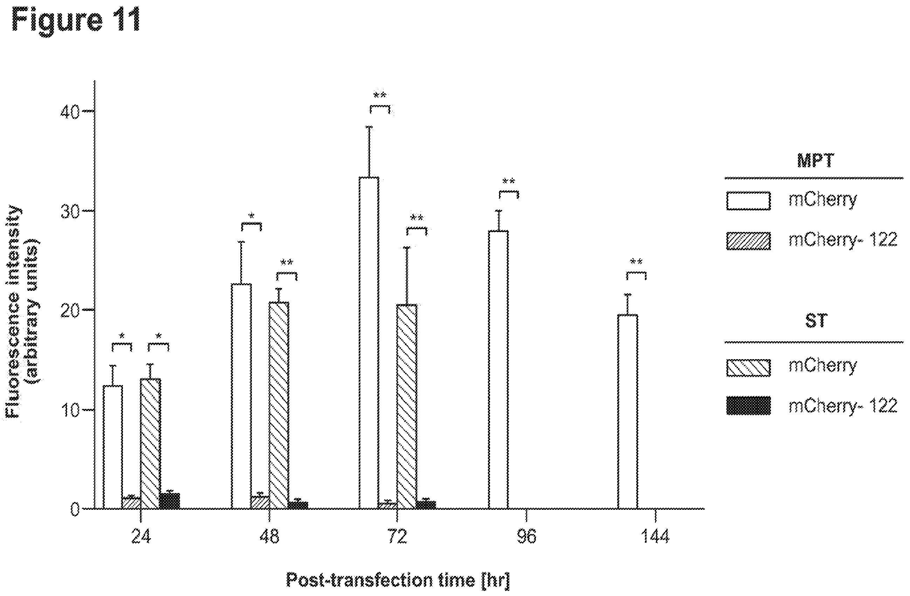

The result obtained in the experiment above showed that native expression of miRNA-122 and colocalisation with an miRNA-122 targeted sequence (Variant 1) could efficiently regulate protein expression in healthy hepatocytes thereby significantly increasing tumor-specific gene expression. In the following experiment, the constitutive expression and activity of miRNA-122 in HMCPP5 cells was evaluated. The HMCPP5 cells were transfected with mRNA-mCherry or mRNA-mCherry-122 three times in total, with an interval of 48 hr each time. Six days after the first transfection (that is, 48 hours after the last transfection) expression of mCherry was determined by fluorescent microscopy. As before, while cells transfected with the mRNA-mCherry construct exhibited clear red fluorescence, those transfected with the mRNA-mCherry-122 construct did not. In Figure 11, comparisons between cells transfected with mRNA-mCherry-122, and those transfected with mRNA-mCherry are shown over a fiveday period after final transfection, both for singly (ST) and multiply-transfected cells (MPT). Multiply-transfected cells can be seen to exhibit the same drastic reduction in fluorescence intensity when transfected with mRNA-mCherry-122 as singly-transfected cells, with the effect lasting longer following multiple transfections. As would be expected, this indicates that the differential expression effect driven by the miRNA control mechanism is robust to repeated transfection events, and that the amount of miRNA-122 available within the cells to drive this mechanism is not exhausted in these timeframes.

To examine the effect of endogenous miRNA-122 activity using the human liver hepatocarcinoma Hep3B and hepatoblastoma HepG2 cell lines, an experiment similar to the above was carried out. Cells were transfected with the mRNA sequence mRNA-mCherry, mRNA-mCherry-122 (Variant 1), or underwent a control transfection. As previously, after 48 hours, fluorescence microscopy was used to determine the expression of mCherry in the transfected Hep3B and HepG2 cells, as shown in **Figure 10A****.** In Hep3B cells (Figure 10A, middle column), mCherry fluorescence was clearly seen both in the mRNA-mCherry and the mRNA-mCherry-122 transfected lines, indicating that the miRNA-122 mediated translation repression is not active in these cells. In HepG2 cells, mCherry fluorescence was clearly seen in the mRNA-mCherry transfected line, but while some fluorescence was evident in the mRNA-mCherry-122 transfected cells, it appeared to be only partially reduced and significantly greater than that seen in normal hepatocytes. Further evidence of this is shown in **Figure 10B****,** where quantification of mCherry fluorescence in mRNA-mCherry-122 transfected lines indicates that no reduction of fluorescence is shown in Hep3B cells, but a reduction of around 50% is seen in the HepG2 cells.

The partial downregulation seen in HepG2 cells further implicates miRNA-122 mediated effects on translation, as cells from this line have been shown to retain residual miRNA-122 activity (Demonstration of the Presence of the "Deleted" MIR122 Gene in HepG2 Cells, PLoS One. 2015; 10(3)).

miRNA-122 is strongly conserved between vertebrate species, and, as in the human, a reduced level of miRNA-122 is associated with hepatocellular carcinoma in mouse (Kutay et al, 2006).The endogenous effect of miRNA-122 activity was therefore examined, using the mouse healthy hepatocyte cell line Aml12.

Healthy mice hepatocytes were also transfected with the mRNA-Cherry sequences previously described, i.e. mRNA-mCherry or mRNA-mCherry-122, encapsulated in DMP^{CTx}. A similar impact of the insertion of miRNA-122 binding site sequence on the mCherry fluorescence was observed at 24 and 72 hours after transfection.

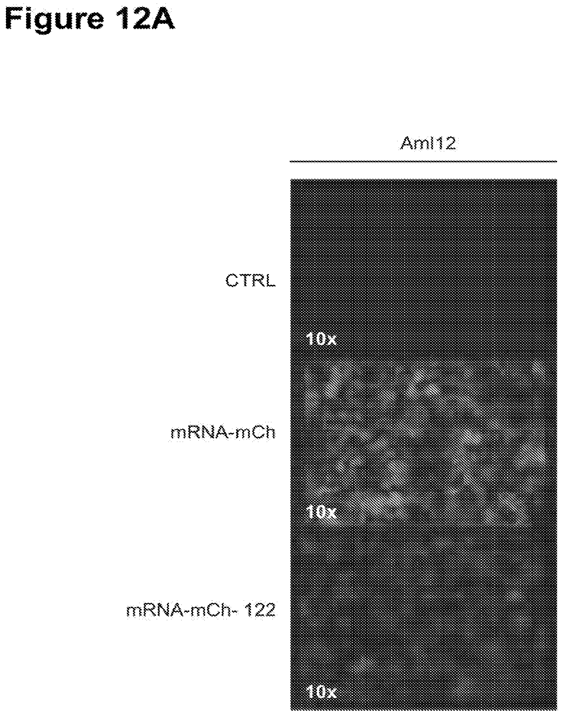

As shown in Figure 12A, fluorescence was observed after transfection with mRNA-mCherry. A marked reduction in fluorescence was shown after transfection with mRNA-mCherry-122, although some signal could still be seen.

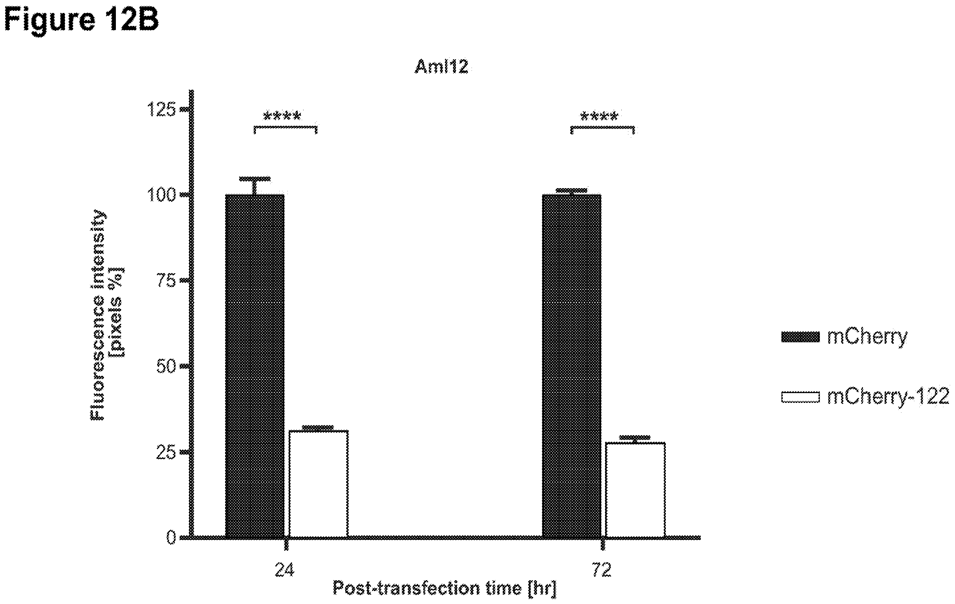

Quantification of mCherry fluorescence 24 and 72 hr after transfection was performed from 3 randomised fields on culture plates from each treatment group (Figure 12B) showed that when transfected with mRNA-mCherry-122, more than 70% translation repression was observed.

As a preliminary conclusion, the above Example shows that the double targeting features of the nanoparticle delivery system, and the inclusion of miRNA-122 target sequence in the mRNA construct are sufficient to obtain quite significant differential expression of a protein product in hepatocarcinoma and hepatoblastoma cells compared to healthy hepatocytes. The observation of differential expression was evident in both human and mouse cell lines.

### Example 2: Protein expression level after tumor-specific gene expression

In another experiment, Western blotting was employed to determine protein expression level ultimately exhibited after transfection as follows.

### Transfection of cell lines and immunoblot - protein A

To evaluate tumor specific expression level of an exemplary 25 kDa human protein (denoted 'protein A') both liver cancer cells (HepG2 and Hep3B) and healthy hepatocytes (HMCPP5) were seeded into 12-well plates and transfected with 0.5 µg/well of nanoformulated mRNA expressing human protein A, 25 kDa (mRNA-A-DMP^{CTx}) or mRNA expressing human protein A (a human protein of approximately 25 kDa) comprising two miRNA122 binding sequences in the 3' UTR (SEQ ID NO: 30), Variant 1 (mRNA-A-miRNA122-DMP^{CTx}), as described above in Example 1 for mCherry transfection. 24 hours after transfection, immunoblot was performed following total protein extraction.

For the immunoblot, culture media was removed, cells were washed with cold PBS (Cellgro) and cell pellets lysed in RIPA (radioimmunoprecipitation assay) buffer (Boston Bioproducts) with a cocktail of protease inhibitors (Sigma). Protein concentration was determined by colorimetric Bradford assay. A total of 10 mg of protein was separated by Novex^{™} 4-12% mini gels (ThermoFisher Scientific) and transferred onto PVDF (polyvinylidene difluoride) membranes by electroblotting (iBlotⓇ 2 Gel Transfer Device, Invitrogen). After blocking with 5% nonfat dry milk in TBS-Tween 20 (Boston Bioproducts), membranes were incubated at 4°C overnight with anti-protein A antibodies (1:2000, Abcam), or β-actin (Cell Signaling), followed by incubation with appropriate HRP (horseradish peroxidase)-conjugated goat anti-rabbit secondary antibodies (1:10000; Abcam) for 1 hour at room temperature. Protein-antibody complexes were visualized and imagined using Clarity^{™} Western ECL Substrate (Bio Rad) and LI-COR^{®} system (LI-COR), respectively.

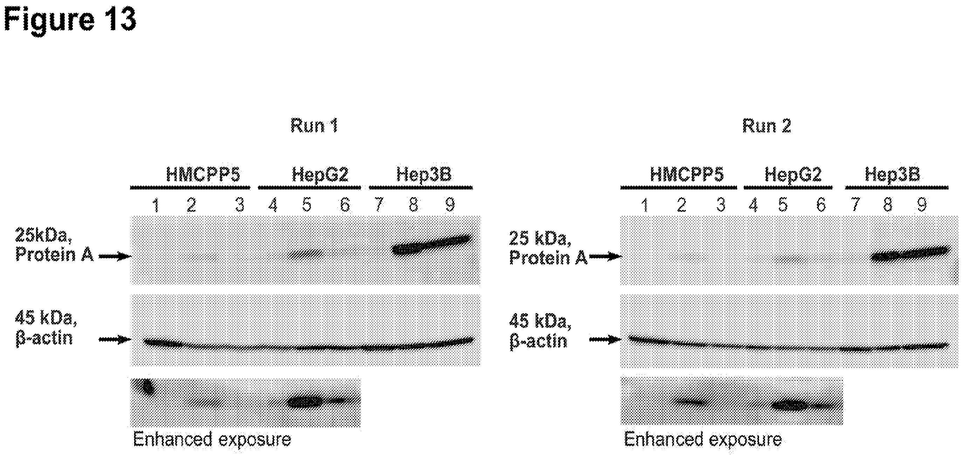

The results of the above can be seen in Figure 13, with the following transfection constructs encapsulated in DMP^{CTx} shown:
Lanes 1, 4 and 7 of Figure 13: vehicle (mock treated, PBS only),
Lanes 2, 5 and 8 of Figure 13: mRNA-A (mRNA comprising the sequence for protein A), and
Lanes 3, 6 and 9 of Figure 13: mRNA-A-122 constructs (comprising the sequence for protein A and miRNA122, inserted in the variant 1 position, as illustrated in Figure 3).

Transfection was carried out in healthy hepatocytes (HMCPP5) in lanes 1-3, hepatocarcinoma model Hep3B in lanes 7 to 9 and cells from the hepatoblastoma model HepG2 in lanes 4 to 6 as described above, using 0.5µg mRNA-DMP^{CTx} per well. Protein was extracted from each cell line 24 hours after transfection. 10 µg of protein was loaded into each lane, and data was taken from two independent experiments. Protein A was detected in all tested cell lines when transfected with mRNA-A, indicating that successful transfection was achieved. While transfected with mRNA-A-122, translation repression was observed only in healthy hepatocytes, but not in Hep3B and HepG2 cells (lanes 3, 6 and 9), indicating that the miRNA-122 does not fulfil its function in tested liver cancer cells. However, for HepG2 cells transfected with mRNA-A-122, expression of protein A was slightly downregulated compared to cells transfected with mRNA-A, similar to the pattern previously seen for mCherry expression, in Example 1. This can be seen clearly in the enhanced exposure photographs (bottom) which show incomplete downregulation in the HepG2 cells. The partial downregulation seen in HepG2 cells further implicates miRNA-122 mediated effects on translation, as cells from this line have been shown to retain residual miRNA-122 activity (Demonstration of the Presence of the "Deleted" MIR122 Gene in HepG2 Cells, PLoS One. 2015; 10(3)).

In summary, modification of 3'UTR mRNA by inserting liver-specific miRNA-122 target sequence can significantly confine mRNA translation to hepatocarcinoma Hep3B and hepatoblastoma HepG2, but not in normal human hepatocytes.

### Example 3: Oncolytic viral combination therapy in vitro

It is described herein that the differential expression of provided mRNA constructs allowed by the method of the invention, and shown in the above Examples, can be used in combination with oncolytic viral therapy. In particular, where oncolytic viruses have been modified to remove virulence genes, attenuating their replicative ability in healthy cells, the invention can be used to restore the function of those genes, or equivalents thereof, in diseased cells such as cancer cells. To investigate this possibility, the combination of the oncolytic virus HSV-1 (R7041), deficient in US3 (see Leopardi et al, 1997, PNAS 94; 7891-7896), and the DMP^{CTx} platform, providing an mRNA construct coding for US3, and modified with miRNA-122 binding sites, was used in a model of liver hepatocarcinoma (SEQ ID NO: 32).

### General Protocols:

### Cell culture

Human liver hepatocarcinoma (HCC) HepG2 and Hep3B cells were cultured in Eagle's Minimum Essential Medium (EMEM, Cellgro, USA), 10 % FBS, streptomycin (100 µg/mL) and penicillin (100 U/mL-1) as monolayers, at 37°C and in an atmosphere of 5% CO2. HepG2 cells were grown on collagen coated plates at a collagen concentration of 5 µg/cm2.

### Virus preparation

Frozen R7041 virus was thawed in a water bath at 37°C, and sonicated for 30 sec using a bath sonicator (Q500 sonicator, Qsonica, USA), then transferred to ice, ready for use.

### Toxicity of R7041 alone against human HCC

The US3 mutant R7041 virus is thought to be virtually apathogenic to healthy cells (Leopardi et al. 1997) and has even shown good safety in immunodeficient, athymic mice (Liu et al. 2007, Clin Cancer Res 2007;13(19)). To establish a baseline of the efficacy of the R7041 virus against liver hepatocarcinoma cells, the model cell lines were treated with oncolytic virus alone. Cells from the Hep3B and HepG2 lines were seeded, in triplicate, into 96-well plates, at 15,000 and 17,000 per well, respectively.

24 hours later, cells were infected with 3-fold serial dilutions of virus, from MOI 0.37 to 0.0001694. 96 hours post-infection, the viability of tested cell lines was measured by MTS assay according to vendor instructions (CellTiter 96^{®} AQueous One Solution Cell Proliferation Assay, Promega, USA). Absorbance was measured at 490nm with a 96-well plate reader (BioTek, Cytation 3, USA). Dose-response curves and 50% effective dose values (ED₅₀) were obtained using GraphPad Prism, 7.03.

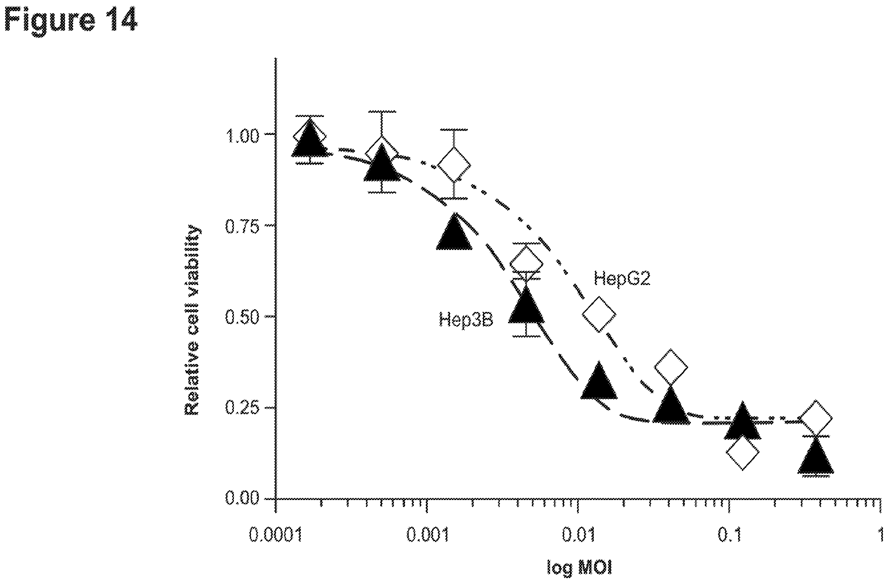

As shown in Figure 14, both Hep3B and HepG2 cell lines exhibited similar susceptibility to R7041, with ED₅₀=0.01 and 0.02 MOI, respectively. However, Hep3B cell lines were seen to be slightly more susceptible to R7041, than were HepG2 cells.

### The combinatorial effect of R7041 and of mRNA-DMP^{CTx} on human HCC viability

Prior to evaluation of the combinatorial effect of R7041 virus and mRNA-US3-DMP^{CTx} on human hepatocarcinoma cells,, we verified that transfection of Hep3B and HepG2 cells with mRNA-US3-DMP^{CTx} at 0.04 µg/mL mRNA-US3 has no significant effect on cell viability, as measured by the MTS assay.

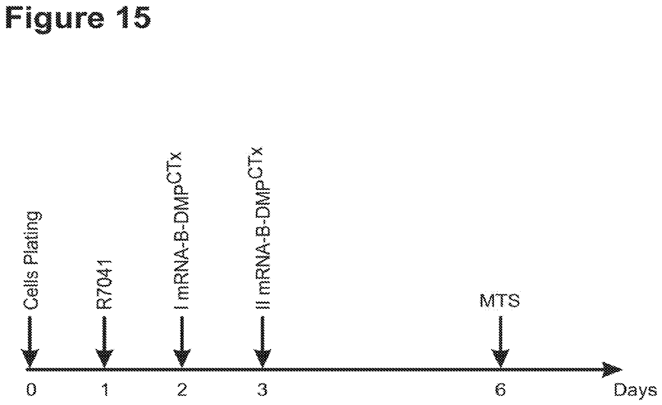

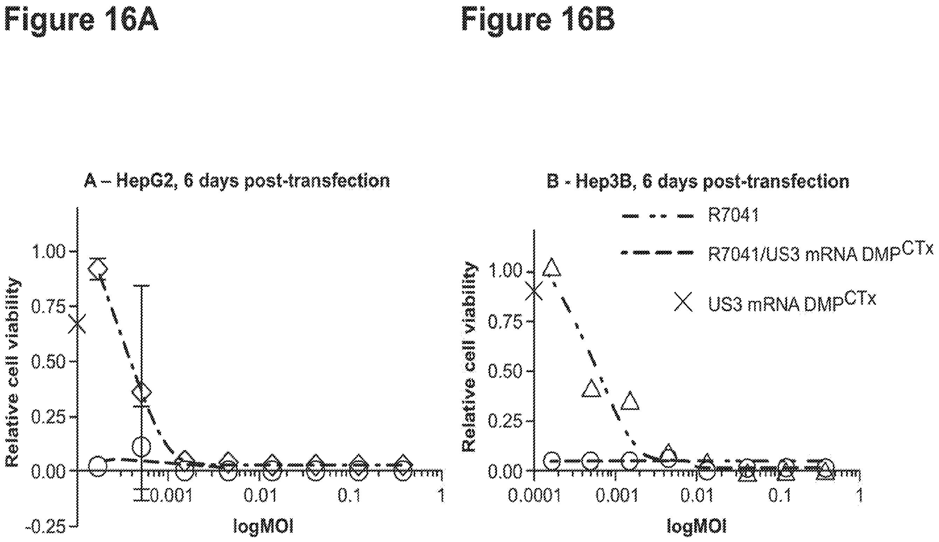

Hep3B and HepG2 cells were seeded in triplicate into 96-well plates at 15,000 and 17,000 per well, respectively. 24 hours later, cells were infected with 3-fold serial dilutions of virus, starting from MOI 0.37 up to 0.0001694. Both tested cell lines were transfected twice with a fixed dose of 0.04 µg/mL mRNA-US3-DM^{CTx}, 24 and 48 hours after infection with R7041, according to the experiment timeline as shown in Figure 15. Three days post-transfection, viability of tested cell lines was measured by MTS assay as described above. For both tested human HCCs, the combination of two different compounds: an oncolytic R7041 and a nontoxic dose of mRNA-US3-DMP^{CTx} (0.04 µg/mL) significantly enhanced tumor destruction at lower viral titres, as shown in Figure 16. In this figure, the effect on viability is shown for mRNA-US3-DMP^{CTx} alone at 0.04 µg/mL (y-axis cross), for R7041 alone at various dilutions (grey triangles/diamonds) and for the combination (black circles).

The above Examples indicate that the combination of an attenuated oncolytic virus with deleted virulence genes, and a supply of differentially expressed replacements for the deleted genes can significantly increase the efficacy of oncolytic viral therapy *in vitro.* In particular, greater effects were seen at lower viral titres when in combination with the composition of the invention.

### Example 4: Expression of delivered fluorescent protein mCherry mRNA constructs in vivo

In order to determine the applicability of the invention for *in vivo* approaches, a mouse model of orthotopic human hepatocellular carcinoma was used. Differential expression of driven by miRNA-122 binding sites has been shown above (see Example 1) to be applicable in healthy mouse Aml12 liver cells *in vitro.*

### Orthotopic human hepatocellular carcinoma (HCC) model

### Animals

Female (CB17/Ics-PrkdcSCID/IcrlcoCrl) Fox Chase SCID mice at 6-8 weeks old, were purchased from Charles River, UK. All *in vivo* procedures were approved by the Subcommittee on Research Animal Care, at CrownBio in UK.

### Cells

To generate an orthotopic HCC model, a bioluminescent variant of the human Hep3B cell line expressing firefly luciferase (Hep3B-cLuX) was used. Cells were cultured in EMEM medium (Sigma, UK) supplemented with 10% heat inactivated FBS, 2 mM L-Glutamine, 1% NEAA; Cells were treated weekly with 2 µg/mL Puromycin (Sigma).

### Intrahepatic injection and tumor growth monitoring

Under anesthesia, human Hep3B-cLuX cells (2 ×10⁶) suspended in 20 µL of 1:1 PBS:Matrigel^{™} were injected in the upper left lobe of the liver using a 29G needle. The injection site was covered using an absorbable gelatin sponge (AGS), the liver was placed back into the abdominal cavity without disturbing the AGS, and the skin was stitched closed. Tumor growth was checked twice weekly by bioluminescent imaging (BLI).

Briefly, the mice were anesthetised, and 150 mg/kg D-Luciferin was injected subcutaneously 15 minutes prior to imaging. BLI image was captured and processed using Living Image 4.3.1 software (Caliper LS, US). Mice were weighed three times weekly, or once weekly prior to dosing. On the indicated days, the mice were sacrificed, and the livers were fixed with 2 or 4% paraformaldehyde solution (PFA), before freezing in OCT (Optimal cutting temperature compound - embedding medium) for further histopathological analysis.

### Formulation of mRNA and evaluation of tumor targeting efficiency

mRNA sequences comprising the mCherry sequence, and the sequence of mCherry comprising miRNA-122 (SEQ ID NO: 31) were formulated as described above in the 'Synthesis of DM^{pCTx} and formulation of mRNA' paragraph, and Table 4.. To evaluate selective tumor targeting and the sparing of non-diseased liver cells, formulated mRNA was injected into the tail vein of mice bearing orthotopic liver cancer. Briefly, 2 ×10⁶ of human Hep3B-cLuX cells suspended in 20 µL of 1:1 PBS:Matrigel^{™} were injected in the upper left lobe of the liver as described above. Tumor growth was then monitored by BLI imaging, also as above. Eight days later, when the tumor was established (BLI ≥ 6 × 10⁶), 20 µg of formulated mRNA-mCherry-DMP^{CTx}, mRNA-mCherry-122-DMP^{CTx} or mRNA-A-122-DMP^{CTx} per mouse was injected through the tail vein, leading to the delivery particles being taken into the liver by returning blood flow. Twenty-four hours later last BLI was performed, mice were euthanised, and the livers were excised and imaged by BLI *ex vivo* at the localised liver lesions.

### Histology

Briefly, following *ex vivo* imaging, left liver lobes with tumor were removed, fixed with 2% PFA, immersed in 30% sucrose solution (in PBS; pH7.4) at 4°C, embedded in OCT and frozen in isopentane pre-cooled with dry ice bath, and then stored at -80°C. 5 µm frozen sections (Leica CM300, USA) were subjected to nuclear counterstain with DAPI (VECTASHIELD, Vector Laboratories, USA) or H&E (hematoxylin end eosin) staining. Tumor targeting was assessed by determination of mCherry vs mCherry-122 expression level in tumor and healthy liver using fluorescence microscopy and/or software . Tumourous and healthy tissue was determined by H&E staining.

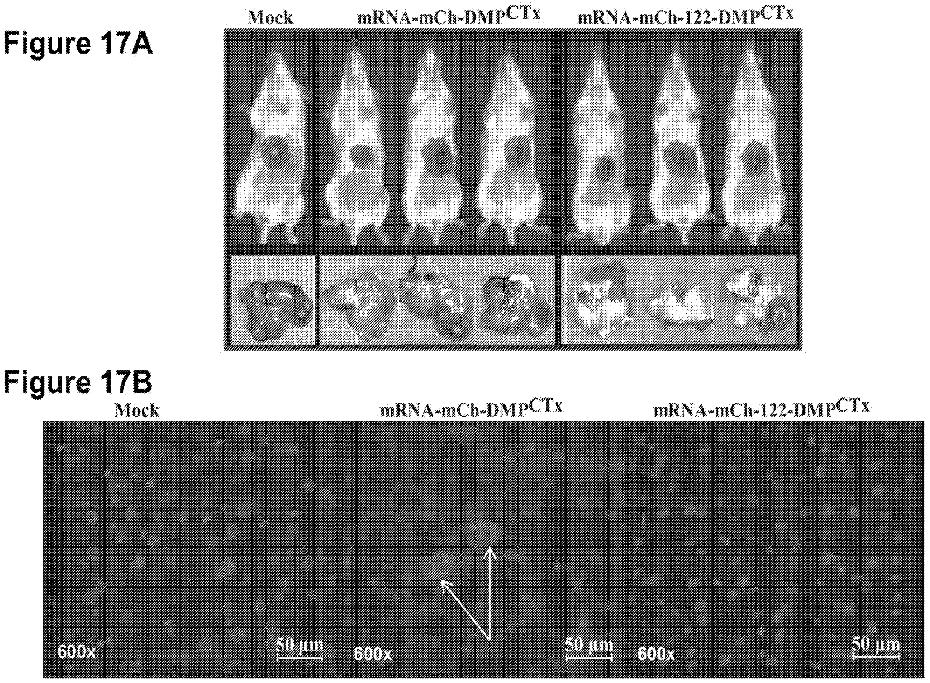

An example of tumor growth monitored by BLI imaging on mock and mRNA-mCherry-DMP^{CTx}, and mRNA-mCherry-122-DMP^{CTx} treated mice. The animals were injected subcutaneously with D-luciferin, and imaged 15 min later. Signal was present in the midsection of the animals only as shown in Figure 17 (A) (upper panel), animals shown are prior to treatment with compositions. All animals with similar intensities in the midsection were dissected, and the livers were imaged *ex vivo,* Figure 17 (A) (lower panel). The left lobe of liver with tumour was sectioned and counterstained with DAPI. Fluorescence microscopy was used to determine the expression of mCherry in healthy liver cells, and in liver tumour cells, 24 hours after injection of formulated mRNA, as shown in Figure 17 (B). mCherry fluorescence was detected in healthy hepatocytes when mice were treated with mRNA-mCherry-DMP^{CTx} (Figure 17**B**, middle panel). When treated with mRNA-mCherry-122-DMP^{CTx} (Variant 1), translation repression was observed (Figure 17**B**, left panel) Figure 17B shows healthy liver cells from (left to right), mock treated, mRNA-mCherry-DMP^{CTx}, and mRNA-mCherry-122-DM^{pCTx} mice.

In conclusion, the compositions of the invention can be administered *in vivo* and can successfully transfect targeted liver cells. When modified with miRNA binding sites, differential expression can be achieved in non-diseased and tumoural cells.

### Example 5: Differential expression of delivered US3 mRNA construct in vivo between non-diseased and diseased tissue in the liver

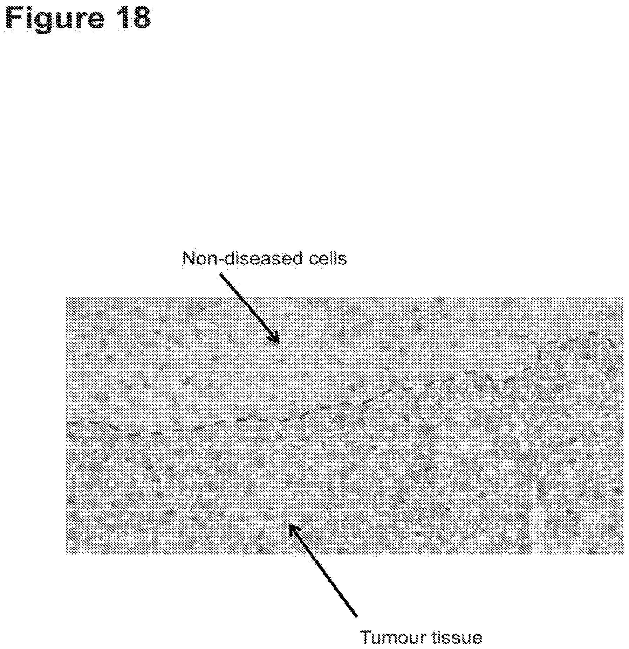

The in vivo mouse model described in Example 4 was applied to administration of a delivery particle composition comprising a US3 mRNA DM^{pCTx} miRNA-122 construct. Differential expression of US3 in the livers of mice containing Hep3B human cancer was analysed using immunohistochemistry with an anti US3 polyclonal antibody. The results are shown in Figure 18, where it can be seen that there is a visible difference in US3 protein levels between the tumour (darker staining) and non-diseased cells (lighter staining). The differential expression tracks the boundary of the tumour, as independently verified by a pathologist. It can be concluded, therefore, that the compositions of the invention can successfully drive differential expression of a potential therapeutic enhancement factor in vivo in a mammalian subject.

### Immunohistochemistry

Fresh frozen sections were cut at 5µm (microns) and air-dried for approximately one hour prior to fixation with 4% paraformaldehyde at room temperature (RT) for 15 minutes. Sections were washed in running tap water and transferred to PBS-0.1%Tween. Sections were incubated with 2.5% normal horse serum (ready to use, ImmPRESS HRP anti-rabbit IgG peroxidase polymer detection kit, Vector MP-7401) for 20 minutes. The slides were drained and incubated with primary antibody to US3 diluted 1:400 (Acris AP55266SU-N). The antibodies were diluted with PBS-0.1%Tween and negative controls were included where the primary antibody was omitted and slides incubated with the antibody diluent PBS-0.1%Tween for one hour at RT. Slides were washed with PBS-0.1%Tween and endogenous peroxidase blocked with 0.3% hydrogen peroxide diluted with elga water for 10 minutes. Slides were washed with PBS-0.1%Tween and incubated with ImmPress anti-rabbit IgG reagent (ready to use, ImmPRESS HRP anti-rabbit IgG peroxidase polymer detection kit, Vector MP-7401) for 30 minutes at RT. Slides were washed with PBS-0.1%Tween and incubated for 5 minutes with chromogen ImmPACT DAB (ImmPACT DAB Peroxidase (HRP) Substrate, Vector SK-4105) then washed with elga water and counterstained as appropriate with Mayer's haematoxylin. A further wash in elga water briefly was carried out and blue in running tap water for 5 minutes. The slides were dehydated, cleared and mounted (95% IMS, 99%IMS x2 and xylene x2) then covered with a coverslip.

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the spirit and scope of the invention as defined by the claims. Any non-human nucleic acid and/or polypeptide sequences that have been included in constructs and vectors according to embodiments of the invention have been obtained from sources within the UK, USA and European Union. To the inventor's knowledge, no genetic resources that would be subject to access and benefit sharing agreements, or associated traditional knowledge has been utilised in the creation of the present invention.

Alternative expressions of the inventive concept are set out in the following numbered clauses:
1. An isolated mRNA sequence for expression of one or more polypeptides within one or more target organs, the sequence comprising:
   at least one coding sequence which codes for the at least one polypeptide;
   at least a first untranslated region (UTR) sequence;
   a plurality of micro-RNA (miRNA) binding site sequences;
   wherein each of the miRNA binding site sequences is located within, immediately 5' to or immediately 3' to, the first UTR sequence; and
   wherein the miRNA binding site sequences allow for differential expression of the coding sequence in at least a first and a second cell type within the target organ or organs.
2. The isolated mRNA sequence of clause 1 wherein the mRNA sequence comprises greater than two, suitably greater than three, typically greater than four binding site sequences.
3. The isolated mRNA sequence of clause 1 or 2, wherein the plurality of miRNA binding site sequences comprise at least two substantially similar sequences.
4. The isolated mRNA sequence of any of clauses 1 to 3, wherein the plurality of miRNA binding site sequences comprise at least two substantially different sequences.
5. The isolated mRNA sequence of any of clauses 1 to 4, wherein the plurality of miRNA binding site sequences are substantially complementary to miRNA sequences selected from at least one or more of the group consisting of: miRNA-122; miRNA-125a; miRNA-125b; miRNA-199, miRNA-124a; Let-7; miRNA-148a; miRNA-148b; miRNA-375; miRNA-143; miRNA-145; miRNA192; miRNA194; miRNA-204; miRNA215; miRNA-30b, and miRNA-30c.
6. The isolated mRNA sequence of any of clauses 1 to 5, wherein at least one of the plurality of miRNA binding site sequences comprises one or more of SEQ ID NOS: 1 to 7.
7. The isolated mRNA sequence of clause 6, wherein at least one of the plurality of miRNA binding site sequences comprises SEQ ID NO: 1.
8. The isolated mRNA sequence of any of clauses 1 to 7 wherein the binding site sequences comprise each of SEQ ID NOs: 1, 2, 3, 4 and 5; or wherein the binding site sequences comprise each of SEQ ID NOs: 1, 2, 5, 6 and 7.
9. The isolated mRNA sequence of any of clauses 1 to 8, wherein the first and second cell types are different selections from the group consisting of non-neoplastic cells, a transformed cell phenotype; a pre-cancerous phenotype; and a neoplastic phenotype.
10. The isolated mRNA sequence of any of clauses 1 to 9, wherein the target organ or organs are selected from the group consisting of: liver; brain; lung; breast; pancreas; colon and kidney.
11. The isolated mRNA sequence of clauses 1 to 10, wherein at least one of the one or more polypeptides comprises a therapeutic enhancement factor.
12. The isolated mRNA sequence of clause 11, wherein the therapeutic enhancement factor is an immunomodulatory molecule selected from the group consisting of:
   (i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
   (ii) dendritic cell activators selected from one or more of: GM-CSF, TLR7 and TLR9;
   (iii) molecules targeting the following cellular receptors and their ligands selected from one or more of: CD40, CD40L, CD160, 2B4, Tim-3, GP-2, B7H3 and B7H4;
   (iv) TGF β inhibitors;
   (v) T-cell membrane protein 3 inhibitors;
   (vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
   (vii) NF-κB inhibitors.
13. The isolated mRNA sequence of any of clauses 1 to 12, wherein the mRNA comprises more than one open reading frame (ORF).
14. The isolated mRNA sequence of any of clauses 1 to 12, wherein the mRNA comprises a sequence selected from one of the group consisting of: SEQ ID NOs: 18 to 29.
15. A pharmaceutical composition comprising the isolated mRNA sequence of any of clauses 1 to 14, and a delivery particle, the sequence being comprised within the delivery particle, and a pharmaceutically acceptable carrier.
16. The composition of clause 15, wherein the delivery particle is selected from at least one of the group consisting of: an aminoalcohol lipidoid particle; a liposome; an exosome; a cell-derived vesicle; and a polymeric particle.
17. The composition of clause 15 or 16, wherein the delivery particle is targeted towards one or more of the target organ or organs.
18. The composition of clause 17, wherein the delivery particle comprises a targeting agent selected from: proteins, peptides, carbohydrates, glycoproteins, lipids, small molecules and nucleic acids; and
   wherein the targeting agents associate preferentially with cells in the target organ or organs.
19. A polynucleotide expression vector construct encoding the mRNA sequence of any of clauses 1 to 14.
20. A viral vector comprising the the mRNA sequence of any of clauses 1 to 19, or the polynucleotide expression vector construct of clause 20.
21. A method for the treatment of cancer, the method comprising administering to a subject in need thereof a composition comprising the isolated mRNA sequence, composition, vector construct, or viral vector of any of clauses 1 to 20.
22. The method of clause 21, the method further comprising administering a therapy or therapeutic agent to the subject.
23. The method of clause 22, wherein the therapy or therapeutic agent is selected from chemotherapy, radiotherapy, a biological agent, an oncolytic virus, a small molecule drug, a CAR-T or adoptive cell therapy, and combinations thereof.
24. The method of any of clauses 21 to 23, wherein the subject is a human.
25. The method of any of clauses 21 to 23 , wherein the subject is a non-human animal.
26. The method of any of clauses 21 to 25 , wherein the cancer is selected from at least one of the group consisting of: liver, brain, lung, breast, pancreas, colorectal and kidney cancer.
27. The method of clause 26, wherein the cancer is liver cancer.
28. The method of clause 27, wherein the liver cancer is primary liver cancer, or a secondary liver cancer.
29. The method of clauses 27 or 28, wherein the liver cancer is a primary liver cancer.
30. The method of clause 27 or 28, wherein the liver cancer is a secondary liver cancer.
31. The method of clause 29, wherein the primary liver cancer is selected from the group consisting of: a hepatocarcinoma; a hepatoblastoma; a cholangiocarcinoma; and a angiosarcoma.
32. The method of clause 30, wherein the secondary liver cancer is a metastatic liver cancer from a known or unknown primary solid tumor.
33. The method of any of clauses 23 to 32, further comprising administering an oncolytic virus to the subject.
34. The method of clause 33, wherein the isolated mRNA sequence codes for a therapeutic agent which increases the efficacy of the oncolytic virus.
35. The method of clause 33 or 34, wherein the oncolytic virus has been attenuated by mutation of one or more virulence genes.
36. The method of clause 35, wherein the mRNA sequence codes for the one or more virulence genes, or an equivalent or homologue thereof.
37. The method of any of clauses 33 to 36 wherein the oncolytic virus is selected from any one of the Groups I - VII of the Baltimore classification of viruses.
38. The method of any of clauses 33 to 37, wherein the oncolytic virus is selected from the group comprising one or more of: Vesicular Stomatitis Virus, Maraba virus, Polio virus, Reovirus, Measles virus, Newcastle disease virus, Coxsackievirus A21, Parvovirus, Herpes Simplex Virus Type 1, Vaccinia Virus, and Adenovirus.
39. The method of any of clauses 33 to 38, wherein the oncolytic virus is a Herpes Simplex Virus.
40. A composition comprising the isolated mRNA sequence, vector construct, or virus of any of clauses 1 to 14, 19, or 20, or the composition of clauses 15 to 18, for use in medicine.
41. A composition comprising the isolated mRNA sequence, vector construct, or virus of any of clauses 1 to 14, 19, or 20, or the composition of clauses 15 to 18, for use in the treatment of cancer, suitably wherein the cancer is selected from the group consisting of: liver, brain, lung, breast, pancreas, colorectal, and kidney cancer.
42. The method of any of clauses 23 to 32, further comprising administering a CAR-T or adaptive cell therapy to the subject.
43. The method of clause 42, wherein the isolated mRNA sequence, composition, vector construct, or viral vector encodes one or more immunomodulatory molecules selected from the group consisting of:
   (i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
   (ii) dendritic cell activators selected from one or more of: GM-CSF, TLR7 and TLR9;
   (iii) molecules targeting the following cellular receptors and their ligands selected from one or more of: CD40, CD40L, CD160, 2B4, Tim-3, GP-2, B7H3 and B7H4;
   (iv) TGF β inhibitors;
   (v) T-cell membrane protein 3 inhibitors;
   (vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
   (vii) NF-κB inhibitors.
44. The method of any of clauses 23 to 32, further comprising administering a cell checkpoint inhibitor to the subject.
45. The method of clause 44, wherein the isolated mRNA sequence, composition, vector construct, or viral vector encodes one or more immunomodulatory molecules selected from the group consisting of:
   (i) cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL 9, and CXCL10;
   (ii) dendritic cell activators selected from one or more of: GM-CSF, TLR7 and TLR9;
   (iii) molecules targeting the following cellular receptors and their ligands selected from one or more of: CD40, CD40L, CD160, 2B4, Tim-3, GP-2, B7H3 and B7H4;
   (iv) TGF β inhibitors;
   (v) T-cell membrane protein 3 inhibitors;
   (vi) inhibitors of programmed death 1 (PD1), programmed death-ligand 1 (PDL1), programmed death-ligand 2 (PDL2), cytotoxic T-lymphocyte antigen 4 (CTLA4), and lymphocyte-activation gene 3 (LAG3); and
   (vii) NF-κB inhibitors.

## Claims

1. An isolated mRNA sequence for expression of one or more cytokines within one or more target organs, the sequence comprising:
at least one coding sequence which codes for one or more cytokines (or their ligands) involved in immune response and inflammation selected from one or more of: TNFα, TNFβ, IFNα, IFNβ, IFNgamma, IL1, IL2, IL3, IL4, IL5, IL6, IL7, IL8, IL9, IL10, IL11, IL12, CCL2, CCL3, CCL4, CCL5, CXCL 9, and CXCL10;
at least a first untranslated region (UTR) sequence;
at least three different micro-RNA (miRNA) binding site sequences;
wherein each of the miRNA binding site sequences is located within, immediately 5' to or immediately 3' to, the first UTR sequence; and
wherein the miRNA binding site sequences allow for differential expression of the coding sequence in at least a first and a second cell type within the target organ or organs.

2. The isolated mRNA sequence of claim 1 wherein the mRNA sequence comprises at least four, typically at least five miRNA binding site sequences.

3. The isolated mRNA sequence of any of claims 1 to 4, wherein the plurality of miRNA binding site sequences are substantially complementary to miRNA sequences selected from at least one or more of the group consisting of: miRNA-122; miRNA-125a; miRNA-125b; miRNA-199, miRNA-124a; Let-7; miRNA-148a; miRNA-148b; miRNA-375; miRNA-143; miRNA-145; miRNA192; miRNA194; miRNA-204; miRNA215; and miRNA-30.

4. The isolated mRNA sequence of any of claims 1 to 3, wherein at least one of the at least three miRNA binding site sequences comprises one or more of SEQ ID NOS: 1 to 7, typically wherein the binding site sequences comprise each of SEQ ID NOs: 1, 2, 3, 4 and 5; or wherein the binding site sequences comprise each of SEQ ID NOs: 1, 2, 5, 6 and 7.

5. The isolated mRNA sequence of any of claims 1 to 4, wherein:
i) the first and second cell types are different selections from the group consisting of non-neoplastic cells, a transformed cell phenotype; a pre-cancerous phenotype; and a neoplastic phenotype; and/or
ii) the target organ or organs are selected from the group consisting of: liver; brain; lung; breast; pancreas; colon and kidney.

6. The isolated mRNA sequence of any of claims 1 to 5, wherein the mRNA comprises more than one open reading frame (ORF), typically wherein the further ORF or ORFs encode: one or more cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5 CXCL9, and CXCL10; or one or more dendritic cell activators selected from one or more of: GM-CSF, TLR7 and TLR9.

7. The isolated mRNA sequence of any of claims 1 to 6, wherein the mRNA comprises SEQ ID NO: 29.

8. A composition comprising the mRNA sequence of any of claims 1 to 7, and a further mRNA sequence comprising:
at least one coding sequence which codes for one or more cytokines (or their ligands) involved in immune response and inflammation selected from one or more of: TNF α, TNFβ, IFNα, IFNβ, IFNgamma, IL1, IL2, IL3, IL4, IL5, IL6, IL7, IL8, IL9, IL10, IL11, IL12, CCL 2, CCL3, CCL4, CCL5, CXCL 9, and CXCL10;
at least a first untranslated region (UTR) sequence;
at least three different micro-RNA (miRNA) binding site sequences;
wherein each of the miRNA binding site sequences is located within, immediately 5' to or immediately 3' to, the first UTR sequence.

9. A composition comprising the mRNA sequence of any of claims 1 to 8, and a further mRNA sequence comprising:
at least one coding sequence which codes for one or more dendritic cell activators selected from one or more of: GM-CSF, TLR7 and TLR9;
at least a first untranslated region (UTR) sequence;
at least three different micro-RNA (miRNA) binding site sequences;
wherein each of the miRNA binding site sequences is located within, immediately 5' to or immediately 3' to, the first UTR sequence.

10. A pharmaceutical composition comprising the isolated mRNA sequence of any of claims 1 to 7, or the composition of claim 8 or claim 9, and a delivery particle, the sequence or sequences being comprised within the delivery particle, and a pharmaceutically acceptable carrier, suitably wherein the delivery particle is selected from at least one of the group consisting of: an aminoalcohol lipidoid particle; a liposome; an exosome; a cell-derived vesicle; and a polymeric particle.

11. The composition of claim 10, wherein the delivery particle is targeted towards one or more of the target organ or organs, typically wherein the delivery particle comprises a targeting agent selected from: proteins, peptides, carbohydrates, glycoproteins, lipids, small molecules and nucleic acids; and
wherein the targeting agents associate preferentially with cells in the target organ or organs.

12. The isolated mRNA sequence or composition of any of claims 1 to 11 for use in a method for the treatment of cancer, the method comprising administering to a subject in need thereof a composition comprising the isolated mRNA sequence or composition.

13. The isolated mRNA sequence or composition for use according to claim 12, the method further comprising administering a therapy or therapeutic agent to the subject, suitably wherein the therapy or therapeutic agent is selected from chemotherapy, radiotherapy, a biological agent, an oncolytic virus, a small molecule drug, a CAR-T or adoptive cell therapy, and combinations thereof.

14. The isolated mRNA sequence or composition for use according to any of claims 12 or 13, wherein:
i) the cancer is selected from at least one of the group consisting of: liver, brain, lung, breast, pancreas, colorectal and kidney cancer;
ii) the cancer is liver cancer;
iii) the cancer is a primary liver cancer;
iv) the cancer is a secondary liver cancer;
v) the cancer is a primary liver cancer selected from the group consisting of: a hepatocarcinoma; a hepatoblastoma; a cholangiocarcinoma; and a angiosarcoma; or
vi) the cancer is a secondary metastatic liver cancer from a known or unknown primary solid tumor.

15. The isolated mRNA sequence or composition for use according to any of claims 12 to 14, the method further comprising administering an oncolytic virus to the subject.

16. The isolated mRNA sequence or composition for use according to claim 15, wherein:
i) the isolated mRNA sequence codes for a therapeutic agent which increases the efficacy of the oncolytic virus; and/or
ii) the oncolytic virus has been attenuated by mutation of one or more virulence genes; and/or
iii) the oncolytic virus has been attenuated by mutation of one or more virulence genes and the mRNA sequence codes for the one or more virulence genes, or an equivalent or homologue thereof; and/or
iv) the oncolytic virus is selected from any one of the Groups I - VII of the Baltimore classification of viruses; and/or
v) the oncolytic virus is selected from the group comprising one or more of: Vesicular Stomatitis Virus, Maraba virus, Polio virus, Reovirus, Measles virus, Newcastle disease virus, Coxsackievirus A21, Parvovirus, Herpes Simplex Virus Type 1, Vaccinia Virus, and Adenovirus; and/or
vi) the oncolytic virus is a Herpes Simplex Virus; and/or
vii) the oncolytic virus is a Vaccinia virus.
